(19) Europäisches Patentamt
European Patent Office
Office européen des brevets

(11) **EP 3 142 728 B1**

(12) **EUROPÄISCHE PATENTSCHRIFT**

(45) Veröffentlichungstag und Bekanntmachung des Hinweises auf die Patenterteilung:
**14.03.2018 Patentblatt 2018/11**

(21) Anmeldenummer: **15715301.6**

(22) Anmeldetag: **15.04.2015**

(51) Int Cl.:
*A61M 5/145* *(2006.01)*    *A61M 5/142* *(2006.01)*
*A61M 5/24* *(2006.01)*    *A61M 5/315* *(2006.01)*
*A61M 5/168* *(2006.01)*

(86) Internationale Anmeldenummer:
**PCT/EP2015/058194**

(87) Internationale Veröffentlichungsnummer:
**WO 2015/172962 (19.11.2015 Gazette 2015/46)**

(54) **DOSIERVORRICHTUNG FÜR DIE ABGABE VON MEDIKAMENTENFLUID AUS EINEM RESERVOIR MIT SPINDELSTANGE ZUR VERSCHIEBUNG DES KOLBENS**

DOSING DEVICE FOR DELIVERY OF FLUID MEDICAMENT FROM A CARTRIDGE HAVING A TELESCOPING ROD FOR DISPLACING THE PISTON

DISPOSITIF DE DOSAGE POUR LA PERFUSION D'UN MEDICAMENT LIQUIDE D'UNE CARTOUCHE COMPRENANT UNE TIGE TELESCOPIQUE POUR DEPLACER LE PISTON

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **14.05.2014 PCT/EP2014/059889**
**18.11.2014 PCT/EP2014/074907**

(43) Veröffentlichungstag der Anmeldung:
**22.03.2017 Patentblatt 2017/12**

(73) Patentinhaber: **Meamedical AG**
**4853 Riken (CH)**

(72) Erfinder: **NIKLAUS, Hanspeter**
**CH-4853 Riken (CH)**

(74) Vertreter: **Schneider Feldmann AG**
**Patent- und Markenanwälte**
**Beethovenstrasse 49**
**Postfach**
**8027 Zürich (CH)**

(56) Entgegenhaltungen:
EP-A1- 1 759 727        EP-A2- 2 146 114
WO-A1-2013/004308        CH-A2- 705 428
DE-A1- 3 910 814        DE-A1- 19 840 992
US-A1- 2003 167 039        US-A1- 2004 000 818

**Beschreibung**

Technisches Gebiet

Die Erfindung betrifft eine Dosiervorrichtung für die Abgabe von Medikamentenfluid in den Körper eines Anwenders nach dem Oberbegriff des Anspruchs 1.

Stand der Technik

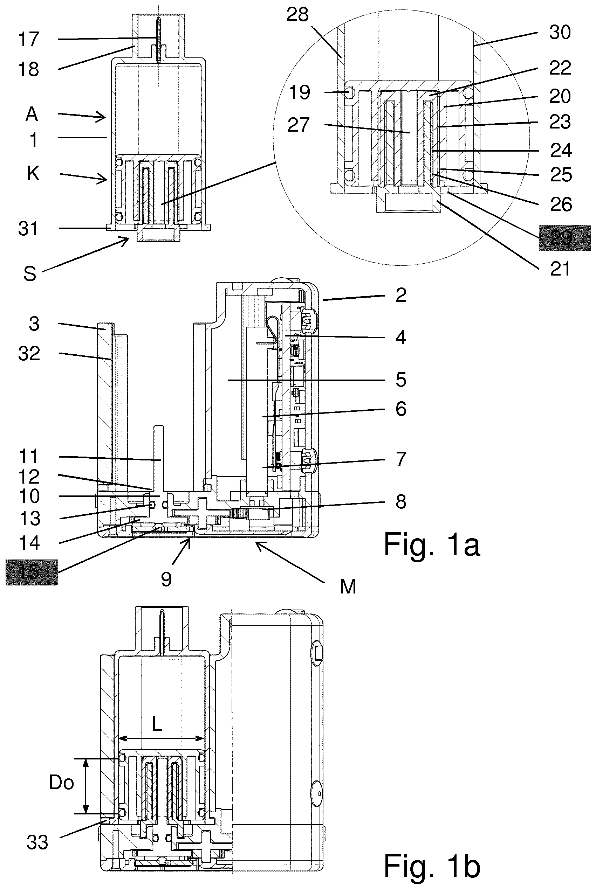

Für die Verabreichung von fluidischen Medikamenten, insbesondere für Insulin in der Diabetesbehandlung kommen tragbare Injektions- und / oder Infusionsgeräte zum Einsatz. Das Medikamentenfluid wird dabei mittels einer Dosiervorrichtung, die eine Antriebsvorrichtung für einen Kolben und ein Reservoir mit dem Fluid umfasst, kontinuierlich oder quasi kontinuierlich gefördert. Dabei wird der Kolben des Reservoirs verfahren und im Reservoir befindliches Medikamentenfluid verdrängt und verabreicht. Einsatz finden derartige Geräte als Pumpengeräte und manuell zu betätigende Pens in der Insulinbehandlung. Sowohl für Injektionspens als auch für Insulinpumpen gilt, dass diese Geräte möglichst kompakt, zuverlässig, einfach in der Bedienung und für den Anwender sicher sein müssen.
Ein Beispiel einer derartigen Dosiervorrichtung ist die D-TRONplus Insulinpumpe der Firma Roche Diabetes Care GmbH. Diese weist eine an der Pumpe permanent angeordnete Spindelvorrichtung auf, die aus zwei teleskopischen, ausfahrenden Verschiebestufen und einer rotierenden, axial festgelagerten Antriebsstufe gebildet ist. Dabei kann die erste Verschiebestufe, welche gegen den Kolben des Reservoirs verfahrbar ist, lediglich einen Vortrieb ausführen. Beim Verfahren der ersten Verschiebestufe stösst sich diese an der zweiten Verschiebestufe ab, wobei die zweite Verschiebestufe dabei von der Antriebsstufe drehend mitgenommen wird. Nach dem Verfahren der ersten Verschiebestufe erfolgt das Verfahren der zweiten Verschiebestufe. Die zweite Verschiebestufe führt dabei lediglich eine Translation aus und stösst sich dabei an der weiter drehend angetriebenen Antriebsstufe ab. Die Spindelvorrichtung der D-TRONplus Insulinpumpe ist in der DE19717107B4 beschrieben. In der DE19840992A1 sind weiter ein Verfahren und eine Vorrichtung zur Überwachung des Drucks im Reservoir der D-TRONplus Insulinpumpe beschrieben. In dieser Druckschrift ist ein Antrieb für einen Kolben beschrieben, der in einem festen Pumpengehäuse über O-Ringe und gegen die Vortriebsrichtung des Kolbens schwimmend gelagert ist, wobei der Antrieb ein eigenes Gehäuse als Verschiebeplattform, einen Motor, ein Getriebe und die teleskopische Spindelvorrichtung umfasst. In der Verschiebeplattform ist mindestens die Spindelvorrichtung angeordnet, vorzugsweise werden sowohl die Motor-Getriebe Einheit als Antriebsvorrichtung als auch die Spindelvorrichtung in der Verschiebeplattform integriert. In der DE19840992A1 wird vorgeschlagen, den schwimmend gelagerten Antrieb über einen Kraftsensor am Gehäuse abzustützen. Über den Kraftsensor kann derart eine axiale Reaktionskraft an der Spindelvorrichtung indirekt gemessen werden, wobei die Reaktionskraft proportional zum Druck im Reservoir ist. In der Fig. 1 ist gezeigt, dass die Verschiebeplattform am Pumpengehäuse über ein Paar von O-Ringen abgedichtet wird. Die elastischen O-Ringe dienen weiter zur schwimmenden Lagerung der Verschiebeplattform im festen Pumpengehäuse. Die Dichtungsstellen weisen relativ ungünstig grosse Durchmesser auf. Diese äusseren, zwischen der Verschiebeplattform und dem Pumpengehäuse angeordneten Dichtungsstellen sind für eine genaue Druckbestimmung im Reservoir nicht geeignet. Bei einem Anstieg des Fluiddrucks im Reservoir verschiebt sich der Antrieb gegen seinen Anschlag. Der zwischen dem Antrieb und dem Pumpengehäuse angeordnete, einen Biegebalken aufweisende Kraftsensor verformt sich dabei proportional zur Reaktionskraft am Biegebalken. Die elastischen O-Ringe verformen sich dabei ebenso und wirken der Bewegung des Antriebs entgegen. Da die O-Ringe einen grossen Umfang aufweisen, sind die durch die elastische Auslenkung erzeugten Rückstellkräfte relativ gross, wodurch das Messresultat für den Druck im Reservoir erheblich verfälscht wird. Die beiden Dichtungsstellen der Fig. 1 der DE19840992A1 ermöglichen lediglich eine Abdichtung zwischen der Verschiebeplattform des Antriebs und dem festen Pumpengehäuse der Dosiervorrichtung. In der Fig. 24 der DE19717107B4 ist weiter gezeigt, dass die teleskopischen Verschiebestufen und die Antriebsstufe der Spindelvorrichtung ebenso Dichtungen aufweisen, um beispielsweise ein Eindringen von Leckagenfluid zu einer Elektronik und / oder einer Energiequelle der Dosiervorrichtung über die Spindelvorrichtung zu verhindern. Hierfür sind gemäss der Fig. 24 drei weitere Dichtungsstellen notwendig, über welche die zwei verfahrbaren Verschiebestufen und die feste Antriebsstufe abgedichtet werden können. Trotz des grossen technischen Aufwandes zur Abdichtung des Antriebs zum Schutze der Elektronik und der Energiequelle verbleibt nach wie vor der Nachteil, dass die Spindelvorrichtung, respektive die Spindelgewinde nicht vor Verschmutzungen geschützt werden können. Die Spindelvorrichtung der D-TRONplus Pumpe ist vor Verschmutzungen, wie beispielsweise Staub, Insulin, Putzmittel zur Reinigung und Wasser, nicht schützbar. Derartige Verschmutzungen können die Lebensdauer und Zuverlässigkeit dieser permanent am Gehäuse angeordneten Spindelvorrichtung wesentlich reduzieren, denn sie beschleunigen die Korrosion und erhöhen die Reibung in den Spindeltrieben. Hingegen ist es vorteilhaft, dass der über den Kraftsensor axial gelagerte Antrieb ein Auffahren der Spindelvorrichtung an den Kolben des Reservoirs detektieren kann. Derart kann der Füllstand, resp. die Menge an Insulin im Reservoir bei Teil- oder Ganzbefüllung automatisch ohne Einwirken des Anwenders bestimmt werden. Hierzu wird ausgehend von einer komplett eingefahrenen Position die Spindelvorrichtung in Vortriebsrichtung verfahren und dabei die Anzahl der Motorschritte bis zum Auffahren am Kolben bestimmt. Das Auffahren an den Kolben, welches einen Kraftanstieg am Kraftsensor bewirkt, kann von einer Steuerung erkannt werden. Erkennt die Steuerung das Auffahren, so setzt sie den Motor in Stillstand. Die bis zum Auffahren gemachten Motorschritte werden von einer Gesamtmotorschrittanzahl subtrahiert und derart schliesslich der Füllstand bestimmt. Die D-TRONplus Pumpe gehört der ersten Generation von Insulinpumpen an, bei welchen die Insulinpumpe körpernah getragen wird, beispielsweise in einer Hosentasche, und über einen Katheterschlauch das Insulin zu einer durch eine Kanüle gebildeten Infusionsstelle für die Abgabe an einen Anwender gefördert wird.

In der WO2006/104806 ist eine Dosiervorrichtung der zweiten Generation offenbart. Geräte der zweiten Generation werden direkt am Körper getragen. Sie weisen eine für die Befestigung am Körper vorbereitete Unterseite auf und können beispielsweise am Bauch oder am Arm direkt aufgeklebt werden. Vorteilhaft ist hier, dass kein Katheterschlauch zwischen der Pumpen und der Injektionsstelle mehr benötigt wird, wie dies bei den Geräten der ersten Generation der Fall ist. Hingegen weisen Geräte der zweiten Generation, wie beispielsweise die Omnipod Insulinpumpe der Firma Insulet Corp., den Nachteil auf, dass diese Geräte lediglich einmalig verwendet werden können. Geräte der zweiten Generation weisen keine Bauteile auf, die mehrmalig von einem Anwender verwendet werden können. Die Spindelvorrichtung gemäss der WO2006/104806 weist eine fest mit dem Kolben verbundene Spindelstange mit einem Gewinde auf. Vor der Befüllung befindet sich der Kolben in seiner ausgefahrenen Position. Bei der Befüllung wird der Kolben samt der Spindelstange rückwärtig verfahren, wobei ein örtlich nicht verschiebbares Antriebsrad zur Bildung eines Spindeltriebs, die Spindelstange bei der rückwärtigen Verschiebung und Befüllung freigibt. Das bedeutet, dass das Antriebsrad und die Spindelstange bei der rückwärtigen Verschiebung und einer damit einhergehenden Befüllung des Reservoirs nicht miteinander gekuppelt sind. Lediglich bei der Abgabe von Medikamentenfluid durch Vortrieb des Kolbens ist das angetriebene Antriebsrad über einen Spindeltrieb mit der Spindelstange gekuppelt. Bei der Befüllung des Reservoirs kommt es zu einer Relativverschiebung zwischen dem örtlich festen Antriebsrad und der rückwärtig verschiebbaren Spindelstange. Bei der rückwärtigen Verschiebung der Spindelstange stösst die Spindelstange bei einem definierten Reservoirvolumen auf eine Sensorstange, wobei durch weiteres Verschieben der Spindelstange die Sensorstange quer zur Spindelstange gegen einen Kontaktpunkt ausgelenkt wird. Durch Berühren des Kontaktpunktes wird ein Kontaktsignal generiert und an eine Steuereinheit geleitet. Sobald die Steuereinheit das Kontaktsignal erkennt, befindet sich der Kolben an einer definierten Position mit einem bekannten Füllvolumen. Nachteilig an dieser neuen Methode der Füllstandsermittlung ist, dass sie technisch anfällig und in Folge vieler geometrischer Toleranzen und Toleranzketten ungenau ist und dass sie lediglich einen minimalen Füllstand überwachen kann. Zur Überwachung des Füllstandes über den ganzen Verfahrweg des Kolbens genügt eine einzelne aus einer Sensorstange und einem Kontaktpunkt gebildete Sensorikeinheit daher nicht. Hierfür müssen entlang des Verfahrweges der Spindelstange mehrere Sensorstangen und Kontaktpunkte angeordnet werden, was die Füllstandsermittlung technisch aufwendiger und teurer macht. Weiter kann das Gerät der WO2006/104806 lediglich einmalig verwendet werden, wodurch die Gerätekosten für die Therapie hoch zu stehen kommen. Weiter weist das Gerät keinen Schutz gegenüber internen Reservoirleckagen auf, was die Sicherheit für den Anwender infolge von Kurzschlüssen und Ausfällen der Elektronik und / oder der Energieversorgung reduziert. Medizinische Dosiergeräte können zur Überwachung weitere Sensoriken aufweisen. Bei der Abgabe von fluidischen Medikamenten können im fluidführenden Kanal Verschlüsse oder Teilverschlüsse auftreten, diese werden in der Fachsprache als Okklusionen bezeichnet. Okklusionen führen dazu, dass der Anwender nicht optimal mit Medikamentenfluid versorgt wird, wodurch das Therapieresultat negativ beeinflusst werden kann. In der Insulinpumpentherapie führen Okklusionen zu einer Unterversorgung des Patienten mit Insulin, was zu einem unkontrollierten Anstieg des Blutzuckerspiegels führt. Mittels geeigneter Sensorik werden die Dosiergeräte bei ihrem Betrieb hinsichtlich des Auftretens von Okklusionen überwacht, so dass die Geräte selbständig Okklusionen erkennen und den Anwender alarmieren können. Die Okklusionserkennung, also die Erkennung von Verschlüssen oder Teilverschlüssen im fluidführenden Kanal, welches durch beispielsweise Knicken einer Kanüle des fluidführenden Pfades oder Einwachsen von Gewebe in die Kanüle entstehen kann, erfolgt bei der WO2006/104806 über eine zweite Sensorikeinheit und kann beispielsweise wiederum aus Sensorstangen und Kontaktpunkten gebildet sein. Nachteilig für das Gerät der WO2006/104806 ist, dass für die Okklusionserkennung und die minimale Füllstandsüberwachung mehrere Sensorikeinheiten benötigt werden.

Als der dritten Generation angehörende Geräte werden hier Geräte bezeichnet, die wie die Geräte der zweiten Generation am Körper getragen werden, diese unterscheiden sich aber gegenüber den Geräten der zweiten Generation darin, dass sie nebst Verbrauchsteilen auch einen wiederverwendbaren Teil aufweisen. Geräte dieser neusten Generation sind beispielsweise aus der WO2010/076792A1 und der US2011/0213329A1 bekannt. Für beide in der WO2010/076792A1 und der US2011/0213329A1 offenbarten Ausführungsformen umfasst der Verbrauchsteil ein Reservoir, einen Kolben, eine am Verbrauchsteil angeordnete Spindelvorrichtung, eine Energiequelle und eine fluidische Verbindung vom Reservoir zur Injektionsstelle. Auch hier wird zur Befüllung des Reservoirs der Kolben von einer anfänglich ausgefahrenen Position in eine befüllte Ausgangsposition rückwärtig verschoben. Dabei überströmt Medikamentenfluid von einem Vorratsbehälter in das Reservoir. Die Spindelvorrichtung der US2011/0213329A1 umfasst eine starr mit dem Kolben verbundene Spindelstange und eine die Spindelstange umgebende, einen variablen Durchmesser aufweisende Spindelmutter. Beim Befüllen kommt es hier wie für die Dosiervorrichtung der zweiten Generation gemäss der WO2006/104806 zu einer Relativverschiebung zwischen der Spindelstange und der Spindelmutter. Erst beim Verbinden des Verbrauchsteils mit dem wiederverwendbaren Teil wird die Spindelmutter mit der Spindelstange über eine Gewindeverbindung gekuppelt. Beim Verbinden des Verbrauchsteils mit dem wiederverwendbaren Teil wird die Spindelstange zuerst in Eingriff mit einer Antriebshülse gebracht. Beim Verbinden der zwei Gehäusehälften, also beim Verbinden des wiederverwendbaren Teils und des Verbrauchsteils, wird die Spindelmutter von der Antriebshülse axial an einem Sitz aufgenommen. Der an der Antriebshülse ausgebildete Sitz zur Aufnahme der Spindelmutter ist konisch ausgebildet. Beim Verschieben der Spindelmutter gegen ihren festen und konisch ausgebildeten Sitz reduziert sich der Durchmesser der Spindelmutter bis ein Innengewinde der Spindelmutter in ein Aussengewinden der Spindelstange eingreift und derart ein fester Spindeltrieb, respektive eine feste Gewindeverbindung zwischen der Spindelmutter und der Spindelstange hergestellt ist. Die Spindelmutter kann sich dabei direkt an der Antriebshülse abstützen. Alternativ ist auch eine Abstützung an einem am Verbrauchsteil ausgebildeten Anschlag möglich. Bei der in der US2011/0213329A1 beschriebenen Vorrichtung sind sowohl Teil- wie auch Ganzbefüllungen des Reservoirs möglich. Dadurch können Restmengen an Insulin, die vom Anwender nicht genutzt werden können, vermieden werden. Der Tagesbedarf - Total Daily Dosage, kurz TDD - kann stark variieren. Kinder benötigen ca. 10 bis 50 IU Insulin pro Tag, während Erwachsene zwischen ca. 40 und 150 IU Insulin pro Tag benötigen. Die in der US2011/0213329A1 offenbarte Dosiervorrichtung weist eine Sensorikeinheit auf, die den Anwender beim Unterschreiten eines definierten Füllstandes alarmieren kann. Hierzu weist die Dosiervorrichtung eine aus einer Lichtquelle und einem Detektor gebildete Sensoreinheit auf, die zur Überwachung der Position der Spindelstange verwendet wird. Nachteilig ist auch hier, dass die besagte Sensoreinheit lediglich einen minimalen Füllstand überwachen kann. Bei Unterschreiten des minimalen Füllstandes kann der Anwender alarmiert werden. Damit der Füllstand genauer überwacht werden kann, wird vorgeschlagen, entlang der Längsachse der Antriebshülse weitere, aus Lichtquellen und Detektoren gebildete Sensoreinheiten anzuordnen. Eine derartige Ausgestaltung mit mehreren entlang der Antriebshülse angeordneten Sensoreinheiten zur Überwachung des Füllstandes benötigt relativ viel Raumbedarf und ist im Betrieb in Folge erhöhter Komplexität anfälliger auf Störungen. Als Alternative wird eine um die Antriebshülse angeordnete induktive Spule für die Füllstandsermittlung vorgeschlagen. Diese Variante benötigt ebenso viel Raumbedarf und ist für kompakte, am Körper zu tragende Dosiervorrichtungen nicht optimal. Die Ausführungsformen für die Füllstandsermittlung und die Okklusionserkennung sind in der WO2009/125398 weiter detailliert beschrieben. Für die Okklusionserkennung wird eine weitere, zweite Sensoreinheit verwendet, die aus einem flexiblen, am Verbrauchsteil angeordneten Verbindungsschlauch und einer federgeführten, am wiederverwendbaren Teil angeordneten Sensorplatte besteht. Die in der US2011/0213329A1 beschriebene Dosiervorrichtung mit den für die Füllstandsermittlung und Okklusionserkennung in der WO2009/125398 beschriebenen Sensoreinheiten ist aufwendig und teuer, in der Montage schwierig und benötigt zudem viel Raumbedarf. Nachteilig ist besonders, dass für die Füllstandsermittlung und die Okklusionserkennung unterschiedliche Sensoreinheiten verwendet werden. Das in der US2011/0213329A1 offenbarte Gerät weist weitere Nachteile auf. Beispielsweise weist die Antriebshülse keine Dichtungsstellen auf, so dass eine vom Reservoir stammende Leckage über die Antriebshülse zum Getriebe und weiter in das Innere des wiederverwendbaren Teils gelangen kann. Gelangt Medikamentenfluid wie beispielsweise Insulin in den wiederverwendbare Teil, können beispielsweise Korrosionsschäden an den Bauteilen auftreten. Eine derartige Schädigung führt meist zu einer erheblichen Reduktion der Lebensdauer infolge von beschleunigter Alterung der Bauteile. Weiter ist anzumerken, dass eine Abstützung der Spindelmutter an der Antriebshülse nachteilig ist. Die über Schnapphaken gebildete Verbindung zwischen dem Gehäuse des Verbrauchsteils und dem Gehäuse des wiederverwendbaren Teils weist generell ein axiales Spiel auf. Ein axiales Zusammendrücken der beiden Gehäusehälften kann dazu führen, dass sich der Kolben relativ zu seiner Wandung verschiebt und dabei die Dosiervorrichtung eine entsprechende Menge an Insulin abgibt. Derartige Effekte können das Therapieresultat negativ beeinflussen, denn kleinste, relative Verschiebungen der Gehäusehälften gegeneinander können therapierelevante Bolusabgaben bewirken. Beispielsweise führt eine Verschiebung von 0.01 mm der Gehäusehälften gegeneinander zu einer gleichwertigen Verschiebung des Kolbens im Reservoir von 0.01 mm. Bei einem Querschnitt des Reservoirs von 125 mm^2 kommt es dabei bei einem Insulin mit einer Konzentration von U100-Insulin zu einer Bolusabgabe von 0.125 IU Insulin.

In der WO2010/076792A1 ist eine weitere Ausführungsform für die Spindelvorrichtung beschrieben, wobei hier nicht die Spindelmutter angetrieben wird, sondern die Spindelstange. Die Spindelstange ist bei dieser Ausführungsform gelenkig mit dem Kolben verbunden und am Kolben über ein Kugelgelenk gelagert. Auch bei dieser Ausführungsvariante werden bei der Befüllung die Spindelstange und damit der Kolben von einer ausgefahrenen Position in eine Ausgangsposition rückwärtig verfahren, um dadurch Medikamentenfluid von einem Vorratsbehälter in das Reservoir zu ziehen. Die Spindelmutter ist wiederum ortsfest am Verbrauchsteil angeordnet, so dass bei der Befüllung die Spindelstange relativ zu der ortfesten Spindelmutter rückwärtig verfahren wird. Da bei dieser Ausführungsform die Spindelstange drehend angetrieben ist, ist für die Erzeugung des Spindelstangenvortriebs eine ortsfeste und verdrehgesicherte Spindelmutter notwendig, an welcher sich die Spindelstange bei der Abgabe von Medikamentenfluid abstützen kann. Nach der Befüllung wird der Verbrauchsteil mit dem wiederverwendbaren Teil verbunden. Dabei wird die Spindelstange in eine am wiederverwendbaren Teil angeordnete Antriebshülse eingeführt. Beim Zusammenführen der beiden Gehäusehälften wird weiter die Spindelmutter mit der Spindelstange über eine Gewindeverbindung in Eingriff gebracht. Das Ausführungsbeispiel der WO2010/076792A1, das sich vom vorgängig beschriebenen Ausführungsbeispiel lediglich darin unterscheidet, dass nicht die Spindelmutter sondern die Spindelstange drehend angetrieben wird, behebt keine wesentlichen Schwachstellen und Nachteile der vorgängigen, in der US2011/0213329A1 beschriebenen Ausführungsform. Für die Füllstandsermittlung und die Okklusionserkennung kommen wiederum die gleichen wie in der WO2009/125398 beschriebenen Sensoreinheiten und Vorrichtungen zur Anwendung. Bei beiden in der US2011/0213329A1 und der WO2010/076792A1 beschriebenen Ausführungsvarianten ist es zudem schwierig, die Spindelstange des Verbrauchsteils in Eingriff mit der Antriebshülse des wiederverwendbaren Teils zu bringen. Gerade für Diabetiker, die motorisch eingeschränkt sein können, sind keine Führungen und Hilfsmittel für eine einfacher zu handhabende Kupplung der beiden Gehäusehälften vorgesehen. Eine Abdichtung für die Antriebshülse ist auch in der WO2010/076792A1 nicht vorgesehen.

In der WO2008/024814A2 ist in den Fig. 18 bis Fig. 22 ein Dosiervorrichtung mit einem wiederverwendbaren Teil und einem Verbrauchsteil dargestellt. Auch dieses Ausführungsbeispiel gehört den Dosiervorrichtungen der dritten Generation an. Das in den Fig. 18 bis Fig. 22 dargestellte Ausführungsbeispiel zeigt eine am Kolben durchgehend und in das Reservoir ragende Spindelstange, wobei der Kolben selbst als nicht rotierende Spindelmutter ausgestaltet ist. Am wiederverwendbaren Teil ist ein Kupplungsglied zur Übertragung einer Antriebsleistung an die Spindelvorrichtung des Verbrauchsteils angeordnet. Das Kupplungsglied ist weiter mit einem Abgangsrad eines Getriebes verbunden, wobei zwischen dem Kupplungsglied und dem Abgangsrad eine Dichtungsstelle angeordnet ist und das Kupplungsglied einen Anschlag für die Spindelstange aufweist. Das in den Fig. 18 bis Fig. 22 der WO2008/024814A2 dargestellte Ausführungsbeispiel ist vorteilhaft hinsichtlich der Abdichtung des wiederverwendbaren Teils. Die Dichtungsstelle ist an einem schlanken und rotierend angetriebenen Schaft ausgebildet. In der technischen Umsetzung ist hingegen die Abdichtung der rotierenden Spindelstange am Kolben schwierig. In der Praxis ist eine derartige, durchgehend am Kolben angeordnete Spindelstange bislang nicht in ein taugliches, am Markt erhältliches Produkt umgesetzt worden. Weiter ist die Befüllung des Reservoirs schwierig. Da die Spindelstange im Innern des Reservoirs angeordnet ist, ist eine gemeinsame Verschiebung des Kolbens samt der am Kolben angeordneten Spindelstange nicht möglich. Für die Befüllung ist es daher eher praktikabel, den Kolben durch Rotation der Spindelstange zu verschieben, um derart Medikamentenfluid von einem Vorratsbehälter in das Reservoir zu ziehen. Mittels dieser Methode, bei welcher der Kolben von einer vorderen Position rückwärtig zur Überleitung von Medikamentenfluid verfahren wird, sind sowohl eine Teil- wie auch eine Ganzbefüllung denkbar. Die am Kolben angeordnete Spindelmutter verfährt hierbei wiederum relativ zur festen Spindelstange, wie dies ebenso der Fall für die bereits besprochenen Beispiele der zweiten und dritten Generation ist.

In der nachfolgenden Tabelle ist der Stand der Technik zusammengefasst dargestellt. Dabei werden Dosiervorrichtungen der ersten Generation, der zweiten Generation und der neusten, dritten Generation einander gegenübergestellt und nach wesentlichen Kriterien für die Zuverlässigkeit und Langlebigkeit, die Handhabung und die Kosten verglichen. Ein wesentliches Kriterium, das die Zuverlässigkeit erhöht, ist die Anordnung der Spindelvorrichtung. Die Spindelvorrichtung wird bei Geräten der ersten Generation am wiederverwendbaren Teil angeordnet und ist damit nicht auswechselbar. Vorteilhaft ist es, wenn die Spindelvorrichtung am Verbrauchsteil angeordnet ist und mit jedem neuen Verbrauchsteil ersetzt wird. Weiter kann die Lebensdauer der Dosiervorrichtung verbessert werden, wenn der wiederverwendbare Teil gegen interne vom Reservoir stammende Leckagen abgedichtet ist. Dadurch wird beispielsweise sichergestellt, dass elektronische Bauteile, das Getriebe und der Motor des wiederverwendbaren Teils nicht mit Medikamentenfluid in Kontakt kommen, was zu einer beschleunigten Alterung der Dosiervorrichtung führen kann. Ein Kriterium zur Verbesserung der Handhabung ist eine automatische Füllstandsermittlung. Diese erleichtert die Handhabung für den Anwender und eliminiert Anwendungsfehler, welche bei einer manuellen Eingabe des Füllstands durch den Anwender auftreten können. Besonders vorteilhaft ist es zudem, wenn die gleiche Sensorik, welche für die Füllstandsermittlung eingesetzt wird, auch für die Drucküberwachung des Reservoirs verwendet werden kann. Dadurch können die Herstellungskosten gesenkt und die Komplexität des Gerätes reduziert werden. Die bei der Therapie entstehenden Kosten sind ein weiteres wesentliches Kriterium zur Beurteilung und Unterscheidung der Dosiervorrichtungen. Eine innovative Dosiervorrichtung soll bei der Anwendung keine hohen Kosten verursachen. Dosiervorrichtungen der zweiten Generation werden als Ganzes nach einer einmaligen Anwendung entsorgt und weisen keine Bauteile auf, die wiederverwendet werden können. Geräte der dritten Generations sind dahingehend verbessert worden, dass sie sowohl Verbrauchsteile aufweisen aber auch einen wiederverwendbaren Teil und derart einen Kostenvorteil erzielen können.

Aus nachfolgender Tabelle geht hervor, dass keine Generation des Standes der Technik vermag, alle Kriterien wie Zuverlässigkeit und Lebensdauer, die Handhabung und die Kosten gleichzeitig zu erfüllen. Von den definierten fünf Kriterien werden maximal drei Kriterien abgedeckt und erfüllt. Einige Dosiervorrichtungen vermögen lediglich zwei der fünf Kriterien gleichzeitig zu erfüllen.

| | Kriterium | Lebensdauer und Zuverlässigkeit | | Handhabung und Sicherheit | | Kosten |
|---|---|---|---|---|---|---|
| | | Spindelvorrichtung am Verbrauchsteil | Abdichtung gegen interne Leckage | Automatische Füllstandermittlung | Drucküberwachung gleiche Sensorik | Semi-Disposable |
| 1. Generation | **D-TRON - Roche** DE19717107B4 DE19840992A1 | Nein | Nein - Spindelvorrichtung nicht abdichtbar | Ja - via Kraftsensor | Ja – via Kraftsensor, jedoch ungenaue Messung | Ja |
| 2. Generation | **OmniPod – Insulet** WO2006/104806A2 | Ja | Nein | Nein, nur Überwachung minimaler Füllstand | Nein, andere Sensorik | Nein |
| 3.Generation | **Medtronic** EP2407192B1 WO2008/024814A2 | Ja | Ja | Nein | Nein, andere Sensorik | Ja |
| 3.Generation | **Solo M - Roche** WO2010/076792A1 | Ja | Nein | Nein, nur Überwachung minimaler Füllstand | Nein, andere Sensorik | Ja |
| 3.Generation | **Solo M - Roche** US2011/0213329A1 | Ja | Nein | Nein, nur Überwachung minimaler Füllstand | Nein, andere Sensorik | Ja |

Geräte der zweiten Generation weisen bei der kontinuierlichen Infusion von Insulin, hiernach CSII genannt - Continuous Subcutaneous Insulin Infusion, lediglich eine Lebensdauer von wenigen Tagen auf, meist ca. drei Anwendungstage. Derartige Geräte werden als Ganzes entsorgt, womit die Therapiekosten unvorteilhaft hoch ausfallen und in Folge der hohen Kosten nicht alle Anwender Zugang zu dieser Technologie finden können, was ein bedeutender Nachteil ist. Dosiervorrichtungen der ersten Generation weisen den Nachteil auf, dass zwischen der Dosiervorrichtung und dem Anwender eine auffällige, nicht diskret tragbare und den Alltage behindernde Katheterschlauchverbindung vorhanden ist. Bei den Dosiervorrichtungen der ersten Generation ist die Spindelvorrichtung fest am wiederverwendbaren Teil angeordnet und dadurch einem dauernden Verschleiss ausgesetzt. Im Falle der D-TRONplus Pumpe, welche in den Druckschriften der DE19717107B4 und der DE19840992A1 beschrieben ist, sind speziell die Gewinde der Spindelvorrichtung infolge einer nicht vorhandenen Abdichtung einem starken Verschleiss infolge von Verschmutzungen ausgesetzt. Ein erhöhter Verschleiss der Spindelvorrichtung kann die Lebensdauer und die Zuverlässigkeit der Insulinpumpe erheblich reduzieren. Vorteilhaft hingegen sind die automatische Füllstandsermittlung sowie die Drucküberwachung des Reservoirs über den Kraftsensor.

Geräte der dritten Generation sind dahingehend verbessert worden, dass die Spindelvorrichtung als wesentlicher Verschleissteil vom wiederverwendbaren Teil zum Verbrauchsteil verlagert worden ist, um dadurch die Zuverlässigkeit der Dosiervorrichtung zu verbessern und die Lebensdauer zu erhöhen. Eine automatische Füllstandsermittlung über beispielsweise einen Kraftsensor weisen diese Geräte nicht auf, was wiederum ein bedeutender Nachteil der dritten Generation ist. Bei den Geräten der zweiten und dritten Generation wird bei der Befüllung des Reservoirs eine mit dem Kolben fest oder über ein Gelenk verbundene Spindelstange relativ zu einer Spindelmutter rückwärtig verschoben. Die relative Verschiebung zwischen der Spindelstange und der Spindelmutter, die bei der Befüllung von einem Anwender ausgeführt werden kann, führt nachteiligerweise dazu, dass weitere Sensorik notwendig ist, um die Lage der Spindelstange in Bezug zu einem absoluten Koordinatensystem festzulegen und letztlich daraus einen minimalen Füllstand zu überwachen. Das vorgängig besagte Gerät der zweiten Generation weist hierzu die beschriebene, aus einer Sensorstange und einem Kontaktpunkt gebildete Sensoreinheit auf. Die Geräte der dritten Generation weisen hierzu eine alternative Sensorlösung auf, welche aus einer Lichtquelle und einem Detektor für die Lichtquelle besteht. Auch diese Alternative kann nachteiligerweise lediglich einen minimalen Füllstand überwachen, die Ermittlung des effektiven Füllstandes ist nicht möglich. Hierzu ist weitere Sensorik notwendig. Damit die Dosiervorrichtungen der dritten Generation eine möglichst geringe Länge aufweisen, wird die axiale Ausdehnung des Kolbens möglichst kurz ausgestaltet. Dies ermöglicht die Bildung einer kompakten Dosiervorrichtung, führt aber im Gegenzug nachteilig dazu, dass der im Reservoir gehaltene Kolben schlecht geführt ist und beim Vortrieb ein Walken des Kolbens mit einer einhergehenden verminderten Ausschüttungsgenauigkeit auftreten kann. Ein Mass für die Qualität der Lagerung des Kolbens in einem Reservoir zur Vermeidung von Walken ist das Längenverhältnis L/Do, wobei L eine Längsachse des ovalen Kolbenquerschnittes ist und Do der Abstand zwischen Dichtstellen am Kolben. Sowohl die Geräte der zweiten Generation als auch die Geräte der dritten Generation weisen hier ungünstig grosse Längenverhältnisse auf von mehr als 2.5, infolge einer starren oder einer gelenkig ausgeführten Verbindung von Spindelstange und Kolben. Vorteilhafterweise weist das Verhältnis L/Do einen Wert von mehr als 0.5 auf und weniger als 2.5.

Weiter sind aus der WO94/15660 und der WO97/00091 teleskopartige und einstufige Spindelvorrichtungen bekannt. Die teleskopartigen Spindelvorrichtungen sind in ihrer Anordnung stets gleich aufgebaut und weisen eine erste und zweite Verschiebestufe auf, wobei die erste Verschiebestufe umhüllend die zweite Verschiebestufe aufnimmt und die zweite Verschiebestufe umhüllend eine Antriebsstufe aufnimmt. Bei dieser Anordnung treibt die Antriebsstufe jeweils eine der beiden Verschiebestufen an, so dass die Verschiebestufen sequentiell ausfahren. Die teleskopartigen Spindelvorrichtungen der WO94/15660 und der WO97/00091 stützen sich dabei an einem festen Stützteil ab, der zudem als Verdrehsicherungsbasis für die Verschiebestufen dient. Ebenso ist die Antriebsstufe axial verschiebefest am Stützteil gelagert. Sowohl der Stützteil als auch ein Reservoirkörper sind mit einem Zwischenteil verbindbar. Die lösbare Verbindung zwischen dem Stützteil und dem Zwischenteil wird über eine Magnetverbindung hergestellt. Der Zwischenteil und das mit ihm verbundene Reservoir sind weiter mit einer wiederverwendbaren Antriebsvorrichtung kuppelbar. Im gekuppelten Zustand treibt ein seitlich angeordnetes, treibendes Zahnrad der Antriebsvorrichtung ein axial angeordnetes, getriebenes Zahnrad der Spindelvorrichtung an. Das getriebene Zahnrad ist starr mit der Antriebsstufe verbunden. Die Antriebsstufe, respektive die Spindelvorrichtung stützen sich dabei nicht an einem festen Gehäuse sondern am Stützteil axial ab. Die teleskopartigen Spindelvorrichtungen der WO94/15660 und der WO97/00091 sind in ihrem Aufbau komplex, benötigen viele Bauteile, weisen eine komplizierte Montage auf und sind in der Handhabung schwierig. Zudem ist es notwendig, das Reservoir stets ganz zu befüllen, denn nur dann ist das getriebene Zahnrad der Spindelvorrichtung mit dem treibenden Zahnrad der Antriebsvorrichtung kuppelbar. Weiter weisen die Ausführungsformen der WO94/15660 und der WO97/00091 keine Mittel auf, um die Antriebsvorrichtung vor internen Leckagen zu schützen. In der WO94/15660 ist weiter eine einstufige Spindelvorrichtung offenbart, die eine an einem Kolben durchgehend angeordnete Spindelstange aufweist. Die Spindelstange stützt sich dabei an einem Zwischenteil ab. Der Zwischenteil lagert hier die Spindelstange axial verschiebefest, nimmt einen Reservoirkörper auf und ist mit einem festen Gehäuse einer Antriebsvorrichtung verbindbar. Axial wirkende Kräfte werden über die Spindelvorrichtung an den Zwischenteil geleitet und von diesem aufgenommen. Die Antriebsvorrichtung weist auch hier ein Kupplungsglied für die Spindelstange auf. Bei der Befüllung des Reservoirs verfährt der Kolben, an welchem eine Spindelmutter ausgeführt ist, rückwärtig und relativ zur Spindelstange. Auch hier sind keine Mittel vorhanden, um die Antriebsvorrichtung vor Verschmutzungen, Leckagen des Reservoirs und anderen flüssigen Medien zu schützen.

Darstellung der Erfindung

Die Aufgabe der vorliegenden Erfindung ist es, eine Dosiervorrichtung für die Abgabe von Medikamentenfluid zu schaffen, insbesondere für die Abgabe von Insulin, welche die beschriebenen Nachteile der ersten, zweiten und dritten Generation überwindet und insbesondere eine Dosiervorrichtung hinsichtlich der Kriterien Lebensdauer und Zuverlässigkeit, Herstellungskosten und Therapiekosten, sowie der Handhabung für den Anwender zu verbessern.

Die gestellte Aufgabe wird durch die im Anspruch 1 definierte Dosiervorrichtung gelöst. Diese weist zur Verabreichung von Medikamentenfluid in den Körper eines Anwenders einen wiederverwendbaren Teil und einen mindestens einen Verbrauchsteil auf, wobei das Medikamentenfluid zum Zwecke einer Infusion oder einer Injektion aus einem Reservoir durch Vortrieb eines im Reservoir gehaltenen Kolbens dosiert ausschüttbar ist. Der wiederverwendbare Teil umfasst ein Gehäuse, eine Steuereinheit und eine Antriebsvorrichtung, wobei die Antriebsvorrichtung ein Kupplungsglied zur Übertragung einer Antriebsleistung an eine Spindelvorrichtung zum Vortrieb des Kolbens aufweist, und das Kupplungsglied mit einem Abgangsrad der Antriebsvorrichtung fest verbunden ist und einen axialen Anschlag für die Spindelvorrichtung aufweist. Weiter kann vorzugsweise zwischen dem Abgangsrad und dem Kupplungsglied eine Dichtungsstelle angeordnet sein. Der mindestens eine Verbrauchsteil umfasst das Reservoir und den im Reservoir gehaltenen, von der Spindelvorrichtung vortriebbaren Kolben, wobei die Spindelvorrichtung am Verbrauchsteil angeordnet ist und mindestens einen, insbesondere aus einem Innengewinde und einem Aussengewinde gebildeten, Spindeltrieb mit einem Verfahrweg V pro Spindeltrieb aufweist, wobei die Spindelvorrichtung (S) des Verbrauchsteils (1) über das Kupplungsglied mit dem wiederverwendbaren Teil (2) kuppelbar ist. Weiter umfasst die Dosiervorrichtung eine fluidführende Verbindung zwischen dem Reservoir und dem Anwender und einen am wiederverwendbaren Teil angeordneten Kraftsensor, der eine als Mass für den Druck im Reservoir dienende Reaktionskraft der Spindelvorrichtung misst. Dadurch, dass

i) die Dosiervorrichtung derart ausgestaltet ist, dass sich die Spindelvorrichtung (S) des Verbrauchsteils (1) nach einer Teil- oder Ganzbefüllung des Reservoirs (A) im eingefahrenen Zustand befindet und so ein definierter, stets konstanter Gesamthub (H) der Spindelvorrichtung (S) verfahrbar ist,

ii) das Kupplungsglied axial gleitgelagert ist und das Abgangsrad (14) sich über den Kraftsensor (15) an einer fixen Basis abstützt, wobei aufgrund der festen Verbindung zwischen Kupplungsglied und Abgangsrad beim Anliegen der Spindelvorrichtung (S) am Anschlag (12) die am Anschlag (12) axial wirkende Reaktionskraft der Spindelvorrichtung (S) über das Abgangsrad an den Kraftsensor (15) geleitet wird,

lässt sich eine Dosiervorrichtung bilden, die gegenüber dem Stand der Technik wesentlich verbessert werden kann.

Es wurde vorteilhaft gefunden, dass die vorliegende Erfindung eine Dosiervorrichtung der dritten Generation derart weiterentwickelt, dass die Füllstandsermittlung und die Okklusionserkennung über den Kraftsensor erfolgen können, wie dies aus der D-TRONplus Pumpe der ersten Generation bekannt ist, während gleichzeitig die Vorteile der Geräte der dritten Generation wie Zuverlässigkeit, gute Abdichtbarkeit und Austauschbarkeit der Spindelvorrichtung zumindest beibehalten oder verbessert werden konnten.

Im Weiteren wurde vorteilhaft gefunden, dass die erfindungsgemässe Dosiervorrichtung, die aus mindestens einem Verbrauchsteil mit einer Spindelvorrichtung und einem wiederverwendbaren Teil gebildet werden kann, für eine Okklusionserkennung und eine Füllstandsermittlung eine gleiche Sensorik in Form eines Kraftsensors verwenden kann, deren Lebensdauer und Zuverlässigkeit durch Verlagerung von Verschleissteilen wie der Spindelvorrichtung vom wiederverwendbaren Teil an den mindestens einen Verbrauchsteil verbessert wird und die sowohl in der Herstellung günstig ist und bei der Therapie für den Kostenträger reduzierte Kosten verursacht.

Die Verbesserungen gegenüber dem Stand der Technik werden nachfolgend aufgelistet, da durch die Kombination der Merkmale i) und ii) wesentliche Fortschritte erzielt werden:

- Durch Kombination der Merkmale i) und ii) wird erreicht, dass sowohl die Füllstandsermittlung als auch die Okklusionsüberwachung über den Kraftsensor erfolgen können, wobei die Spindelvorrichtung erfindungsgemäss am mindestens einen Verbrauchsteil angeordnet ist. Nach der Kupplung kann die Spindelvorrichtung erst gegen den Anschlag entgegen der Vortriebsrichtung verfahren. Da der Kolben im Reservoir über Reibung gehalten ist, wird beim Auffahren der Spindelvorrichtung an ihren am Kupplungsglied ausgebildeten Anschlag eine Reaktionskraft erzeugt. Diese Reaktionskraft wird über das axial gleitgelagerte Kupplungsglied zusammen mit dem Abgangsrad an den Kraftsensor geleitet. Erreicht die Reaktionskraft einen definierten Sollwert, so kann die Steuereinheit das Auffahren der Spindelvorrichtung an den Anschlag erkennen und die Antriebseinheit in Stillstand setzen. Die Antriebseinheit kann eine Untersetzung in Form eines Getriebes und / oder antreibende Mittel in Form eines Motors, einer Formgedächtnislegierung oder anderer antreibender Mittel aufweisen. Am Ausgang der Antriebseinheit ist erfindungsgemäss das Kupplungsglied angeordnet, über welches die Spindelvorrichtung angetrieben wird. Generell wird die Antriebseinheit in diskreten Schritten verfahren, wobei ein Schritt einem konstanten Winkelinkrement entsprechen kann. Die Anzahl der Schritte bis zum Auffahren der Spindelvorrichtung an den Anschlag kann von der Steuereinheit zur Berechnung des Füllstandes verwendet werden. Für jedes neue Reservoir, unabhängig davon ob es teil- oder ganzbefüllt ist, verfährt die Antriebseinheit stets eine konstante Anzahl von Schritten, da sich die Spindelvorrichtung erfindungsgemäss nach der Befüllung in ihrem eingefahrenen Zustand befindet. Die Anzahl der nach dem Auffahren verbleibenden, für die Abgabe von Medikamentenfluid zur Verfügung stehenden Schritte ist proportional zum anfänglichen Füllstand. Durch Aufsummierung der Schritte bei der Abgabe von Medikamentenfluid kann weiter auch ein momentaner Füllstand des Reservoirs berechnet werden.

Bei der Abgabe von Medikamentenfluid überwacht die Steuerung über das Messsignal desselben Kraftsensors wie für die Füllstandsermittlung das Auftreten von Okklusionen im Fluidpfad, welche durch beispielsweise Knicken einer Kanüle oder Einwachsen von Gewebe in die Kanüle entstehen können. Kommt es zu einem Verschluss in der fluidführenden Verbindung, so steigt der Druck im Reservoir bei der weiteren Abgabe von Medikamentenfluid stetig an. Wird beispielsweise eine Schwelle überschritten, so kann die Steuerung den Anwender über eine Okklusion alarmieren. Zur Auswertung des Messsignals des Kraftsensors sind verschiedenste Algorithmen anwendbar. Im einfachsten Fall, wird eine Okklusion alarmiert, sobald ein konstanter Kraftwert oder Schwelle überschritten wird. Denkbar sind aber auch Algorithmen, bei welchen eine Serie von Messwerten über ein bestimmtes Zeitintervall verwendet wird. Nebst den Kraftwerten, welche über den Kraftsensor ermittelt werden und in einer Steuerung gespeichert werden können, können auch andere Signale wie beispielsweise ein Motorstrom bei der Abgabe von Medikamentenfluid, in die Algorithmen miteinbezogen werden. Infolge des ansteigenden Drucks im Reservoir bei einer Okklusion, steigen die Motorströme infolge der ansteigenden Last am Motor an.

- Erfindungsgemäss ist die Spindelvorrichtung am mindestens einen Verbrauchsteil angeordnet. Hierdurch wird erreicht, dass für jedes neue Reservoir eine neue Spindelvorrichtung verwendet wird. Die Zuverlässigkeit und die Lebensdauer der erfindungsgemässen Dosiervorrichtung lassen sich dadurch verbessern, denn die Spindelvorrichtung weist bei Förderung stets ein konstantes Antriebsdrehmoment auf, da sie stets ersetzt wird und so keinem Verschleiss ausgesetzt ist. Bei der D-TRONplus Pumpe des Standes der Technik, bei welcher die Spindelvorrichtung am wiederverwendbaren Teil angeordnet ist, nimmt das Drehmoment der Spindelvorrichtung infolge Verschleiss und Reibung mit der Lebensdauer zu, was zu einer Herabsetzung von Zuverlässigkeit und Lebensdauer führt.

- Die erfindungsgemässe Dosiervorrichtung erleichtert zudem die Handhabung für den Anwender, da ein Zurückdrehen der Spindelvorrichtung in den Ausgangszustand nicht notwendig ist. Ein Zurückdrehen der Spindelvorrichtung in den Ausgangszustand kann bei der D-TRONplus Pumpe mehrere Minuten dauern und benötigt viel Energie.

- Weiter ist der Kraftsensor erfindungsgemäss direkt unter dem Abgangsrad angeordnet. Dadurch kann die am Anschlag wirkende Reaktionskraft, welche der Stosskraft der Spindelvorrichtung entspricht, genauer gemessen werden als bei den Ausgestaltungen des Standes der Technik. Die am Anschlag wirkende Reaktionskraft wird über das Abgangsrad auf den Kraftsensor geleitet. Die Dichtungsstelle zwischen den Abgangsrad und dem Kupplungsglied beeinflusst die am Kraftsensor wirkende Kraft und kann das Messresultat des Kraftsensors beeinflussen. Grundsätzlich wird angestrebt, den Druck im Reservoir über den Kraftsensor möglichst genau zu messen. Der Druck im Reservoir übt eine Druckkraft auf den Kolben aus, an der Spindelvorrichtung wirkt nebst der Druckkraft auch die zwischen Kolben und Reservoir wirkende Reibkraft. Die an der Spindelvorrichtung wirkende Stosskraft wird über den Anschlag des Kupplungsgliedes auf das Abgangsrad geleitet, welches über den Kraftsensor gehalten ist. Die am Kraftsensor axial eingeleitete Reaktionskraft ist daher proportional zur Druckkraft im Reservoir, wobei sie durch die Reibkraft am Kolben und die Reibkraft der Dichtungsstelle zwischen Abgangsrad und Kupplungsglied beeinflusst werden kann. Angestrebt wird, die Reibkraft an der Dichtungsstelle möglichst gering zu halten. Dies kann erreicht werden, indem das Abgangsrad und das Kupplungsglied über einen schlanken Schaft miteinander verbunden werden. Der schlanke Schaft ermöglicht eine gute Abdichtung, zudem sind die Reibkräfte der Dichtungsstelle gering, wodurch die Genauigkeit der Messung der Druckkraft über den Kraftsensor verbessert werden kann.

Dosiervorrichtungen der ersten Generation sind nachteilig, da diese infolge des Katheterschlauchs nicht diskret tragbar sind und den Anwender in seiner Bewegungsfreiheit einschränken können. Zudem ist die Spindelvorrichtung permanent am wiederverwendbaren Teil angeordnet und dadurch im Betrieb einem steten Verschleiss ausgesetzt, was die Lebensdauer der Dosiervorrichtung limitieren kann. Die D-TRONplus Pumpe der ersten Generation bietet jedoch den Vorteil, dass die Okklusionserkennung und die Füllstandsermittlung über eine gleiche Sensorik, das heisst über den Kraftsensor, erfolgen können. Dadurch lassen sich die Komplexität und die Herstellungskosten der Dosiervorrichtung reduzieren. Bei Geräten der zweiten und dritten Generation wird bei der Befüllung stets eine Spindelstange relativ zu einer Spindelmutter verfahren, wodurch eine Füllstandsermittlung über einen Kraftsensor nicht anwendbar ist, da die Position der Spindelstange zur Spindelmutter nach der Befüllung nicht bekannt ist. Bei allen Geräten der zweiten und dritten Generation ist die Spindelvorrichtung nicht am wiederverwendbaren Teil sondern am Verbrauchsteil angeordnet, um derart die Zuverlässigkeit der Dosiervorrichtung zu verbessern. Bei der Befüllung wird stets die Spindelstange relativ zu einer Spindelmutter verschoben. Durch diese relative Verschiebung entsteht ein Informationsverlust bezüglich der Position der Spindelstange relativ zur Spindelmutter, denn nach der Befüllung ist die Position der Spindelstange relativ zur Spindelmutter unbekannt. Bei den Geräten der zweiten und dritten Generation wird nach dem Verbinden des Verbrauchsteils mit dem wiederverwendbaren Teil die Lage der Spindelstange zu einer ortsfesten Sensorik, welche am wiederverwendbaren Teil angeordnet ist, bestimmt. Dadurch kann zumindest ein minimaler Füllstand überwacht werden. Aufwändigere Sensorik erlaubt hier maximal eine Füllstandsüberwachung entlang der Spindelstange, was nachteilig ist. Geräte der zweiten und dritten Generation benötigen daher für die Okklusionserkennung und die Füllstandsermittlung zwei separate Sensorikeinheiten, was die Kosten treibt und die Komplexität der Pumpe erhöht. Die Füllstandsermittlung, respektive die minimale Füllstandsüberwachung werden stets durch Überwachen der Spindelstangenposition bestimmt.

Die Erfindung hat hier erkannt, dass die Spindelvorrichtung wie bei den Systemen der zweiten und dritten Generation am mindestens einen Verbrauchsteil angeordnet sein soll, um eine möglichst zuverlässige und langlebige Dosiervorrichtung zu bilden. Im Gegensatz zu den Geräten der zweiten und dritten Generation hat die Erfindung weiter erkannt, dass die Spindelvorrichtung nach einer Teil- oder Ganzbefüllung im eingefahrenen Zustand sein muss, bei welchem beispielsweise die Position einer Spindelstange relativ zur einer Spindelmutter eines Spindeltriebes bekannt ist. Dadurch kann erreicht werden, dass die Spindelvorrichtung nach der Befüllung in einem stets gleichen Gesamthub verfahrbar ist. Dies gilt sowohl für Spindelvorrichtungen mit einem Spindeltrieb, aber auch für teleskopische Spindelvorrichtungen mit mindestens zwei Spindeltrieben. Dadurch, dass die Spindelvorrichtung nach der Befüllung in ihrer eingefahrenen, bekannten Position ist, kann ein wesentlicher Nachteil der Geräte der zweiten und dritten Generation überwunden werden, bei welchen die Position der Spindelvorrichtung nach der Befüllung unbekannt ist. Dieser Verhalt, wonach die Position der Spindelvorrichtung nach der Befüllung definiert und bekannt ist, nützt die Erfindung aus. Diese Erkenntnis ermöglicht für die erfindungsgemässe Dosiervorrichtung, dass nebst der Okklusionserkennung auch die Füllstandsermittlung über den Kraftsensor erfolgen kann. Im Gegensatz zu der D-TRONplus Pumpe verfährt die Spindelvorrichtung nicht von einer eingefahrenen Position lediglich in Vortriebsrichtung, sondern erst rückwärtig, d.h. entgegen der Vortriebsrichtung, gegen den Anschlag und anschliessend in Vortriebsrichtung zur Abgabe von Medikamentenfluid, wobei unabhängig vom anfänglichen Füllstand stets ein Gesamthub verfahren wird.

Bei der erfindungsgemässen Dosiervorrichtung wird der Kraftsensor direkt unter dem Abgangsrad angeordnet. Das Abgangsrad ist dabei gegen den Kraftsensor gleitgelagert. Bei der D-TRONplus Pumpe der ersten Generation ist der Kraftsensor zwischen der Verschiebeplattform und dem Pumpengehäuse angeordnet, wobei die Verschiebeplattform über O-Ringe im Pumpengehäuse gehalten ist. Die Erfindung hat weiter erkannt, dass es besonders vorteilhaft ist, den Kraftsensor nicht unter einer Verschiebeplattform anzuordnen, sondern direkt unter dem Abgangsrad der Antriebsvorrichtung. Das Abgangsrad und das Kupplungsglied können über einen Schaft miteinander verbunden sein. An diesem Schaft kann vorzugsweise eine Dichtungsstelle angeordnet sein. Diese kann über O-Ringe gebildet sein, sie kann aber auch über eine passgenaue Führung am Gehäuse ohne Verwendung von O-Ringen ausgeführt sein. Bei letzterer erfolgt die Dichtung lediglich über die eng ausgeführte Führung und Lagerung des Schaftes am Gehäuse. Der Schaft wird generell schlank ausgeführt; weiter ist er zur Übertragung der Antriebsleistung an die Spindelvorrichtung drehend angetrieben. Bei einer durch einen O-Ring ausgebildeten Abdichtung entstehen infolge der schlanken Ausführung des Schaftes und der Drehbewegung des Schaftes lediglich geringe axiale Reibkräfte. Der schlanke Schaft erlaubt daher eine bessere Abdichtung im Vergleich zur D-TRONplus Pumpe der ersten Generation. Weiter kann die erfindungsgemässe Dosiervorrichtung den im Reservoir herrschenden Druck im Vergleich zur D-TRONplus Pumpe der ersten Generation genauer messen. Okklusionen können derart schneller und zuverlässiger erkannt werden, was eine deutliche Therapieverbesserung darstellt. Durch die raschere Erkennung wird zudem das bei einer Okklusion im Reservoir aufgestaute Volumen, fachsprachlich Okklusionsvolumen oder Okklusionsbolus genannt, erheblich reduziert. Die schnellere Erkennung von Okklusionen sowie die Reduktion der entsprechenden Okklusionsvolumina können die Therapie und damit beispielsweise die Kontrolle des Blutzuckers wesentlich verbessern.

Die Leistung der Erfindung ist es, die technischen Merkmale i) und ii) miteinander zu kombinieren, so dass gleichzeitig alle vorgängig im Stand der Technik definierten fünf Kriterien erfüllt werden können. Die Kriterien werden hier nochmals aufgeführt und die Verbesserungen gegenüber dem Stand der Technik besprochen. Die erfindungsgemässe Dosiervorrichtung soll derart ausgestaltet sein, dass sie zuverlässig und langlebig ist. Dies wird erreicht, indem die Spindelvorrichtung am mindestens einen Verbrauchsteil angeordnet wird und vorzugsweise der wiederverwendbare Teil nach aussen gut abdichtbar ist. Weiter sollen die Füllstandsermittlung und die Okklusionserkennung über eine gleiche Sensorik erfolgen, wodurch die Zuverlässigkeit und Langlebigkeit der erfindungsgemässen Dosiervorrichtung verbessert werden kann und die Herstellungskosten reduziert werden können. Dies wird erreicht, indem hierfür ein Kraftsensor direkt unter dem Abgangsrad der Antriebsvorrichtung angeordnet wird und nach dem Befüllen die Spindelvorrichtung stets im eingefahrenen Zustand ist. Dadurch kann über den Kraftsensor die Füllstandsermittlung automatisch ausgeführt werden. Zwischen dem Abgangsrad und dem Kupplungsglied kann zudem ein schlanker Schaft angeordnet sein, welcher sich besonders gut für eine Abdichtung des Kupplungsgliedes eignet. Der Druck im Reservoir kann infolge reduzierter Reibkräfte an der Dichtungsstelle genauer bestimmt werden, wodurch Okklusionen schneller und zuverlässiger erkannt werden können. Weiter soll die erfindungsgemässe Dosiervorrichtung in der Lage sein, teilbefüllte Reservoire aufzunehmen und auszustossen. Zur Reduktion der Therapiekosten soll die erfindungsgemässe Dosiervorrichtung aus Verbrauchsteilen und einem wiederverwendbaren Teil gebildet sein.

Vorzugsweise kann die erfindungsgemässe Dosiervorrichtung derart ausgestaltet sein, dass nach Kupplung zwischen einem neu befüllten Verbrauchsteil und dem wiederverwendbaren Teil die Spindelvorrichtung gegen den Anschlag des Kupplungsglieds zur Aufhebung eines Längsspiels entgegen der Vortriebsrichtung ausfahrbar ist und / oder bei Anliegen der Spindelvorrichtung am Anschlag in Vortriebsrichtung ausfahrbar ist. Grundsätzlich ist es denkbar, dass bei der Kupplung zwischen einem neu befüllten Reservoir und dem wiederverwendbaren Teil kein Längsspiel vorhanden ist. In diesem Fall stösst die Spindelvorrichtung bei der Kupplung bereits an den Anschlag und ist von diesem lediglich in Vortriebsrichtung ausfahrbar. Bei diesem Spezialfall ist das Reservoir ganzbefüllt und die Spindelvorrichtung verfährt lediglich in Vortriebsrichtung.

Vorteilhafte Ausgestaltungen der Erfindung sind in den Ansprüchen 2 bis 52 definiert.

Vorteilhafterweise wird das Kupplungsglied als profilierte Mitnehmerstange mit dem Anschlag für die Spindelvorrichtung ausgebildet. Besonders vorteilhaft ist es, wenn bei der Kupplung zwischen dem mindestens einen teil- oder ganzbefüllten Verbrauchsteil und dem wiederverwendbaren Teil die Mitnehmerstange des wiederverwendbaren Teils in ein axiales Langloch der Spindelvorrichtung des Verbrauchsteils einführbar ist und durch Ausziehen der Mitnehmerstange vom axialen Langloch der Verbrauchsteil vom wiederverwendbaren Teil entkuppelbar ist. Die Mitnehmerstange als Kupplungsglied kann besonders gut von Verunreinigungen gereinigt werden. Die Spindeltriebe der Spindelvorrichtungen werden generell aus Spindelmuttern und Spindelstangen gebildet, wobei beispielsweise bei einer einstufigen Spindelvorrichtung die Spindelmutter das Innengewinde und die Spindelstange das Aussengewinde aufweisen können. Das von der Antriebsvorrichtung angetriebene Element der Spindelvorrichtung wird als Antriebsstufe bezeichnet und kann beispielsweise eine Spindelmutter oder eine Spindelstange sein. Das verfahrbare Element der Spindelvorrichtung wird als Verschiebestufe bezeichnet und kann beispielsweise eine Spindelmutter oder eine Spindelstange sein. Definitionsgemäss wird das Langloch an der Antriebsstufe ausgebildet, so dass bei der Kupplung die Mitnehmerstange der Antriebsvorrichtung in das Langloch der Antriebsstufe der Spindelvorrichtung eingeführt wird. Die Antriebsstufe wird also rotatorisch angetrieben und kann axial verfahrbar oder axial nicht verfahrbar sein. Beispielsweise kann eine Spindelmutter rotatorisch angetrieben sein und eine korrespondierende Spindelstange einer einstufigen Spindelvorrichtung ausfahrbar sein, so dass die Spindelmutter als Antriebsstufe dient, aber axial nicht verschiebbar ist. Denkbar ist auch, dass eine Spindelmutter axial und rotatorisch fest angeordnet ist, wobei die korrespondierende Spindelstange rotatorisch angetrieben sein kann und so als Antriebsstufe ausgebildet sein kann und zudem ausfahrbar ist. Nach der Kupplung mit einem neubefüllten Verbrauchsteil, ist die Antriebsstufe mit der Mitnehmerstange drehfest verbunden. Beim Drehen der Mitnehmerstange durch die Antriebseinheit wird die Antriebsstufe drehend angetrieben. Axial verfährt die Antriebsstufe erst entgegen der Vortriebsrichtung und somit relativ zur Mitnehmerstange bis die Spindelvorrichtung an den Anschlag anschlägt. Nach dem Auffahren verfährt die Spindelvorrichtung in Vortriebsrichtung. Die Spindelvorrichtung der erfindungsgemässen Dosiervorrichtung kann einen Spindeltrieb aufweisen und so einstufig ausgeführt sein, sie kann aber auch teleskopisch ausgeführt sein und zwei oder mehr Spindeltriebe umfassen, um derart eine besonders kompakte Dosiervorrichtung zu bilden. Für eine teleskopische Spindelvorrichtung mit zwei Spindeltrieben kann die Antriebsstufe aus zwei Hülsen gebildet sein, die miteinander an einer Stirnseite, vorzugsweise an den dem Kolben zugewandten Stirnseiten, verbunden sein können. An der äusseren Hülse können beispielsweise ein Aussen- und ein Innengewinde angeordnet sein. Die innere Hülse kann das Langloch für die Mitnehmerstange aufweisen. Das Aussengewinde kann in Eingriff mit einem Innengewinde einer ersten Verschiebestufe sein und das Innengewinde kann in Eingriff mit einem Aussengewinde einer zweiten Verschiebestufe stehen.

Da die Spindelvorrichtung nach der Befüllung erfindungsgemäss im eingefahrenen Zustand ist, muss die Mitnehmerstange eine definierte Länge aufweisen, damit ein teilbefülltes Reservoir über die Mitnehmerstange kuppelbar ist. Vorteilhaft ist es, wenn die Länge der Mitnehmerstange gleich gross oder grösser als ein Verfahrweg V eines Spindeltriebs ist. Dadurch kann sichergestellt werden, dass teilbefüllte Reservoire mit dem wiederverwendbaren Teil kuppelbar sind und ausgeschüttet werden können. Besteht beispielsweise eine Spindelvorrichtung aus lediglich einem Spindeltrieb mit einem Verfahrweg V, so sind anfängliche Füllstände von 0% bis 100 % für das Reservoir zulässig. Weist die Spindelvorrichtung hingegen eine Teleskopspindel mit zwei Spindeltrieben mit je einem Verfahrweg V auf, so sind Füllstände von 50% bis 100 % zulässig und ausschüttbar bei einer Mitnehmerstange mit einer Länge V. Die Länge der Mitnehmerstange richtet sich also nach dem anfänglich gewünschten minimalen Füllstand, wobei auch die Anzahl der Spindelstufen der Spindelvorrichtung einen direkten Einfluss auf den minimalen Füllstand des Reservoirs hat. Der minimale Füllstand kann für einstufige Spindelvorrichtungen generell Werte zwischen 0% und 100% annehmen, die Mitnehmerstange ist dabei dem gewünschten minimalen Füllstand anzupassen. Für ein Reservoir mit einer minimalen, anfänglichen Befüllung muss bei der Kupplung die Spindelvorrichtung mit der Mitnehmerstange noch kuppelbar sein. Aus dieser Vorgabe kann die Länge der Mitnehmerstange bestimmt werden. Die Länge der Mitnehmerstange definiert also den minimalen Füllstand, der von der Dosiervorrichtung ausschüttbar ist. In der Insulinbehandlung sind minimale Füllstände von 50% bis 100% wünschenswert, damit jeder Anwender die für ihn optimale Menge an Medikamentenfluid für beispielsweise drei Anwendungstage zur Verfügung hat. Die Mitnehmerstange der Dosiervorrichtung wird bei der Kupplung in das Langloch der Antriebsstufe eingeführt. Das Langloch kann an einer Spindelstange oder einer Spindelmutter ausgebildet sein. Vorzugweise ist das Langloch an der Spindelstange ausgebildet, womit die Spindelstange bei Förderung drehend angetrieben ist; die Spindelmutter wird in diesem Fall drehfest ausgebildet. Natürlich ist hier auch eine Umkehrung denkbar, bei welcher die Spindelmutter ein Langloch aufweist, in welches das Kupplungsglied der Antriebsvorrichtung einführbar ist. In diesem Fall ist die Spindelmutter drehend angetrieben und die Spindelstange drehfest ausgebildet. Das Kupplungsglied kann beispielsweise als Gabel ausgebildet sein, deren Zinken parallel zur Achse der Spindelvorrichtung verlaufen können. Vorzugsweise wird die Spindelstange konzentrisch mit dem Abgangsrad und der Spindelvorrichtung ausgebildet, womit alle Komponenten, das heisst, der Kraftsensor, das Abgangsrad, die Mitnehmerstange, die Spindelvorrichtung, der Kolben und das Reservoir auf einer Achse liegen. Generell sind Anwendungsfälle denkbar, bei welchen die Dosiervorrichtungen stets mit ganzbefüllten Reservoiren beladen werden. Bei derartigen Anwendungsfällen genügt es, die Mitnehmerstange als kurzes Kupplungsglied auszugestalten.

Grundsätzlich kann der Kolben selbst als Verschiebestufe dienen, dabei kann der Kolben eine fest mit dem Kolben verbundene Spindelstange oder eine fest am Kolben ausgebildete Spindelmutter aufweisen. Vorteilhaft ist eine Ausgestaltung des Kolbens mit einer Spindelmutter, wobei die Spindelmutter Teil der Kolbenwandung sein kann. Dadurch lässt sich eine flachere Dosiervorrichtung bilden als bei der Ausführungsvariante mit einer am Kolben fest angeordneten Spindelstange. Zudem ist es wünschenswert, wenn der Spindeltrieb einen möglichst grossen Durchmesser aufweist, denn dadurch lässt sich die Knickneigung der Spindelvorrichtung reduzieren. Spindeltriebe mit einem geringen Durchmesser weisen eine grössere Knickneigung auf als Spindeltriebe mit einem grösseren Durchmesser. Um einen möglichst flachen Kolben zu bilden und die Knickneigung der Spindelvorrichtung zu minimieren, ist es vorteilhaft, wenn der Kolben als Verschiebestufe ein Innengewinde aufweist, welches Teil der Kolbenwandung sein kann. Der Kolben mit seinem Innengewinde dient somit als Spindelmutter und weist einen günstig grossen Durchmesser für den Spindeltrieb auf. Durch die reduzierte Knickneigung kann der Kolben bei der Abgabe von Medikamentenfluid genauer vorgetrieben werden, da ein Walken des Kolbens reduziert werden kann. Bei der Umkehrvariante, bei welcher die Spindelstange fest am Kolben angeordnet ist, ergeben sich sowohl hinsichtlich der Grösse des Kolbens als auch hinsichtlich der Knickneigung des Spindeltriebes Nachteile. Eine Ausgestaltung des Kolbens als Spindelmutter bietet daher Vorteile in Grösse, Reduktion von Knickneigung für die Spindelvorrichtung und Reduktion von Walken des Kolbens. Ein Aufbau der Spindelvorrichtung mit einer zentrischen Mitnehmerstange, welche in ein zentrisches Langloch einer Antriebsstufe einführbar ist und die Antriebsstufe mindestens mit der am Kolben angeordneten Spindelmutter gewindeverbunden sein kann, ist besonders vorteilhaft. Der Kolben dient dabei als erste Verschiebestufe. Die Antriebsstufe kann über eine zweite Gewindeverbindung mit einer zweiten Verschiebestufe verbunden sein, so dass eine teleskopische Spindelvorrichtung gebildet werden kann. Die besagte Anordnung ist anderen Anordnungen hinsichtlich Grösse zur Bildung von flachen auf dem Körper zu tragenden Dosiervorrichtungen überlegen und weist beste Eigenschaften hinsichtlich Knickfestigkeit auf.

Sowohl für das entgegen der Vortriebsrichtung gerichtete Ausfahren als auch für das in Vortriebsrichtung gerichtete Ausfahren weist die Antriebsvorrichtung einen gleichen Drehsinn auf. Dadurch können die Komplexität der Dosiervorrichtung vereinfacht und Kosten reduziert werden. Beispielsweise lässt sich die Steuerung der Antriebsvorrichtung vereinfachen, da die Antriebsvorrichtung stets nur in eine Richtung betrieben wird. Geräte der ersten Generation benötigen eine Soft- und Hardware, die einen Motorbetrieb in beide Drehrichtungen ermöglichen, da die permanent am wiederverwendbare Teil angeordnete Spindelvorrichtung stets wieder in ihren Ausgangszustand zurückgefahren werden muss. Ein Zurückfahren der Spindelvorrichtung in den eingefahrenen Zustand entfällt für die erfindungsgemässe Dosiervorrichtung, da die Spindelvorrichtung an dem mindestens einen Verbrauchsteil angeordnet ist. Dadurch kann die für ein Zurückfahren notwendige Energie eingespart werden. Vorzugweise ist die Spindelvorrichtung am Kolben angeordnet. Bei der Befüllung kann der Kolben zusammen mit der Spindelvorrichtung verschoben werden. Der Anwender kuppelt den Kolben mit einer Aufziehstange für das Befüllen des Reservoirs mit Medikamentenfluid. Dadurch, dass die Spindelvorrichtung am Kolben integriert ist, kann der Anwender die Befüllung derart vornehmen, wie er es von den Geräten der ersten Generation gewohnt ist und es ihm vertraut ist. Bei diesen wird der im Reservoir befindliche Kolben über eine Aufziehstange in beide Richtungen zur Befüllung verschoben. Ein für den Anwender ungewohntes Verschieben einer Spindelstange relativ zu einer Spindelmutter wie dies der Stand der Technik der zweiten und dritten Generation vorschlägt, kann durch die erfindungsgemässe Dosiervorrichtung vermieden werden. Der Anwender bevorzugt eine ihm bekannte und naheliegende Handhabung, bei welcher er den Kolben im Reservoir vorzugsweise über die Aufziehstange verschieben kann. Die Spindelvorrichtung kann am Kolben derart integriert sein, dass der Anwender lediglich den Kolben, nicht aber die Spindelvorrichtung wahrnimmt. Die Spindelvorrichtung ist für jedes neue Reservoir stets über einen konstanten Gesamthub H ausfahrbar, unabhängig davon, ob das Reservoir teil- oder ganzbefüllt ist. Der Gesamthub H setzt sich so aus einem rückwärtigen Hub H1 zur Aufhebung des Längsspiels und einem in Vortriebsrichtung gerichteten Hub H2 zur Verabreichung von Medikamentenfluid zusammen. Weiter ist es vorteilhaft, wenn die Antriebsvorrichtung einen Motor und ein vom Motor angetriebenes Getriebe umfasst, wobei ein letztes Getriebezahnrad als das Abgangsrad ausgestaltet sein kann. Vorzugsweise werden elektrische Motoren eingesetzt, welche in diskreten Motorschritten verfahren werden können. Beim Auffahren der Spindelvorrichtung an den an der Mitnehmerstange ausgebildeten Anschlag kann eine Reaktionskraft vom Kraftsensor gemessen und an die Steuereinheit geleitet werden und darauf der Motor der Antriebseinheit durch die Steuereinheit in Stillstand gesetzt werden. Die Anzahl der Motorschritte bis zum Auffahren an den Anschlag werden von einer Gesamtmotorschrittzahl für den Gesamthub H subtrahiert und so eine verbleibende Motorschrittanzahl für die Abgabe von Medikamentenfluid ermittelt. Weiter kann die verbleibende Motorschrittanzahl mit einer Proportionalitätskonstante multipliziert werden und daraus die Menge an Medikamentenfluid im Reservoir bestimmt werden. Der verwendete Motor kann beispielsweise ein bürstenloser Gleichstrommotor sein, der über Hallsensoren gesteuert werden kann. Die Rückmeldung einer Rotorlage kann über drei um 120 Winkelgrad versetzte Hallsensoren erfolgen. Die um 120 Winkelgrad versetzten Hallsensoren liefern pro Umdrehung sechs verschiedene Schaltkombinationen. Der Motor kann drei Teilwicklungen umfassen, die entsprechend den Sensorinformationen in sechs verschiedenen Leitphasen bestromt werden können, wodurch der Motor pro Umdrehungen sechs Motorschritte aufweist. Strom- und Spannungsverlauf können blockförmig sein. Die Steuerung überwacht über die Hallsensoren die Motorschritte, zudem kann sie die Motorschritte auch zählen. Die drei Hallsensoren ermöglichen es, eine Umdrehung des Motors in sechs diskreten Motorschritten zu 60 Winkelgrad zu erfassen. Die Rotorlage des Motors kann aber auch über ein am Rotor angeordnetes Flügelrad bestimmt werden. Das Flügelrad kann einen von einer Lichtquelle ausgehenden Lichtstrahl unterbrechen oder zu einem Detektor durchlassen. Aus dem alternierend durchgehenden und unterbrochenen Lichtstrahl kann die Steuerungseinheit die Lage des Rotors des Motors bestimmen. Aus dem Stand der Technik sind weitere antreibende Mittel für die Antriebseinheit bekannt wie beispielsweise Gedächtnisformlegierungen. Den antreibenden Mitteln ist gemein, dass diese kleinste Winkelinkremente aufweisen, welche proportional zu kleinsten Dosierinkrementen der Dosiervorrichtungen sind. Das kleinste Dosierinkrement oder die Dosierauflösung der Dosiervorrichtung ist eine für jede Dosiervorrichtung charakteristische Grösse.

Weiter ist es besonders vorteilhaft, wenn das Gehäuse aus drei Gehäuseteilen gebildet wird, wobei ein erster Gehäuseteil mindestens die Steuereinheit und / oder eine Energiequelle aufnimmt, ein zweiter Gehäuseteil als Chassis ausgebildet ist und dabei mindestens den Motor, das Getriebe und das als Mitnehmerstange ausgebildete Kupplungsglied aufnimmt und ein dritter Gehäuseteil als Getriebeboden ausgebildet ist. Grundsätzlich kann der Kraftsensor am Getriebeboden abgestützt sein. Vorteilhafter ist aber, die Basis für den Kraftsensor durch eine am Chassis befestigbare Platte zu bilden. Dadurch kann erreicht werden, dass der Motor, das Getriebe und das Kupplungsglied in Form der Mitnehmerstange, sowie der Kraftsensor am Chassis direkt angeordnet werden können. Dadurch kann eine besonders kompakte Einheit gebildet werden, an welcher alle wesentlichen Komponenten aufgenommen werden können. Zudem wird die Reaktionskraft an der Spindelvorrichtung nicht an den Getriebeboden geleitet sondern von der am Chassis befestigbaren Platte aufgenommen. Die Platte kann beispielsweise aus Stahl gefertigt sein und an zwei am Chassis ausgebildeten, quer zur Vortriebsrichtung verlaufenden Führungen befestigt sein. Der Kraftsensor kann sich dabei direkt an der Platte abstützen. Das Aufteilen des Gehäuses in drei Gehäuseteile ist besonders vorteilhaft, denn es erlaubt an jedem Gehäuseteil jeweilige Komponenten der Dosiervorrichtung vorzumontieren. Nach der Vormontage werden die drei Gehäusebauteile miteinander verbunden. Generell ist die Mitnehmerstange starr mit dem Abgangsrad verbunden, wobei ein O-Ring als Dichtungselement dienen kann und dieser zwischen dem Abgangsrad und der Mitnehmerstange an einem Schaft angeordnet sein kann. Bei der Montage wird erst der O-Ring an seiner am Schaft ausgebildeten Dichtungsstelle montiert. Nachfolgend wird die aus dem Abgangsrad, dem Schaft, dem O-Ring und der Mitnehmerstange gebildete Einheit von einer Unterseite in das Chassis eingefahren. Schliesslich kann der Kraftsensor unter dem Abgangsrad des Getriebes platziert werden und zur Sicherung des Kupplungsgliedes und des Kraftsensors die Stahlplatte mit seiner am Chassis ausgebildeten Führung in Eingriff gebracht werden. In einem ersten Montageschritt wird das ganze Chassis samt der am Chassis befestigbaren Komponenten vormontiert. Die fertigmontierte Chassis-Einheit kann dabei das Chassis, das aus Abgangsrad und Mitnehmerstange gebildete Kupplungsglied, den Kraftsensor, die Stahlplatte, Zahnräder des Getriebes und einen Motor mit beispielsweisen Hallsensoren für die Ansteuerung umfassen. Weiter ist es vorteilhaft, wenn das Chassis den ersten und den dritten Gehäuseteil aufnehmen kann. Wiederum kann die Batterie und die Steuerung vorgängig am ersten Gehäuseteil vormontiert werden. Die Dosiervorrichtung besteht also vorzugweise aus drei Gehäusebauteilen. Bauteile der Dosiervorrichtung können durch Vormontagen am ersten und zweiten Gehäuseteil vormontiert werden. Überdies nimmt der Getriebeboden keine Reaktionskräfte der Spindelvorrichtung auf, wodurch die Dosiervorrichtung weiter verbessert werden kann. Die Gehäuseaufnahme des Getriebebodens am Chassis ist durch diese Bauweise kräftefrei, die Langlebigkeit dieser Verbindung kann so erhöht werden. Auch die Gehäuseaufnahme des ersten Gehäuseteils am Chassis ist frei von inneren Kräften. Die besagte Anordnung der Gehäuseteile ermöglicht eine gut verbindbare, von Kräften freie und gut abdichtbare Aufnahme der Gehäuseteile am Chassis.

Vorteilhaft ist es, den mindestens einen Verbrauchsteil und den wiederverwendbaren Teil über eine Bajonettverbindung miteinander zu verbinden. Besonders vorteilhaft ist es, die Bajonettverbindung am Chassis auszubilden. Weiter kann die Bajonettverbindung in Bezug zu einer axialen Verfahrachse für die Spindelvorrichtung auf in etwa der Höhe des Anschlags für die Spindelvorrichtung angeordnet sein. Damit der Anwender den mindestens einen Verbrauchsteil einfacher und sicherer mit dem wiederverwendbaren Teil verbinden kann, kann das Gehäuse eine axiale Längsführung für den mindestens einen Verbrauchsteil aufweisen, über welche die Spindelvorrichtung geführt mit dem Kupplungsglied des wiederverwendbaren Teils kuppelbar ist und über welche der mindestens eine Verbrauchsteil geführt einer Lagerstelle für die Bajonettverbindung zuführbar ist. Vorteilhaft wird die axiale Längsführung aus zwei zueinander gerichteten Nuten am Gehäuse gebildet, wobei der Verbrauchsteil zwei, jeweils in eine Nut eingreifende Nocken aufweist. Die am Gehäuse ausgebildete Längsführung zur Aufnahme des mindestens einen Verbrauchsteils ist sehr vorteilhaft. Der Anwender verbindet in einem ersten Schritt den mindestens einen Verbrauchsteil mit dem wiederverwendbaren Teil, indem er die radial nach aussen ragenden Nocken des Verbrauchsteils an der am Gehäuse ausgebildeten Längsführung einführt. Derart wird der Verbrauchsteil am Gehäuse radial gesichert und ist nur noch axial verschiebbar. Durch Verschieben entlang der Führung gegen die Lagerstelle kommt es erst zum Einführen der Mitnehmerstange am Langloch, wodurch die Antriebsvorrichtung mit der Spindelvorrichtung zur Übertragung der Antriebsleistung gekuppelt wird. Durch weiteres Verschieben des mindestens einen Verbrauchsteils werden schliesslich die Nocken zur als Bajonettverbindung ausgebildeten Lagerstelle geführt. Die Lagerstelle ist am Gehäuse ausgebildet, vorzugsweise wird sie direkt am Chassis ausgebildet. Die Nocken werden vorzugsweise bis an einen am Chassis ausgebildeten, axialen Anschlag verschoben, nachfolgend werden die Nocken durch Drehen des mindestens einen Verbrauchsteils um seine Längsachse in am Gehäuse ausgebildete Lagerschlitze der Bajonettverbindung eingedreht. Die Längsführung für die Nocken und die Lagerschlitze sind miteinander verbunden. In diesen kann sich der Verbrauchsteil geführt bewegen, was die Handhabung vereinfacht. Die Lagerschlitze der Bajonettverbindung sind so ausgelegt, dass die Nocken passgenau in die Schlitze eingeführt werden können. Der mindestens eine Verbrauchsteil wird derart am Gehäuse sowohl in Vortriebsrichtung wie auch entgegen der Vortriebsrichtung gehalten. Diese Art der Lagerung über eine Bajonettverbindung, bei welcher der mindestens eine Verbrauchsteil mit dem wiederverwendbaren Teil direkt am Gehäuse, vorzugsweise am Chassis verbunden wird, ist besonders vorteilhaft. Beispielsweise kann so verhindert werden, dass durch eine äussere Kraft der mindestens eine Verbrauchsteil relativ zum Chassis verschoben werden kann. Beim Stand der Technik wird das Reservoir oft an einem Gehäuse lediglich in Vortriebsrichtung abgestützt, beispielsweise an einem Adapter. Der Adapter des Standes der Technik ist generell am Pumpengehäuse fixierbar, stützt das Reservoir in Vortriebsrichtung ab und verbindet über beispielsweise eine Konnektierungsnadel das Reservoir mit einem Fluidpfad. Eine axiale, äussere Kraft auf den Adapter kann bewirken, dass bedingt durch kleinste, axiale Lagerspiele der Adapter und das an ihm abgestützte Reservoir sich relativ zum Pumpengehäuse bewegen können. Der Kolben des Reservoirs stützt sich hingegen an einer festen Spindelvorrichtung ab, so dass sich das Reservoir relativ zum Kolben bewegen kann und es so zu einer Abgabe von Medikamentenfluid kommen kann. Die Lagerung des Verbrauchsteils, respektive des Reservoirs über Nocken an einer am Chassis ausgebildeten Lagerstelle, wobei dies erfindungsgemäss in Form einer Bajonettverbindung vorzugsweise ausgeführt wird, kann diesen Nachteil des Standes der Technik beheben. Für Dosiervorrichtung des Standes der Technik, speziell der ersten Generation, müssen stets die Längenausdehnungen und Toleranzen des Gehäuses miteinbezogen werden, da sich das Reservoir bei diesen über den Adapter am Gehäuse abstützt. Bei diesen bekannten Ausführungen stützt sich die Spindelvorrichtung am hinteren Gehäuseboden ab und kann einen immer gleichen maximalen Hub ausfahren, der beispielsweise einer konstanten Anzahl an Motorschritten entsprechen kann. Das Reservoir hingegen stützt sich an der gegenüberliegenden Gehäuseinnenwand über beispielsweise den Adapter ab. Das Gehäuse selbst hat eine grosse Fabrikationstoleranz, speziell Ausführungen des Gehäuses, die mittels Kunststoffspritzgiessverfahren hergestellt werden, weisen grosse Toleranzen auf. Dies ist nachteilig und führt dazu, dass bei der maximalen Gehäuselänge ein Restvolumen im Reservoir verbleiben kann. Bei der minimalen Gehäuselänge vermag der Gesamthub der Spindelvorrichtung das gesamte, im Reservoir befindliche Medikamentenfluid gerade auszustossen. Durch eine derartige Dimensionierung wird vermieden, dass der Kolben am Ende der Ausschüttung in die Wandung des Reservoirs läuft und dabei einen falschen Alarm auslöst. Aus dieser Betrachtung wird ersichtlich, dass die Längstoleranz des Gehäuses einen massgeblichen Einfluss auf das Restvolumen hat. Die Längentoleranzen von Gehäusen können in der Regel mehrere Zehntelmillimeter betragen, dementsprechend kann das Restvolumen ungünstig gross ausfallen. Beim Stand der Technik, beispielsweise Insulinpumpen der ersten Generation, kann das Restvolumen bis 5% des Reservoirvolumens betragen. Für ein Reservoir mit 2 ml Volumen und der Verwendung von U100-Insulin kann so ein nicht nutzbares Restvolumen von 10 IU Insulin resultieren, wobei das Reservoir maximale 200 IU Insulin fasst. Die vorliegende Erfindung erschafft hier eine bessere Lösung. Dadurch, dass die Bajonettverbindung auf in etwa der gleichen Höhe angeordnet ist wie der Anschlag für die Spindelvorrichtung, hat hier die Längentoleranz des Gehäuses keinen Einfluss mehr. Lediglich die Längenausdehnungen des Reservoirs und der Spindelvorrichtung haben einen direkten Einfluss auf das Restvolumen an Medikamentenfluid nach der Medikamentenabgabe. Die erfindungsgemässe Dosiervorrichtung schafft es daher, das Restvolumen an Medikamentenfluid am Ende der Medikamentenabgabe zu minimieren und zu reduzieren. Dies wird dadurch erreicht, dass die Lagerung des Reservoirs als Verbrauchsteil auf in etwa der Höhe der Abstützung der Spindelvorrichtung erfolgt, wodurch der Einfluss der Längentoleranz des Gehäuses eliminiert werden kann. Mathematisch betrachtet bedeutet dies, dass bei einer Toleranzanalyse die Toleranz des Gehäuses nicht in die Berechnung einfliesst. Lediglich die Toleranzen des Reservoirs und der Spindelvorrichtung fliessen in eine sogenannte Toleranzkette ein. Das bedeutet weiter, dass die die grösste Toleranz aufweisende Komponente, das heisst die Gehäuselänge, mittels der erfindungsgemässen Ausgestaltung aus der Toleranzkette eliminiert werden kann, wodurch ein bedeutender Fortschritt erzielt werden kann. Das Zusammenbringen des mindestens einen Verbrauchsteils mit dem wiederverwendbaren Teil über eine Längsführung und eine Bajonettverbindung stellt eine eigene Erfindung dar. Sowohl die Handhabung kann verbessert werden, weiter kann das nicht nutzbare Restvolumen reduziert werden.

Die Dosiervorrichtung kann einen Verbrauchsteil aufweisen, sie kann aber auch aus mehreren Verbrauchsteilen gebildet sein. Sehr vorteilhaft ist eine Bauweise mit zwei Verbrauchsteilen. Für eine Dosiervorrichtung mit zwei Verbrauchsteilen ist eine folgende Anordnung sinnvoll und vorteilhaft. Bei dieser besonders interessanten Ausführungsform umfasst der erste Verbrauchsteil das Reservoir und die am Kolben angeordnete Spindelvorrichtung. Der zweite Verbrauchsteil kann eine Grundplatte und einen Insertionskopf umfassen. Die Grundplatte kann eine auf das Gewebe flächig aufsetzbare und für eine Fixierung auf dem Gewebe vorbereitete Unterseite aufweisen. Dadurch kann die erfindungsgemässe Dosiervorrichtung direkt am Körper getragen werden, was für den Anwender besonders vorteilhaft ist. Der Insertionskopf kann fest mit der Grundplatte verbunden sein, alternativ und vorteilhaft ist eine Anordnung, bei welcher der Insertionskopf von einer gesicherten Ausgangsposition durch Aufbringen einer minimalen Auslösekraft in eine gesicherte Endposition gegen die Grundplatte geradverschiebbar ist, wobei die Verfahrrichtung des Insertionskopfs winklig zu der Verfahrachse der Spindelvorrichtung sein kann. Der Winkel zwischen der Verfahrachse der Spindelvorrichtung und der Verfahrrichtung des Insertionskopfes beträgt günstigerweise 90 Winkelgrad. Der Einführungswinkel kann aber weniger als 90 Winkelgrad aufweisen, beispielsweise 20 Winkelgrad bis 60 Winkelgrad für eine flache Einführung. Die fluidführende Verbindung zur subkutanen Verabreichung des Medikamentenfluids wird vorteilhafterweise am Insertionskopf angeordnet. Bei dieser besonders günstigen Ausführungsvariante umfasst der Insertionskopf ein Kanülengehäuse mit einer im Gewebe zu platzierenden Kanüle, einen zur Kanüle führenden Durchgangskanal für das Medikamentenfluid und zwei durch Septen ausgestaltete Begrenzungen des Durchgangskanals, wobei das eine Septum von einer am Reservoir angeordneten Konnektierungsnadel penetrierbar ist und das andere Septum sowie die Kanüle in der Ausgangsposition von einer Einstichnadel in Verfahrrichtung des Insertionskopfes durchdrungen sein kann. Grundsätzlich ist auch eine Umkehrung der Platzierungen von Konnektierungsnadel und Septum denkbar. Beispielsweise kann das Reservoir ein Septum aufweisen und der Insertionskopf die Konnektierungsnadel zu Konnektierung mit dem Reservoir. Vorteilhafter ist eine Anordnung, bei welcher die Konnektierungsnadel am Reservoir angeordnet ist. Diese Anordnung bietet den Vorteil, dass nach dem Verbinden des ersten Verbrauchsteils mit dem wiederverwendbaren Teil, der Anwender ein Priming durchführen kann. Dabei kann die Spindelvorrichtung erst rückwärtig an ihren Anschlag verfahren, nachfolgend kann sie in Vorschubrichtung verfahren werden. Sobald der Anwender einen austretenden Tropfen mit Medikamentenfluid an der Konnnektierungsnadel beobachtet, kann er das Priming über die Steuerung abbrechen. Ist hingegen der Insertionskopf fest mit der Grundplatte verbunden, wie dies die besagte alternative Ausführungsform vorschlägt, so ist es günstig, die Konnektierungsnadel am Insertionskopf anzuordnen und das Reservoir mit einem durch die Konnektierungsnadel penetrierbaren Septum auszubilden. Bei dieser Variante verbindet der Anwender erst den ersten Verbrauchsteil mit dem wiederverwendbaren Teil und nachfolgend die derart gebildete Einheit mit dem zweiten Verbrauchsteil, wodurch die ganze Dosiervorrichtung für eine Platzierung am Körper vorbereitet ist. Vorgängig kann der Anwender noch ein Priming durchführen, bei welchem die ganze fluidführende Verbindung mit Medikamentenfluid befüllt wird. Nach dem Priming kann der Anwender die gesamte, aus den zwei Verbrauchsteilen und dem wiederverwendbaren Teil gebildete Dosiervorrichtung an einer Körperstelle aufbringen. Dabei wird erst die Einstichnadel in die Haut eingeführt und nachfolgend die klebende Unterseite der Grundplatte auf eine ebene Hautstelle aufgebracht und angeklebt.

Für die Geradverschiebung des Insertionskopfs kann die Grundplatte Führungsmittel aufweisen. Vorteilhaft werden die Führungsmittel in Form von mindestens einer profilierten Schiene ausgebildet. Die mindestens eine Schiene greift in eine Führungsbahn ein, die Führungsbahn kann vorteilhafterweise direkt am Kanülengehäuse ausgebildet sein. Nebst den Führungsmitteln sind für das Halten des Insertionskopfes Haltemittel notwendig. Vorzugsweise sind die Haltemittel an der Grundplatte angeordnet. Über die Haltemittel kann der Insertionskopf erst in der gesicherten Ausgangsposition und nach einem Auslösen in der gesicherten Endposition gehalten werden, wobei das Auslösen durch Aufbringen der minimalen Auslösekraft erfolgt und in der Endposition die Kanüle im Gewebe des Anwenders platziert ist. Vorzugsweise werden die Haltemittel aus mindestens einen an der Grundplatte angeordneten, flexiblen Schnapphaken gebildet. Der mindestens eine Schnapphaken kann am Kanülengehäuse an zwei Positionen einrasten und derart das Kanülengehäuse in der Ausgangsposition und der Endposition lagesichern. Der Insertionskopf kann weiter durch ein entfernbares Sperrelement in der Ausgangsposition gesichert werden. Das Sperrelement erleichtert dem Anwender die Handhabung und verhindert, dass beim Aufkleben des zweiten Verbrauchsteils an einer Körperstelle der Insertionskopf ungewollt gegen den Körper verschoben wird. Erst wenn der Anwender alle Handhabungsschritte ausgeführt hat, entfernt er das Sperrelement, anschliessend ist der Insertionskopf bedienbar. Das Auslösen des Insertionskopfes von seiner oberen Position in seine Endposition kann durch Aufbringen einer minimalen Auslösekraft erfolgen. Das Auslösen kann aber auch durch eine Hilfsvorrichtung ausgelöst werden, beispielsweise mittels einer Kolben-Feder Vorrichtung. Nach dem Auslösen befindet sich die Kanüle in ihrer Endposition im subkutanen Gewebe eines Anwenders. Durch ein Entfernen der Einstichnadel wird die Kanüle für die Infusion mit Medikamentenfluid freigegeben. Der gegen eine Grundplatte verschiebbare Insertionskopf mit einem Kanülengehäuse zur Platzierung einer Kanüle kann eine eigene Erfindung darstellen. Das Kanülengehäuse kann als Abdeckung noch eine Haube aufweisen. Sowohl die Führungsmittel als auch die Haltemittel können direkt am Kanülengehäuse des Insertionskopfes wirken.

Der zweite Verbrauchsteil weist vorzugsweise Mittel zur Aufnahme des mit dem ersten Verbrauchsteil verbundenen, wiederverwendbaren Teils auf. Dafür weist die Grundplatte mindestens eine Linearführung auf, welche als Linearführung für mindestens eine am Gehäuse ausgebildete Längsnut dient. Die Linearführung ist derart gerichtet, dass im gekuppelten Zustand die Konnektierungsnadel des ersten Verbrauchsteils mit dem ersten Septum fluchtet. Durch lineares Verschieben des Gehäuses an eine axiale, an der Grundplatte ausgebildete Anschlagsbasis kann das Septum von der Konnektierungsnadel penetriert werden. Dadurch wird erreicht, dass die Konnektierungsnadel das Septum geführt penetriert, wodurch Folgen wie Dichtigkeitsprobleme und dergleichen bei schlechter Pentrierung vermieden werden können. Besonders bevorzugt ist eine Ausführungsform, bei welcher die Grundplatte zwei separate, parallele Linearführungen aufweist, die an korrespondierenden, am Gehäuse ausgebildeten Längsnuten einführbar sind. Weiter ist es von Vorteil, wenn die erste Linearführung seitlich an der Grundplatte angeordnet ist und die zweite Linearführung parallel zur ersten Linearführung angeordnet ist. Dadurch kann eine besonders stabile Verbindung zwischen dem zweiten Verbrauchsteil und dem wiederverwendbaren Teil gebildet werden. Weiter ist es vorteilhaft, wenn erst nur eine Linearführung in Eingriff gebracht wird. Damit der Anwender das Gehäuse leicht mit dem zweiten Verbrauchsteil verbinden kann, wird das Gehäuse erst mit der seitlichen, ersten Linearführung gekuppelt und durch nachfolgendes Verschieben des Gehäuses entlang der seitlichen, ersten Linearführung, wobei dabei das Gehäuse auf der Grundplatte eben aufliegen kann, mit der zweiten Linearführung gekuppelt. Die Kupplung des Gehäuses mit dem zweiten Verbrauchsteil, wie sie hier beschrieben ist, ist besonders erfinderisch und kann selbst eine eigene Erfindung darstellen. Besonders hervorzuheben sind die intuitive Handhabung, bei welcher das Gehäuse nacheinander an zwei am Verbrauchsteil angeordneten Längsführungen in Eingriff gebracht wird und die nachfolgende, geradgeführte Verschiebung mit der einhergehenden Penetrierung des Septums durch die Konnektierungsnadel. Die vorgeschlagene Verbindung und Kupplung zwischen dem Gehäuse und dem zweiten Verbrauchsteil ist intuitiv und einfach durch den Anwender auszuführen, zudem ist eine geradgeführte Penetrierung des Septums durch die Konnektierungsnadel möglich, wodurch Dichtigkeitsprobleme zwischen dem Septum und der Konnektierungsnadel deutlich reduziert werden können.

Die Bajonettverbindung zwischen dem ersten Verbrauchsteil und dem wiederverwendbaren Teil wird vorzugsweise als Festlager ausgebildet und der erste Verbrauchsteil wird weiter am zweiten Verbrauchsteil über ein rotationsgesichertes Gleitlager gelagert. Das Gleitlager nimmt dabei keine axiale, in Vortriebsrichtung wirkende Kraft auf. Vorteilhafterweise weist das rotationsgesicherte Gleitlager mindestens eine durch eine Führung und einen in die Führung einführbaren Führungsstift gebildete Lagerpaarung auf. Besonders vorteilhaft ist es, wenn die Führung des Gleitlagers an der Anschlagsbasis der Grundplatte ausgebildet ist und der Führungsstift am ersten Verbrauchsteil angeordnet ist. Beim in Eingriff Bringen des ersten Verbrauchsteils mit dem zweiten Verbrauchsteil wird dabei erst der mindestens eine Führungsstift mit der mindestens einen Führung in Eingriff gebracht und nachfolgend das Septum von der geradgeführten Konnektierungsnadel penetriert. Vorzugsweise weist der erste Verbrauchsteil zwei Führungsstifte auf. Weiter ist eine Ausgestaltung günstig, bei welcher sich das Gehäuse des wiederverwendbaren Teils an der Anschlagsbasis des zweiten Verbrauchsteils abstützen kann. Die vorgeschlagene Lagerung, bei welcher der erste Verbrauchsteil über ein Festlager am wiederverwendbaren Teil und über die Gleitlagerung am zweiten Verbrauchsteil gelagert wird, ist besonders vorteilhaft. Das Festlager fixiert den ersten Verbrauchsteil in Vortriebsrichtung und entgegen der Vortriebsrichtung, so dass der erste Verbrauchsteil axial nicht verschiebbar ist, zudem kann es Querkräfte aufnehmen. Das Gleitlager zwischen dem ersten Verbrauchsteil und dem zweiten Verbrauchsteil kann hingegen keine axiale Kraft aufnehmen, hingegen kann das Gleitlager derart ausgestaltet sein, dass es eine Rotation des ersten Verbrauchsteils entlang seiner Längsachse verhindern kann. Weiter kann das Gleitlager derart ausgestaltet sein, dass es Kräfte quer zur Längsachse des ersten Verbrauchsteils aufnehmen kann. Mit einer derartigen erfinderischen Kombination für die Lagerung des ersten Verbrauchsteils kann erreicht werden, dass der erste Verbrauchsteil statisch bestimmt und nicht überbestimmt gelagert ist. Das Gleitlager verhindert eine Rotation und nimmt dabei ein Drehmoment auf, zudem nimmt es Lagerkräfte quer zur Längsachse auf. Es erlaubt aber eine relative Verschiebung zwischen dem ersten und zweiten Verbrauchsteil, bei welcher die Konnektierungsnadel relativ zum Septum lediglich axial verschoben werden kann. Dabei bleibt die fluidische Verbindung zwischen dem Reservoir und dem fluidführenden Kanal erhalten. Über die Bajonettverbindung wird eine axiale Einspannung, also in Vortriebsrichtung und entgegen der Vortriebsrichtung, erreicht. Weiter ist es vorteilhaft, das Gehäuse des wiederverwendbaren Teils an der Anschlagsbasis des zweiten Verbrauchsteils abzustützen. Dies ist besonders vorteilhaft, denn dadurch kann erreicht werden, dass äussere, am Gehäuse wirkende Kräfte, beispielsweise Druckkräfte bei der Bedienung lediglich dazu führen, dass sich das Gehäuse an der Anschlagsbasis abstützt. Da der erste Verbrauchsteil am zweiten Verbrauchsteil gleitgelagert ist, führt die relative Verschiebung des zweiten Verbrauchsteils zum wiederverwendbaren Teil zu keiner Verschiebung des ersten Verbrauchsteils relativ zum wiederverwendbaren Teil. Dies ist besonders günstig, denn dadurch wird erreicht, dass beim Zusammendrücken der beiden Teile - wiederverwendbarer Teil gegen zweiten Verbrauchsteil - keine Verschiebung des Kolbens relativ zu einer Wandung des Reservoirs erfolgt, wodurch Medikamentenfluid abgegeben werden könnte. Gerade beim Stand der Technik, beispielsweise die D-TRONplus Pumpe, wird das Reservoir über einen Adapter abgestützt. Der Adapter kann durch den Anwender im Rahmen seiner Lagerspiele gegen seinen axialen Anschlag verschoben oder zusammengedrückt werden. Dabei wir das Reservoir mitverschoben und verfährt relativ zum festen, durch die Spindelvorrichtung gestützten Kolben rückwärtig, wodurch eine Abgabe von Medikamentenfluid auftreten kann. Beispielsweise führen ein axiales Lagerspiel von 0.01 mm zwischen dem Adapter und dem Gehäuse und ein Kolbenquerschnitt von 100 mm^2 zu einer Abgabe von 0.1 IU Insulin bei der Verwendung von U100-Insulin. Klar ist ersichtlich, dass die erfindungsgemässe Lagerung über ein Festlager und ein Gleitlager hier einen wesentlichen Vorteil bietet, denn bei dieser kann eine derartige, ungewollte Medikamentenabgabe komplett vermieden werden. Die Lagerung des ersten Verbrauchsteils über ein Festlager und ein Gleitlager stellt somit eine weitere, eigenständige Erfindung dar, welche gegenüber dem Stand der Technik eine deutliche Verbesserung erreicht. Weiter kann zwischen dem wiederverwendbaren Teil und dem zweiten Verbrauchsteil eine lösbare, aus mindestens einer Nut und einer Nocke gebildete Verbindung zur Sicherung des Insertionskopfes in seiner Endposition vorhanden sein. Über diese zusätzliche Verbindung kann sichergestellt werden, dass der Insertionskopf in seiner Endposition, bei welcher die Kanüle im Gewebe eines Anwenders platziert ist, gehalten wird. Weiter kann das Sperrelement derart ausgebildet sein, dass der wiederverwendbare Teil am zweiten Verbrauchsteil erst nach Entfernen des Sperrelementes befestigbar ist. Hierzu kann beispielsweise die aus einer Nut und einer Nocke zu bildende Verbindung zur Sicherung des Insertionskopfes in seiner Endposition über das Sperrelement gesperrt sein, so dass die Nocke nicht an der Nut einfahrbar ist. Erst wenn das Sperrelement entfernt ist, ist die Nocke an der Nut einfahrbar.

Weiter soll die Dosiervorrichtung eine axiale Fixierung für eine axiale Fixierung des wiederverwendbaren Teils am zweiten Verbrauchsteil aufweisen. Die axiale Fixierung kann beispielsweise aus mindestens einer lösbaren Schnapphakenverbindung gebildet sein. Die Schnapphakenverbindung wird vorzugsweise durch einen am Gehäuse ausgebildeten Haken und eine für den Haken vorgesehene und an der Grundplatte ausgebildete Aussparung gebildet. Eine Umkehrung der Anordnung von Haken und Aussparung ist auch denkbar. Der Haken kann an einer Kante der Aussparung einhaken und den mit dem ersten Verbrauchsteil verbundenen wiederverwendbaren Teil axial sichern. Weiter kann das Gehäuse einen Bedienknopf aufweisen, durch dessen Betätigung die flexible Grundplatte in einem Bereich der Schnapphakenverbindung vom Gehäuse gespreizt werden kann, um dadurch die Schnapphakenverbindung zu lösen. Vorzugsweise kann die flexible Grundplatte weitgehend senkrecht zur Auflage am Gehäuse gespreizt werden. Der Bedienknopf kann generell am Gehäuse, sogar an einem Verbrauchsteil angeordnet sein. Besonders vorteilhaft ist eine Anordnung des Bedienknopfes am dritten Gehäuseteil, da dieser als Getriebeboden für Reparaturzwecke leicht austauschbar ist. Der Bedienknopf kann elastisch ausgebildet sein und einen keilförmig ausgestalteten Spreizkopf aufweisen. Bei Betätigung, das heisst durch elastische Auslenkung über eine vom Anwender ausgeübte Kraft, kann der keilförmige Spreizkopf an einer durch das Gehäuse und die Grundplatte gebildeten Trennlinie zur Spreizung der Grundplatte angreifen. Besonders vorteilhaft ist es, die Grundplatte an einem Eingriffsort des Spreizkopfs ebenfalls keilförmig auszubilden, so dass einander zugewandte, schiefe Kontaktebenen des Spreizkopfs und der Grundplatte parallel zueinander liegen, wodurch der Spreizkopf bei Betätigung die Grundplatte besonders gut vom Gehäuse trennen kann. Weiter ist es vorteilhaft, wenn der elastische Bedienknopf eine begrenzte Auslenkung aufweist. Dadurch kann erreicht werden, dass der Anwender den Bedienknopf nicht über ein definiertes, sicheres Mass auslenken kann und derart beschädigen kann. Eine Auslenkung über eine definierte Dehnungsgrenze kann die Lebensdauer dieses Bauteils reduzieren. Besonders vorteilhaft ist eine Ausgestaltung, bei welcher die Dosiervorrichtung zwei durch Bedienknöpfe zu lösende Schnapphakenverbindungen aufweist. Die Bedienknöpfe sollen bei dieser Ausgestaltung mit einer Hand gleichzeitig bedienbar sein. Dazu können die Bedienknöpfe an gegenüberliegenden Seitenflächen des Gehäuses angeordnet sein. Ziel ist es hier, dass der Anwender die Dosiervorrichtung mit einer Hand bedienen kann. Sowohl das in Eingriff bringen des wiederverwendbaren Teils mit dem am Körper aufgeklebten zweiten Verbrauchsteil, als auch das Lösen dieser Teile durch Betätigung der Bedienknöpfe soll mit einer Hand erfolgen. Die Bedienknöpfe sind so angeordnet und ausgelegt, dass der Anwender sie mit einem Daumen und einem Zeigefinger lösen kann. Zur weiteren Sicherung der Dosiervorrichtung kann eine weitere Schnapphakenverbindung zwischen dem Gehäuse und der Grundplatte vorgesehen sein. Vorzugsweise wird diese Verbindung durch eine minimale axiale Zugkraft am Gehäuse gelöst. Der Anwender betätigt zuerst die Bedienknöpfe über einen Daumen und einen Zeigefinger und löst dabei die zwei Schnapphakenverbindungen. Durch axiales Ziehen entlang der Längsführung generiert er nachfolgend die nötige Kraft, um die Schnapphakenverbindung zu lösen. Die Schnapphakenverbindung kann aus lediglich einem am Gehäuse ausgebildeten flachen Keil und einer an der elastischen Grundplatte für den Keil vorgesehenen Aussparung gebildet sein. Die hier beschriebene Ausgestaltung zur Fixierung des wiederverwendbaren Teils mit dem zweiten Verbrauchsteil über zwei Schnapphakenverbindungen und einer eventuellen Schnapphakenverbindung ist besonders vorteilhaft und stellt für sich eine eigene Erfindung dar. Hervorzuheben ist hier die intuitive Handhabung, wobei der Anwender die Fixierung wie auch das Lösen dieser Fixierung über eine Hand ausführen kann.

Weiter ist es besonders vorteilhaft, wenn die Abstützung und Lagerung des Reservoirs am Chassis nicht nur über die Bajonettverbindung erfolgt, sondern hier noch weitere Mittel vorhanden sind. Am Chassis kann hierzu mindestens ein Stützelement angeordnet sein. Beim Verschieben des Reservoirs zu seiner Lagerstelle entlang der Längsführung greifen die Stützelemente in das Reservoir ein. Dabei berühren die Stützelemente an ihrem äusseren Umfang die Innenwandung des Reservoirs und können so eine radiale Auslenkung des Reservoirs weiter einschränken. Nachfolgend wird das Reservoir in die Lagerschlitze eingedreht. Dabei greifen die Nocken in die Lagerschlitze und bilden derart die Bajonettverbindung. Die Innenwandung des Reservoirs ist fortan an den Stützelementen nicht mehr über den Umfang sondern entlang einer Kontaktlinie abgestützt. Eine äussere, auf das Reservoir wirkende Kraft wird so teilweise über die Bajonettverbindung an das Chassis geleitet, gleichzeitig wird ein Teil der beaufschlagten Kraft von den Stützelementen entlang der Kontaktlinien aufgenommen. Dadurch kann erreicht werden, dass die Spindelvorrichtung frei von Querkräften ist.

Weiter wird angemerkt, dass die Dosiervorrichtung, insbesondere der Insertionskopf auch einen Sensor zur Blutzuckermessung aufweisen kann. Besonders vorteilhaft ist es, den Sensor derart zu gestalten, dass der Sensor mit der fluidführenden Kanüle zusammen einen Bauteil bildet, so dass der Anwender nur eine Einstichstelle wahrnehmen kann.

## Kurze Beschreibung der Zeichnungen

Ein Ausführungsbeispiel der Erfindung wird nachfolgend anhand der Zeichnungen näher beschrieben, dabei zeigen:

Fig. 1a        eine erfindungsgemässe Dosiervorrichtung vor einer Kupplung mit einem ganzbefüllten, ersten Verbrauchsteil im Längsschnitt,

Fig. 1b          die in Fig. 1a dargestellte Dosiervorrichtung nach einer Kupplung im Längsschnitt,

Fig. 2a          eine erfindungsgemässe Dosiervorrichtung vor einer Kupplung mit einem teilbefüllten, ersten Verbrauchsteil im Längsschnitt,

Fig. 2b          die in Fig. 2a dargestellte Dosiervorrichtung nach einer Kupplung im Längsschnitt,

Fig. 2c          die in Fig. 2b dargestellte Dosiervorrichtung nach einem Auffahren einer Spindelvorrichtung an ihren Anschlag im Längsschnitt,

Fig. 2d          die in Fig. 2c dargestellte Dosiervorrichtung mit einer ganz ausge-fahrenen Spindelvorrichtung im Längsschnitt,

Fig. 3a - 3e     den Aufbau und die Montage des wiederverwendbaren Teils be-stehend aus drei Gehäuseteilen in 3d Darstellung,

Fig. 4a          den ersten, mit einer Aufziehstange verbundenen Verbrauchsteil in 3d Darstellung,

Fig. 4b          den in Fig. 4a dargestellten, ersten Verbrauchsteil in Explosionsdarstellung,

Fig. 5a          den zweiten Verbrauchsteil mit einem Sperrelement gesichert in 3d Darstellung,

Fig. 5b          den in Fig. 5a dargestellten zweiten Verbrauchsteil nach Entfernen des Sperrelementes vor einem Auslösen des Insertionskopfes in 3d Darstellung,

Fig. 5c          den in Fig. 5b dargestellten zweiten Verbrauchsteil in Explosionsdarstellung,

Fig. 5d          den in Fig. 5c dargestellten zweiten Verbrauchsteil nach dem Auslösen des Insertionskopfes in 3d Darstellung,

Fig. 5e          den in Fig. 5d dargestellten zweiten Verbrauchsteil nach dem Auslösen des Insertionskopfes im Längsschnitt,

Fig. 6a - 6c     die Handhabungsschritte zum Verbinden des ersten Verbrauchsteils mit dem wiederverwendbaren Teil in 3d Darstellung,

Fig. 6d - 6f     die Handhabungsschritte für den zweiten Verbrauchsteil im Schnitt quer zur Vortriebsrichtung dargestellt,

Fig. 6g - 6j     die Handhabungsschritte zum Verbinden des mit dem ersten Verbrauchsteil verbundenen wiederverwendbaren Teils mit dem zweiten Verbrauchsteil in 3d Darstellung,

Fig. 6k          die in Fig. 6i gezeigte Dosiervorrichtung im Schnitt quer zur Vortriebsrichtung, Schnitt A-A,

Fig. 6l          die in Fig. 6i gezeigte Dosiervorrichtung im Schnitt längs zur Vortriebsrichtung , Schnitt B-B,

Fig. 7a - 7c     die in Fig. 6i gezeigte Dosiervorrichtung, wobei hier ein Bedienknopf betätigt und nicht-betätigt in 3d Darstellung und im Schnitt gezeigt sind,

Fig. 7c          die in den Fig. 7a und 7b gezeigte Dosiervorrichtung, wobei hier die Anordnung und die Betätigung der Bedienknöpfe verdeutlicht sind, und

Fig. 8a - 8b     eine vorteilhafte Ausgestaltung für die Lagerung des ersten Verbrauchsteil am wiederverwendbaren Teil.

## Wege zur Ausführung der Erfindung

Fig. 1a und Fig. 1b zeigen eine erfindungsgemässe Dosiervorrichtung D. Die erfindungsgemässe Dosiervorrichtung D wird aus Verbrauchsteilen und einem wiederverwendbaren Teil gebildet. Der wiederverwendbare Teil 2 umfasst ein Gehäuse 3, an welchem eine Elektronikleiterplatte 4 mit einer Steuerung, eine Batterie 5, ein Motor 6 und ein mit dem Motor 6 gekuppeltes Planetengetriebe 7 sowie Zahnräder 8 eines Stirnradgetriebes 9 zur Umlenkung angeordnet sind. Ein letztes Abgangsrad 14 des Stirnradgetriebes 9 ist über einen Schaft 10 mit einem Kupplungsglied ausgestaltet. Das Kupplungsglied ist als Mitnehmerstange 11 ausgebildet, beispielsweise mit einem Vierkantprofil, und weist einen Anschlag 12 für eine Spindelvorrichtung S auf. Das Abgangsrad 14 ist schwimmend, geradgelagert im Gehäuse 3 angeordnet, wobei am Schaft eine Dichtungsstelle ausgebildet ist. Die Dichtungsstelle kann über eine kleine Toleranzen aufweisende Führung für den Schaft gebildet sein. Im Ausführungsbeispiel der Fig. 1a und 1b ist die Dichtungsstelle in Form eines O-Rings 13 gebildet. Das Abgangsrad 14 stützt sich dabei über einen Kraftsensor 15 an einer festen Basis ab. In Fig. 1a sind der erste Verbrauchsteil 1 in Form eines Reservoirs A und der wiederverwendbare Teil 2 der Dosiervorrichtung D voneinander getrennt dargestellt. Der Anwender befüllt in einem ersten Handhabungsschritt das Reservoir A. Dabei kann er wie gewohnt den ersten Verbrauchsteil 1 über eine Aufziehstange 16 befüllen. Das in Fig. 1a dargestellte Reservoir A ist für eine Anwendung in einer Insulinpumpe geeignet. Es weist ein Volumen von 2 ml auf und kann so 200 IU Insulin mit einer Konzentration U100 fassen. Das Reservoir A weist hier eine Konnektierungsnadel 17 auf, die für die Konnektierung einer fluidführenden Verbindung F zwischen dem Reservoir A und dem Anwender vorgesehen ist. Damit der Anwender sich nicht an der Nadel 17 verletzen kann, weist das Reservoir A einen um die Nadel 17 angeordneten Schutzkragen 18 auf. Im Reservoir A ist ein über zwei O-Ringe 19 gehaltener Kolben K angeordnet. Am Kolben K selbst ist eine Spindelvorrichtung S angeordnet. Diese ist in Form einer Teleskopspindel ausgeführt und besteht aus zwei Verschiebestufen 20,21 und einer Antriebsstufe 22. Die erste Verschiebestufe 20 ist am Kolben K selbst ausgebildet, der Kolben weist daher ein Innengewinde 23 auf. Die zweite Verschiebestufe 21 weist ein Aussengewinde 24 auf und verfährt lediglich rückwärtig. Die Antriebsstufe 22 ist aus zwei zylindrischen Hülsen gebildet, die an einer Stirnseite miteinander verbunden sind. Vorzugsweise sind die Hülsen an der dem Kolben K zugewandten Stirnseite miteinander verbunden. Die Antriebsstufe 22 weist an der äusseren Hülse ein Aussengewinde 25 auf, das mit dem Innengewinde 23 der ersten Verschiebestufe 20 einen ersten Spindeltrieb bildet. Die äussere Hülse weist weiter ein Innengewinde 26 auf, das mit dem Aussengewinde 24 der zweiten Verschiebestufe 21 einen zweiten Spindeltrieb bildet. Die Innere Hülse weist ein profiliertes Langloch 27 auf, in welches die Mitnehmerstange 11 bei der Kupplung einführbar ist. Die gebildeten Spindeltriebe sind gegenläufig. Im gezeigten Ausführungsbeispiel beträgt die Spindelsteigung 0.5 mm/Umdrehung. Bei Förderung stösst sich die Antriebsstufe 22 von der zweiten Verschiebestufe 21 ab, wobei hier eine Abstossung von 0.5 mm/Umdrehung entsteht. Weiter stösst sich die erste Verschiebestufe 20 von der Antriebsstufe 22 um wiederum 0.5 mm/Umdrehung ab. Dadurch ergibt sich für den Kolben K eine Vorschubbewegung in Abhängigkeit der Umdrehung der Antriebsstufe 22 von 1.0 mm/Umdrehung. Das Reservoir A ist im Querschnitt oval ausgeführt. Sowohl für den Kolben K, respektive für die ersten Verschiebestufe 20, als auch für die zweite Verschiebestufe 21 dient eine Reservoirwandung 28 als Verdrehsicherungselement. Die zweite Verschiebestufe 21 weist hierzu radiale, nach aussen ragende Flügel 29 auf, die sich an einer Innenwandung 30 abstützen können. Die zweite Verschiebestufe 21 ist zudem mit der Aufziehstange 16 kuppelbar. Beim Befüllen kann der Anwender den Kolben K über die Aufziehstange 16 verschieben. Beim Befüllen wird generell das Reservoir A mit einem Vorratsbehälter gekuppelt, hierzu eignet sich beispielsweise die Konnektierungsnadel 17. Nachfolgend wird der Kolben K verschoben und im Reservoir A befindliche Luft in den Vorratsbehälter verdrängt. Durch rückwärtiges Verschieben des Kolbens K strömt Medikamentenfluid vom Vorratsbehälter in das Reservoir A. Dieser Vorgang kann, bis das Reservoir A den gewünschten Füllstand erreicht hat und das Reservoir A frei von Blasen ist, mehrfach wiederholt werden. In der Fig. 1a ist das Reservoir A ganzbefüllt, dementsprechend ist der Kolben K in seiner hinteren Position. Hervorzuheben ist hier, dass beim Befüllen der Kolben K zwar verschoben wird, jedoch die Spindelvorrichtung S in ihrem Ausgangszustand bei der Befüllung verbleibt. Das bedeutet, dass nach dem Befüllen die Spindelvorrichtung S in ihrer eingefahrenen, definierten Position bzw. Ausgangsposition ist, von welcher aus sie stets einen ganzen Gesamthub H verfahrbar ist. Das in Fig. 1a dargestellte Reservoir A weist an der Wandung 28 zwei nach aussen stehende Nocken 31 auf. Zum Verbinden des Verbrauchsteils 1 mit dem wiederverwendbaren Teil 2 werden die Nocken 31 erst mit zwei am Gehäuse 3 ausgebildeten Längsführungen 32 in Eingriff gebracht. Im Eingriff stehend ist der Verbrauchsteil 1 lediglich entlang der Führungen 32 axial verschiebbar. Durch Verschieben des Verbrauchsteils 1 gegen eine Lagerstelle wird die Spindelvorrichtung S mit einer Antriebsvorrichtung M gekuppelt. Die Antriebsvorrichtung M umfasst den Motor 6 samt Planetengetriebe 7, das Getriebe 9 und das Abgangsrad 14, an welchem die Mitnehmerstange 11 als Kupplungsglied für die Spindelvorrichtung S angeordnet ist. Die Antriebsvorrichtung M ist dem wiederverwendbaren Teil 2 zugeordnet, während die Spindelvorrichtung S dem Verbrauchsteil 1 zugeordnet ist. Konkret wird beim Längsverschieben des ersten Verbrauchsteils 1 die Mitnehmerstange 11 in das Langloch 27 der Spindelvorrichtung S eingeführt. Antriebsvorrichtung M und Spindelvorrichtung S sind so gekuppelt zur Übertragung einer Antriebsleistung an die Spindelvorrichtung S. Die rotative Antriebsleistung wird von der Spindelvorrichtung S in eine translatorische Hubleistung zum Verdrängen von Medikamentenfluid umgewandelt. Durch weiteres Verschieben gelangt der erste Verbrauchsteil 1 zu seiner Lagerstelle, an welcher die Nocken 31 des Verbrauchsteils 1 in Lagerschlitze 33 eingedreht werden. In dieser Position ist der Verbrauchsteil 1 mit dem wiederverwendbaren Teil 2 gekuppelt. Einerseits ist der Verbrauchsteil 1 fest mit dem wiederverwendbaren Teil 2 verbunden, dabei ist die Wandung 28 des Reservoirs A über die Nocken 31 fest an seiner am Gehäuse 3 ausgebildeten Lagerstelle gesichert. Sowohl ein Verschieben der Reservoirwandung 28 in Vortriebsrichtung als auch entgegen der Vortriebsrichtung wird durch die Lagerung verhindert. Die Lagerung über zwei in Schlitze 33 eingreifende Nocken 31 entspricht einer Bajonettverbindung. Weiter ist nach der Kupplung gemäss der Fig. 1b die Mitnehmerstange 11 im Langloch 27 eingeführt. Ein Drehen der Mitnehmerstange 11 durch die Antriebsvorrichtung M führt zu einem Drehen der Antriebsstufe 22 der Spindelvorrichtung S. Nach der Kupplung ist generell zwischen der zweiten Verschiebestufe 21 und dem an der Mitnehmerstange 11 ausgebildeten Anschlag 12 ein Längsspiel vorhanden. Durch Antreiben der Spindelvorrichtung S verfährt die zweite Verschiebestufe 21 erst rückwärtig gegen ihren Anschlag 12. Beim Anfahren an den Anschlag 12 wird eine Reaktionskraft erzeugt, die der Kraftsensor 15 an die Steuerung leitet. Die Steuerung setzt hierauf den Motor 6 in Stillstand. Das rückwärtige Verfahren gegen den Anschlag 12 entspricht einem Hub H1. Der verwendete Motor 6 kann ein bürstenloser Gleichstrommotor sein, der über Hallsensoren gesteuert werden kann. Die Rückmeldung einer Rotorlage erfolgt über drei um 120 Winkelgrad versetzte Hallsensoren. Die um 120 Winkelgrad versetzten Hallsensoren liefern pro Umdrehung sechs verschiedene Schaltkombinationen. Der Motor 6 kann drei Teilwicklungen umfassen, die entsprechend den Sensorinformationen in sechs verschiedenen Leitphasen bestromt werden können, wodurch der Motor pro Umdrehungen sechs Motorschritte aufweist. Strom- und Spannungsverlauf können blockförmig sein. Die Steuerung überwacht über die Hallsensoren die Motorschritte, zudem kann sie die Motorschritte auch zählen. Die drei Hallsensoren ermöglichen es, eine Umdrehung des Motors in sechs diskreten Motorschritten zu 60 Winkelgrad zu erfassen. Der Motor weist im Ausführungsbeispiel eine Winkelauflösung von 60 Winkelgrad auf. Kennt man also die Anzahl der Motorschritte vom Start bis zum Auffahren der zweiten Verschiebestufe 21 an ihren Anschlag 12, so kann daraus der Hub H1 berechnet werden. Hierfür muss die Anzahl Motorschritte $n_{Motor}$ mit 60 Winkelgrad multipliziert werden, daraus erhält man den gesamten Motorwinkel $n_{Motor} \cdot 60$. Dieser gesamte Motorwinkel muss nachfolgend durch die Untersetzung des Getriebes i geteilt werden, woraus man den Winkel des Abgangsrades $\frac{n_{Motor} \cdot 60}{i}$, respektive der Antriebsstufe 22 errechnen kann. Weiter kann der Winkel der Antriebsstufe 22 mit der Steigung des Gewindes $p$ mutipliziert werden, womit schliesslich der Hub H1 beim Zurückfahren berechnet werden kann. Dieser Hub H1 beträgt $\frac{n_{Motor} \cdot 60}{i} \cdot p$. Anhand der Motorschritte von der Ausgangsposition bis zum Auffahren an den Anschlag 12 kann so der Hub H1 bestimmt werden. Ausgehend von diesem bekannten Hub H1, kann weiter der Füllstand berechnet werden. Hierzu wird der Hub H1 vom Gesamthub H der Spindelvorrichtung S subtrahiert. Man erhält somit den Hub H2, der für eine Abgabe von Medikamentenfluid noch zur Verfügung steht. Der Füllstand ergibt sich daher aus dem ganzen Reservoirvolumen $V_0$ multipliziert mit dem Verhältnis von H2/H. Der anfängliche Füllstand $V_1$ kann also mit der folgenden Formel berechnet werden.

$$V_1 = \frac{H - \frac{n_{Motor} \cdot 60}{i} \cdot p}{H} \cdot V_0 = \frac{H - H_1}{H} \cdot V_0$$

Ausgehend vom anfänglichen Füllstand $V_1$ des Reservoirs und den weiter bekannten, durch die Steuerung erfassbaren Motorschritte bei der Medikamentenabgabe, kann stets der momentane Füllstand des Reservoirs berechnet werden. Unterschreitet der Füllstand ein Minimum, kann vorteilhaft der Anwender über den bevorstehenden Wechsel des Reservoirs alarmiert werden. Der Anwender kann beispielsweise über eine Steuerungseinheit zur Steuerung der Dosiervorrichtung, welche beispielsweise an einem Blutzuckermessgerät angeordnet sein kann, stets den momentanen Füllstand des Reservoirs abfragen. Das kleinste Dosierinkrement $V_{Inkrement}$ entspricht dem Volumen, das beim Verfahren des Motors um einen Motorschritt ausgestossen wird. Hierzu wird der Hub mit der Kolbenquerschnittsfläche $\frac{\pi \cdot D_K^2}{4}$ multipliziert. Das Volumen des kleinsten Dosierinkrementes entspricht daher der folgenden Gleichung.

$$V_{Inkrement} = \frac{1 \cdot 60}{i} \cdot p \cdot \frac{\pi \cdot D_K^2}{4}$$

Teilt man das Volumen des Reservoirs durch das Volumen des kleinsten Dosierinkrementes so erhält man schliesslich die konstante Anzahl an Dosierinkrementen oder Motorschritten zum Ausstossen eines Reservoirs.

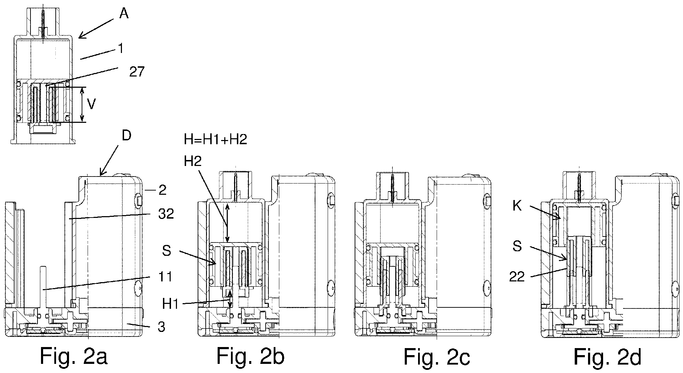

In Fig. 2a bis 2d ist eine Abfolge dargestellt, bei welcher ein teilbefülltes Reservoir A mit dem wiederverwendbaren Teil 2 der Dosiervorrichtung D verbunden wird. Eine fluidführende Verbindung F zwischen dem Reservoir A und einer Injektionsstelle ist hier nicht gezeigt. Ausgehend von der Fig. 2a kann die erfindungsgemässe Dosiervorrichtung D auch teilbefüllte Reservoire aufnehmen. Die Handhabung ist für den Anwender analog der Handhabung für ein ganzbefülltes Reservoir A. In einem ersten Handhabungsschritt wird das teilbefüllte Reservoir A mit dem wiederverwendbaren Teil 2 verbunden. Über die Längsführung 32 wird das Reservoir A seiner am Gehäuse 3 ausgebildeten Lagerstelle zugeführt. Dabei wird die Mitnehmerstange 11 in das Langloch 27 der Antriebsstufe 22 eingeführt. Nach der Kupplung gemäss Fig. 2b ist das teilbefüllte Reservoir A mit dem wiederverwendbaren Teil 2 gekuppelt. Hierzu ist das Reservoir A über die Bajonettverbindung mit dem Gehäuse 3 verbunden. Bei Teilbefüllung ist die Überschneidung zwischen der Mitnehmerstange 11 und dem Langloch 27 geringer als bei Ganzbefüllung. Das in Fig. 2b gezeigte Ausführungsbeispiel weist für die Mitnehmerstange 27 ausgehend vom Anschlag 12 ungefähr eine Länge von einem Verfahrweg V eines Spindeltriebs auf. Hiernach beträgt die minimale anfängliche Befüllung 50% der Ganzbefüllung. Dies bedeutet, dass ein Reservoir A, welches lediglich zu 50% befüllt ist, gerade noch kuppelbar ist. Dabei kommt es gerade noch zu einer Kupplung zwischen der Mitnehmerstange 11 und der Antriebsstufe 22. In den Fig. 2a und 2b ist zudem ersichtlich, dass nach der Befüllung die Spindelvorrichtung S in ihrer eingefahrenen Position ist, wie dies die Erfindung voraussetzt. Nach der Kupplung verfährt die Spindelvorrichtung S erst rückwärtig an ihren Anschlag 12 und verfährt dabei den Hub H1. Die Steuereinheit kann aus den Motorschritten den Hub H1 berechnen und daraus den anfänglichen Füllstand automatisch bestimmen. Ausgehend von diesem anfänglichen Füllstand kann nun für die Ausschüttung stets der momentane Füllstand berechnet werden, da die Steuereinheit stets die weiter verfahrenen Motorschritte bei der Abgabe von Medikamentenfluid kennt. Am Ende der Ausschüttung ist der Kolben K in seiner ausgefahrenen Position der Fig. 2d. Die teleskopische Spindelvorrichtung S ist nun ganz ausgefahren. Zwischen der Mitnehmerstange 11 und der Antriebsstufe 22 ist nur noch ein minimaler Eingriff vorhanden.

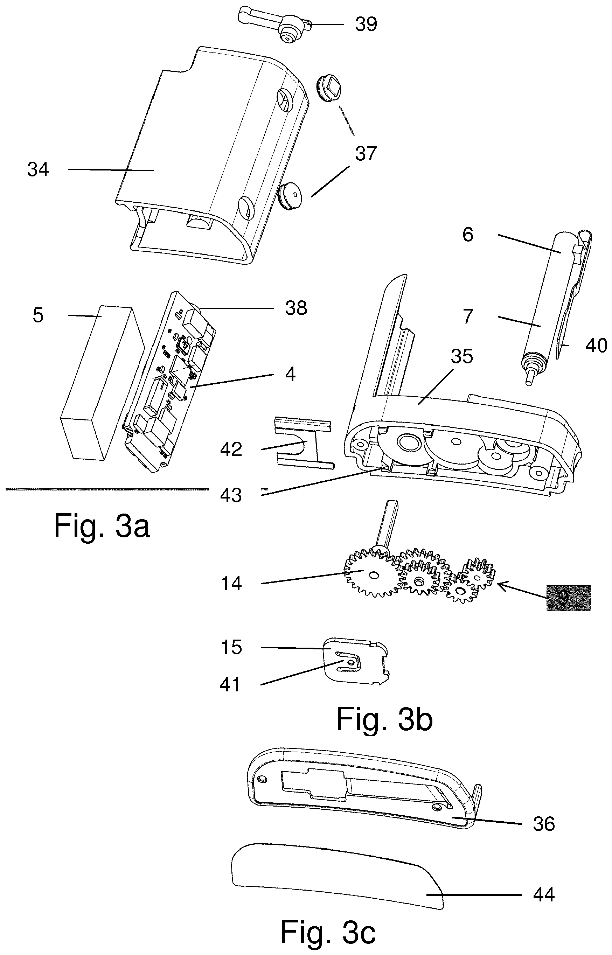

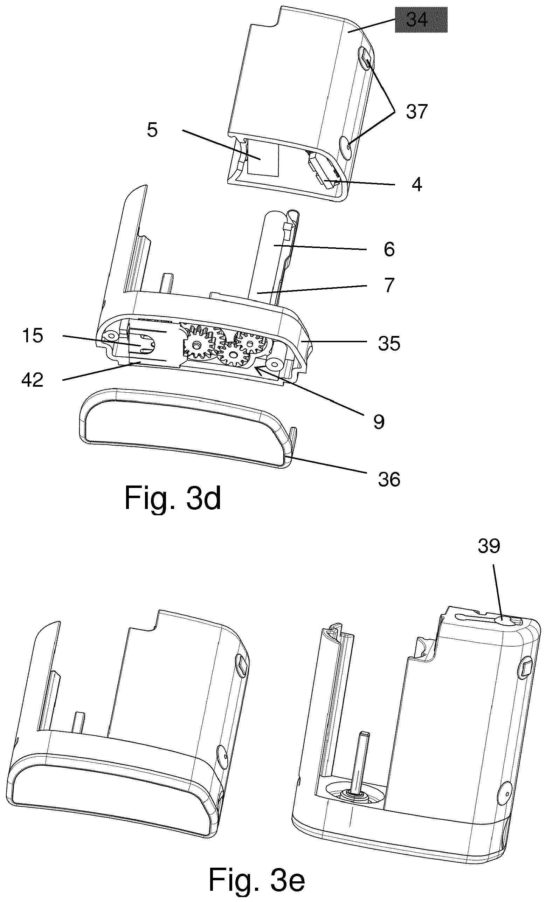

In den Fig. 3a bis 3e ist der wiederverwendbare Teil 2 der Dosiervorrichtung D beschrieben. Vorzugsweise besteht der wiederverwendbare Teil 2 aus drei Gehäusebauteilen. An einem ersten Gehäusebauteil 34 kann die Batterie 5 angeordnet sein, dies kann beispielsweise eine aufladbare Akkubatterie sein, die über ein herkömmliches Ladegerät für ein Mobiltelefon beispielsweise aufgeladen werden kann. Weiter ist am ersten Gehäusebauteil 34 die Elektronikleiterplatte 4 angeordnet. Auf dieser sind beispielsweise zwei Druckknöpfe 37 für die manuelle Bedienung direkt angeordnet. Ebenso ist ein Ladestecker 38 für die Batterie direkt auf der Elektronikleiterplatte 4 angeordnet. Damit der erste Gehäuseteil 34 dicht ist, ist ein am Gehäuse 3 ausgebildeter Zugang zum Ladestecker über eine elastische Lasche 39 schliessbar und dichtbar. Die Elektronikleiterplatte 4 weist mindestens einen Mikroprozessor für die Steuerung der Dosiervorrichtung D auf. Der Motor 6 ist über einen Flexprint 40, also über eine flexible, elektrische Verbindung, mit der Elektronikleiterplatte 4 verbunden. Die Elektronikleiterplatte 4 kann zudem Mittel für die Alarmierung sowie beispielsweise Bluetooth Kommunikationsmittel aufweisen. Generell sind der Datenaustausch der Dosiervorrichtung D, sowie die Programmierung und die Steuerung über externe Kommunikationsgeräte möglich. Beispielsweise kann die Dosiervorrichtung D über ein Smartphone oder über ein Blutzuckermessgerät gesteuert werden. Der Anwender kann die Steuerung der Dosiervorrichtung D aber auch manuell vornehmen, dazu stehen ihm die beiden an der Pumpe angeordneten Bedienknöpfe 37 zur Verfügung. Über beispielsweise eine Bluetooth Kommunikationsschnittstelle kann die Dosiervorrichtung D Daten mit beispielsweise einem Computer, einem Smartphone oder einem Blutzuckermessgerät austauschen. Auch die Kommunikation zu einem am Körper getragenen Blutzuckersensor, insbesondere einem implantierten Sensor, ist möglich. In der Fig. 3b ist der zweite Gehäusebauteil 35 dargestellt. Dieser Bauteil ist als Chassis 35 ausgebildet und nimmt die wesentlichen Bauteile der Antriebsvorrichtung M auf. Am Chassis ist der Motor 6 mit dem nachgelagerten Planetengetriebe angeordnet. Über das weitere Stirnradgetriebe 9 wird das letzte Abgangsrad 14 des Getriebes angetrieben. Das letzte Abgangsrad ist mit der Mitnehmerstange als Kupplungsglied verbunden. Das Stirnradgetriebe wird von einer unteren Seite des Chassis montiert. Das letzte Abgangsrad 14 stützt sich dabei am Kraftsensor 15 ab. Der Kraftsensor 15 selbst weist einen Biegebalken 41 auf, wobei die Krafteinlenkung des Abgangsrades 14 auf den Biegebalken 41 einwirkt und ein elektrisches Signal, das proportional zur Auslenkung ist, generiert und an die Steuereinheit über eine nicht dargestellte Verbindungsleitung geleitet wird. Der Kraftsensor 15 sowie das Abgangsrad 14 sind am Chassis 35 über eine Platte 42 gesichert. Die Platte 42 kann an zwei Linearführungen 43 seitlich eingeschoben werden und den Kraftsensor 15 und das Abgangsrad 14 in der montierten Position halten. In der Fig. 3c ist der dritte Gehäusebauteil 36 dargestellt, dieser ist lediglich ein Getriebeboden. Der Getriebeboden 36 kann geklebt aber auch geschraubt mit dem Chassis 35 verbunden werden. Vorteilhaft ist hier, dass keine an einer Schnittstelle zwischen Getriebeboden 36 und Chassis 35 wirkende Kraft auftreten kann, so dass diese Verbindung gut abdichtbar ist und eine lange Lebensdauer aufweisen kann. Weiter kann der Getriebeboden 36 mit einem klebbaren Deckplatt 44 geschlossen werden. Gemäss Fig. 3d werden zuerst der erste Gehäuseteil 34 und das Chassis 35 vormontiert. Das bedeutet, dass am ersten Gehäuseteil 34 erst die Batterie 5 und die Elektronikleiterplatte 4 montiert werden sowie die beiden Bedienknöpfe 37 und die Lasche 39 für den Batteriestecker. Das Chassis 35, sowie die am Chassis 35 angeordneten Bauteile werden ebenso vormoniert. Dabei wird der Motor 6, das Planetengetriebe 7, das Stirnradgetriebe 9 samt Abgangszahnrad 14 montiert. Nachfolgend wird das Abgangsrad 14 über den Kraftsensor 15 und die Platte 42 axial fixiert. Schliesslich wird der erste Gehäusebauteil 34 am Chassis 35 montiert, dies kann über eine Schraubenverbindung oder über eine Klebeverbindung erfolgen. Weiter wird der Getriebeboden 36 am Chassis 35 befestigt. In der Fig. 3e ist der fertigmontierte, wiederverwendbare Teil 2 dargestellt. Vorteilhaft sind die einfache Montage und die kräftefreien Aufnahmen von Getriebeboden 36 und erstem Gehäuseteil 34 am Chassis 35.

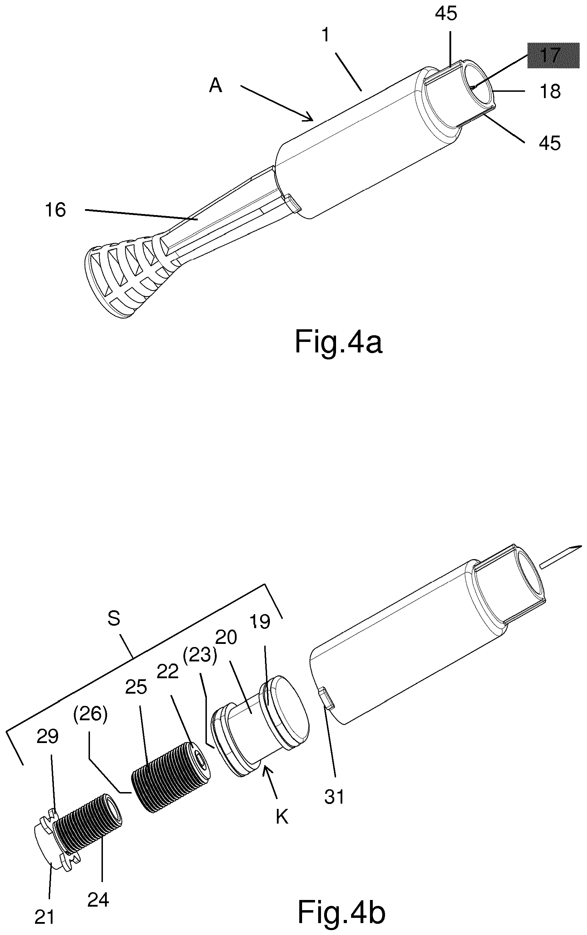

In den Fig. 4a und 4b ist der erste Verbrauchsteil 1 dargestellt. Beim ersten Verbrauchsteil 1 handelt es sich um das Reservoir A. Das Reservoir A weist die Reservoirwandung zur Aufnahme des Medikamentenfluids auf. Das Ausführungsbeispiel hier weist die Konnektierungsnadel 17 auf, über welche das Reservoir A mit einer nachgelagerten fluidführenden Verbindung F verbunden werden kann. Damit sich der Anwender nicht an der Konnektierungsnadel 17 verletzen kann, ist die Nadel 17 vom Schutzkragen 18 umgeben. Der Schutzkragen 18 weist zwei Führungsstifte 45 auf, über welche das Reservoir A rotationsgesichert gelagert werden kann. Weiter weist das Reservoir A den Kolben K auf. Der Kolben K selbst ist Teil der Spindelvorrichtung S und weist daher das Innengewinde 23 auf, mit welchem das Aussengewinde 25 der Antriebsstufe 22 im Eingriff steht. Die Antriebsstufe 22 besteht aus zwei Hülsen, die an der dem Kolben K zugewandten Seite miteinander verbunden sind. Das Aussengewinde 24 der zweiten Verschiebestufe steht im Eingriff mit dem Innengewinde 26 der Antriebsstufe 22. Bei der Montage wird beispielsweise die Antriebsstufe 22 in den Kolben K eingeschraubt, anschliessend wird die zweite Verschiebestufe 21 in die Antriebsstufe 22 geschraubt. Die beiden Spindeltriebe sind gegenläufig, das bedeutet, dass der eine Spindeltrieb ein Rechtsgewinde aufweist, der andere Spindeltrieb hingegen ein Linksgewinde. Der Kolben K weist zwei Dichtungsstellen auf, an welche O-Ringe 19 angeordnet sein können. Der Kolben K samt der am Kolben K angeordneten Spindelvorrichtung S wird nach der Montage in das Reservoir A verschoben. Die zweite Verschiebestufe 21 wird gegen Verdrehung über die radial nach aussen stehenden Flügel 29 gesichert, welche sich an der Innenwandung 30 abstützen können. Sowohl die erste Verschiebestufe 20 als auch die zweite Verschiebestufe 21 sind verdrehgesichert, wobei die Verdrehsicherung über die Reservoirwandung 28 erfolgt. Die zweite Verschiebestufe 21 ist zudem mit der Aufziehstange 16 verbindbar. Vorteilhaft ist hier, dass die teleskopische Spindelvorrichtung S aus lediglich zwei zusätzlichen Bauteilen gebildet werden kann, die durch einfache Montage miteinander verbunden und montiert werden können. Diese zusätzlichen Bauteile sind die Antriebsstufe 22 und die zweite Verschiebestufe 21. Überdies ist der Kolben K über die zweite Verschiebestufe 21 mit der Aufziehstange 16 verbindbar. Das Befüllen erfolgt durch Verschieben des Kolbens K über die Aufziehstange 16, was für den Anwender eine gewohnte Handhabung darstellt. Weiter weist der Kolben K eine gute Stabilität im Reservoir A auf. Ein Mass für die Qualität der Lagerung des Kolbens K in einem Reservoir A zur Vermeidung von Walken ist das Längenverhältnis L/Do, wobei L eine Längsachse des ovalen Kolbenquerschnittes ist und Do der Abstand zwischen Dichtstellen am Kolben K. Weiter weist das Reservoir A die zwei Nocken 31 auf, über welche das Reservoir A am wiederverwendbaren Teil 2 gelagert werden kann. Die Nocken 31 bilden einen Teil einer Bajonettverbindung 31,33. Die teleskopische Spindelvorrichtung S besteht aus den zwei Verschiebestufen 20,21 und der von der Mitnehmerstange 11 angetriebenen Antriebsstufe 22. Bei der teleskopischen Spindelvorrichtung S verfährt die erste Verschiebestufe 20 in Vortriebsrichtung beim Hub H2, die zweite Verschiebestufe 21 verfährt beim Hub H1 lediglich rückwärtig. Die drehend angetriebene Antriebsstufe 20 kann hier sowohl in Vortriebsrichtung wie auch rückwärtig, d.h. entgegen der Vortriebsrichtung, axial verfahren.

Die im Stand der Technik beschriebenen Geräte der zweiten und dritten Generation weisen lediglich eine Verschiebestufe und eine Antriebsstufe auf und gehören der Kategorie der einstufigen Spindelvorrichtungen mit einem Spindeltrieb an. Bei diesen kann beispielsweise eine Spindelstange von einer Antriebsvorrichtung angetrieben sein und als Antriebsstufe dienen. Gleichzeitig dient die Spindelstange aber auch als Verschiebestufe, denn sie stösst sich bei ihrem Antrieb an einer festen Spindelmutter ab. Bei dieser Ausführungsform ist die Spindelstange sowohl Antriebsstufe wie auch Verschiebestufe. Bei einer anderen Ausführungsvariante des Standes der Technik ist die Spindelstange fest mit dem Kolben verbunden und dient daher lediglich als Verschiebestufe. Die Spindelmutter ist axial ortsfest, kann aber drehend von einer Antriebsvorrichtung angetrieben sein. Die Spindelmutter dient bei dieser Variante als Antriebsstufe der Spindelvorrichtung, leistet aber keine Verschiebearbeit.

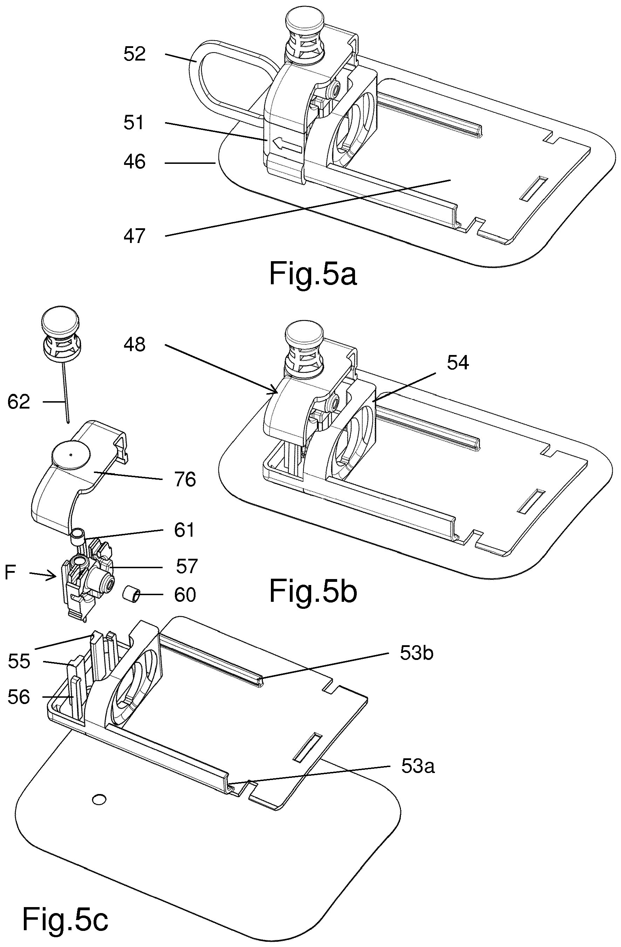

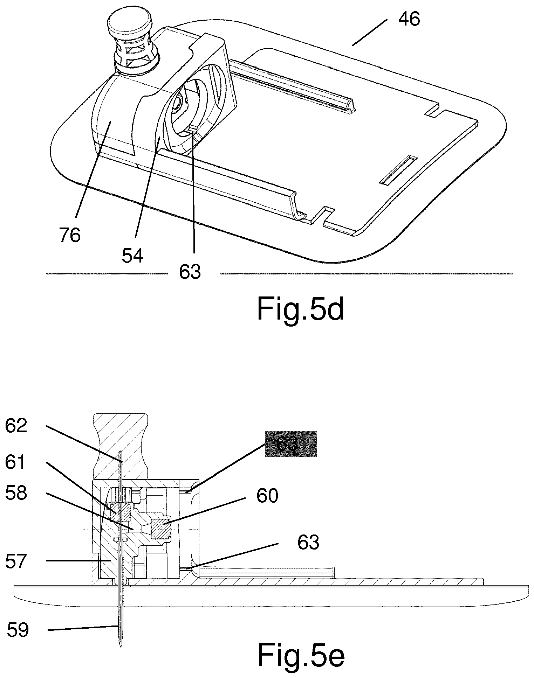

Anhand der Fig. 5a bis 5e wird der zweite Verbrauchsteil 46 beschrieben. Der zweite Verbrauchsteil 46 weist eine Grundplatte 47 und einen Insertionskopf 48 auf. Die Grundplatte 47 weist eine auf das Gewebe flächig aufsetzbare und für eine Fixierung auf dem Gewebe vorbereitete Unterseite 49 auf. Die Unterseite 49 kann selbstklebend ausgeführt sein. Der Anwender entfernt erst eine Schutzfolie 50 und klebt anschliessend den zweiten Verbrauchsteil 46 über die selbstklebende Unterseite 49 an einer desinfizierten Körperstelle auf. Bevor der Anwender den aufgeklebten zweiten Verbrauchsteil 46 bedienen kann, muss er ein Sperrelement 51 entfernen. Das Sperrelement 51 hat hierzu einen Griff 52, über welchen der Anwender das Sperrelement 51 gut greifen kann. Durch Ziehen entfernt der Anwender das Sperrelement 51 gemäss den Fig. 5a und 5b. In der Fig. 5c ist der zweite Verbrauchsteil 46 in Explosionsdarstellung gezeigt. Nebst der selbstklebenden Unterseite 49, weist der zweite Verbrauchsteil 46 die Grundplatte 47 auf. An der Grundplatte 47 sind zwei Längsführungen 53 für die Aufnahme des wiederverwendbaren Teils 2 vorgesehen. Weiter weist die Grundplatte 47 eine Anschlagsbasis 54 für den wiederverwendbaren Teil 2 auf. In Vortriebsrichtung stützt sich der wiederverwendbare Teil 2 an der Anschlagsbasis 54 ab. Weiter umfasst die Grundplatte Führungsmittel in Form von profilierten Schienen 55 für die winklige Verschiebung des Insertionskopfes 48. Die profilierten Schienen 55 haben hier einen L-förmigen Querschnitt. Damit der Insertionskopf 48 in zwei Positionen gesichert gehalten werden kann, weist die Grundplatte Haltemittel auf. Hier sind die Haltemittel als zwei an der Grundplatte angeordnete, flexible Schnapphaken 56 ausgebildet, die am Insertionskopf an zwei Positionen einrastbar sind. Weiter weist der Insertionskopf 48 ein Kanülengehäuse 57 auf. Das Kanülengehäuse 57 ist der Kernteil des Insertionskopfes 48, am welchem die fluidführende Verbindung F zwischen dem Reservoir A und dem Anwender ausgebildet ist. Die fluidführende Verbindung F besteht aus einem im Kanülengehäuse 57 gebildeten Kanal 58, der zu einer Softkanüle 59 führt. Die Softkanüle 59 kann am Kanülengehäuse 57 eingespritzt oder eingeklebt sein. Der fluidführende Kanal 58 ist über zwei Septen 60,61 begrenzt. Das eine Septum 60 ist über die am Reservoir A angeordneten Konnektierungsnadel 17 penetrierbar. Das andere Septum 61 sowie die Kanüle 59 sind in der Ausgangsposition von einer Einstichnadel 62 in Verfahrrichtung des Insertionskopfes 48 durchdrungen. Ausgehend von der Ausgangsposition in Fig. 5b betätigt der Anwender den Insertionsknopf 48. Beim Aufbringen einer minimalen Auslösekraft lösen sich die beiden Schnapphaken 56 aus ihren Verrastungen und geben den Insertionskopf 48 frei. Dieser verfährt dabei mit hoher Geschwindigkeit entlang seiner Führung 55 gegen seine Endposition. In der Endposition ist der Insertionskopf 48 wieder über die Schnapphaken 56 gesichert. Zudem befindet sich die Kanüle 59 im Gewebe des Anwenders, wie dies in den Fig. 5d und Fig. 5e gezeigt ist. In der Fig. 5e ist der durch die Septen 60,61 begrenzte fluidführende Durchgangskanal 58 im Längsschnitt gezeigt. In Verfahrrichtung des Insertionskopfes 48 ist sowohl das Septum 61 als auch die am Kanülengehäuse 57 angeordnete Kanüle 59 von der Einstichnadel 62 durchdrungen. An der Anschlagsbasis 54 sind zudem Führungen 63 ersichtlich, die für die Führungsstifte 45 des Reservoirs A vorgesehen sind. Die Führungen 63 und die Führungsstifte 45 bilden zusammen ein rotationsgesichertes Gleitlager, über welches das Reservoir A an der Anschlagsbasis 54 gleitgelagert wird. Zudem ermöglicht eine derartige Lagerung, dass das Septum 60 geradlinig von der Konnektierungsnadel 17 penetriert werden kann beim Verbinden des wiederverwendbaren Teils 2 mit dem zweiten Verbrauchsteil 46. Durch ein Entfernen der Einstichnadel 62 wird die Kanüle 59 für die subkutane Infusion mit Medikamentenfluid freigegeben. Generell kann der Insertionskopf 48 eine Haube 76 aufweisen, welche als Teil des Gehäuses 3 dienen kann. Die Haube 76 kann dabei am Kanülengehäuse 57 befestigt sein.

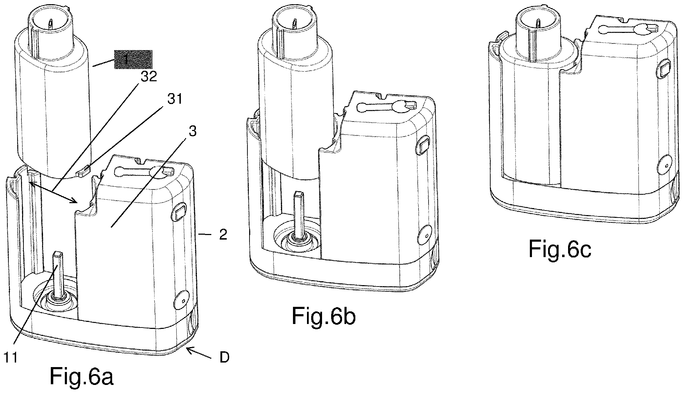

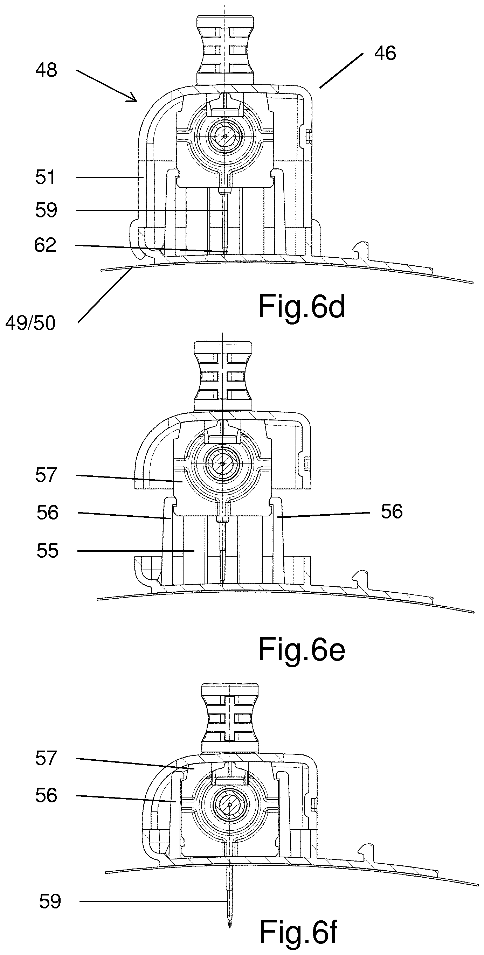

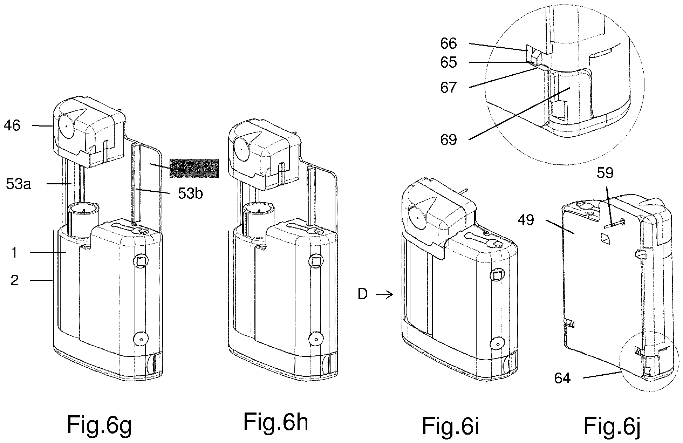

In den Fig. 6a bis 6j wird die Handhabung der Dosiervorrichtung D für den Anwender besprochen. In einem ersten Handhabungsschritt wird der erste Verbrauchsteil 1 über die am Gehäuse 3 ausgebildete Längsführung 32 aufgenommen und über diese geradgeführt verschoben. Beim Verschieben wird die Mitnehmerstange 11 in das axiale Langloch 27 der Spindelvorrichtung S eingeführt. Durch weiteres Verschieben wird der erste Verbrauchsteil 1 über die am Gehäuse 3 ausgebildete Längsführung 32 seiner Lagerstelle am Gehäuse 3 zugeführt. Dabei werden die am ersten Verbrauchsteil 1 angeordneten Nocken 31 in die am Gehäuse ausgebildeten Lagerschlitze 33 eingedreht. Der Anwender kuppelt so den ersten Verbrauchsteil 1 mit dem wiederverwendbaren Teil 2. Dabei ist nun der erste Verbrauchsteil 1 sowohl in Vortriebsrichtung als auch gegen die Vertriebsrichtung gesichert. Weiter ist die Antriebsvorrichtung M über die Mitnehmerstange 11 mit der Spindelvorrichtung S gekuppelt. Der Anwender kann nun ein Priming ausführen. Da generell die Spindelvorrichtung S nach der Befüllung, unabhängig davon ob das Reservoir A teil- oder ganzbefüllt ist, in ihrer eingefahrenen Position ist, verfährt die Spindelvorrichtung S erst rückwärtig gegen ihren Anschlag 12. Das Auffahren an den Anschlag 12 wird von der Steuerung detektiert und der Motor 6 durch die Steuerung in Stillstand gesetzt. In den Fig. 6d bis 6f ist die Handhabung des zweiten Verbrauchsteils 46 dargestellt. Der Anwender entfernt erst die Schutzfolie 50 der Unterseite 49 und klebt die selbstklebende Unterseite 49 des zweiten Verbrauchsteils 46 an einer geeigneten Körperstelle auf. Damit der Anwender sich nicht verletzen kann, ist die Kanüle 59 im Insertionskopf 48 gesichert und nicht zugänglich angeordnet. Speziell für Anwender, die an einer Nadelphobie leiden, ist die Ausführung des Insertionskopfes 48 sehr vorteilhaft. Denn der Anwender kann in der Grundstellung weder die Einstichnadel 62 noch die Kanüle 59 optisch wahrnehmen, da sie im Innern des Insertionskopfes 48 angeordnet ist. Nachfolgend wird das Sperrelement 51 entfernt, womit der Insertionskopf 48 für ein Einbringen der Kanüle 59 bereit ist. In Fig. 6e ist gut ersichtlich wie die beiden seitlichen Schnapphaken 56, das Kanülengehäuse 57 in der Ausgangsposition halten und sichern. Durch Aufbringen einer minimalen Auslösekraft werden die Schnapphaken 56 ausgelenkt bis diese nicht mehr im Eingriff mit dem Kanülengehäuse 57 stehen. Das Kanülengehäuse 57 verfährt hierauf ungesichert infolge der beaufschlagten Kraft entlang der Führungsmittel 55 mit hoher Geschwindigkeit gegen eine Endposition. In der Endposition sind die Schnapphaken 56 wieder im Eingriff mit dem Kanülengehäuse 57 und sichern das Kanülengehäuse 57 in der neuen Position. Weiter ist nun die Kanüle 59 im Gewebe platziert. In den Fig. 6g bis 6j wird der mit dem ersten Verbrauchsteil 1 verbundene wiederverwendbare Teil 2 mit dem zweiten Verbrauchsteil 46 verbunden. Der mit dem ersten Verbrauchsteil 1 verbundene wiederverwendbare Teil 2 kann mit mindestens einer an der Grundplatte 47 ausgebildeten Längsführung 53 als Linearführung in Eingriff gebracht werden und nachfolgend entlang der mindestens einen Längsführung 53a axial verschoben werden. Beim axialen Verschieben entlang der Längsführung 53a wird der mit dem ersten Verbrauchsteil 1 verbundene wiederverwendbare Teil 2 mit einer zweiten Längsführung 53b in Eingriff gebracht. Durch axiales Verschieben an die an der Grundplatte 47 ausgebildete Anschlagsbasis 54 wird der erste Verbrauchsteil 1 mit dem zweiten Verbrauchsteil 46 geführt und rotationsgesichert gelagert verbunden. Dabei greifen die Führungsstifte 45 des ersten Verbrauchsteils 1 in die Führungen 63 des zweiten Verbrauchsteils 46. Durch weiteres Verschieben wird schliesslich das Septum 60 von der geführten Konnektierungsnadel 17 geradgeführt penetriert. In den Fig. 6i und 6j ist die Dosiervorrichtung D komplett dargestellt. In der Fig. 6j ist die komplette Dosiervorrichtung D von der Unterseite 49 dargestellt. Die Kanüle 59 ragt dabei von der Grundplatte 47 hervor und kann derart ausgehend von der Grundplatte 47 mehrere Millimeter in das Gewebe des Anwenders ragen. Bei der Insulinpumpentherapie beträgt die Eindringtiefe der Kanüle 59 zwischen 5 und 12 mm. Beim Ausführungsbeispiel beträgt der Einstichwinkel 90 Winkelgrad. Der Einstichwinkel kann auch zwischen 10 und 60 Winkelgrad betragen und das Kanülengehäuse 57 dabei an einer schiefen Ebene verfahren. Weiter ist in der Fig. 6j eine Schnapphakenverbindung 64 zwischen dem wiederverwendbaren Teil 2 und der Grundplatte 47 im Detail dargestellt. Diese Verbindung wird aus einem Haken 65, welcher am Gehäuse 3 ausgebildet ist und einer Aussparung 66 an der Grundplatte 47 gebildet. Im gekuppelten Zustand gemäss der Fig. 6j greift der Haken 65 in die Aussparung 66 und fixiert den wiederverwendbaren Teil 2 an der Grundplatte 47. Hierzu stützt sich der Haken 65 an einer Kante 67 der Aussparung 66 ab. Die Grundplatte 47 ist im Bereich der Aussparung 66 elastisch ausgeführt. Weiter wird angemerkt, dass das Auslösen des Insertionskopfes 48 auch über ein zusätzliches Gerät erfolgen kann. Bei einem derartigen Gerät kann beispielsweise ein Stempel von einer Feder vorgespannt sein. Durch Auslösen des Stempels verfährt der Stempel und trifft auf den Insertionskopf 48. Dabei wird der Insertionskopf 48 von seiner Ausgangsposition über den Stempel in seine Endposition gebracht und die Kanüle 59 so im Gewebe platziert.

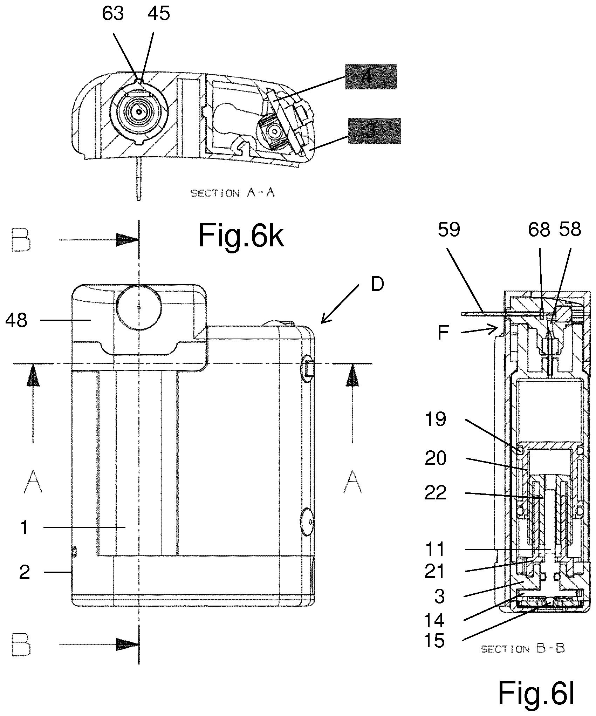

In der Fig. 6k ist ein Schnitt der Dosiervorrichtung D quer zur Vortriebsachse dargestellt. Der Schnitt erfolgt dabei auf der Höhe des Insertionskopfes 48 an der Anschlagsbasis 54. Gut ist in dieser Darstellung sichtbar, wie das Reservoir A an der Anschlagsbasis 54 gelagert ist. Das Reservoir A weist hierzu die beiden Führungsstifte 45 auf, die in die Führungen 63 an der Anschlagsbasis 54 eingreifen. Eine derartige Lagerung ist besonders vorteilhaft, denn dadurch wird erreicht, dass Querkräfte vom Lager aufgenommen werden können, zudem wird eine Rotation des Reservoirs A entlang der Vortriebsachse vermieden. Hingegen nimmt die Lagerstelle keine in Vortriebsrichtung wirkenden Kräfte auf. Eine Verschiebung zwischen dem Gehäuse 3 und dem zweiten Verbrauchsteil 46, das heisst der Anschlagsbasis 54, führt zu keiner Verschiebung des Reservoirs A. Dadurch wird vermieden, dass derartige Verschiebungen ungewollte Medikamentenabgaben verursachen. Beim Stand der Technik verursacht eine analoge Verschiebung des Adapters relativ zum Gehäuse eine ungewollte Medikamentenabgabe, da sich bei diesen Geräten das Reservoir A am Adapter abstützt. Dadurch, dass sich das Reservoir A nicht an der Anschlagsbasis 54 abstützt, sondern an der Anschlagsbasis 54 lediglich geleitgelagert ist, kann die besagte ungewollte Medikamentenabgabe des Standes der Technik, vermieden werden. In der Fig. 6k ist zudem die Lagerung der Elektronikleiterplatte 4 im Gehäuse 3 an einer Führung sichtbar. Der Schnitt der Fig. 6l erfolgt entlang der Vortriebsachse und zeigt den Aufbau der Dosiervorrichtung D im Längsschnitt. Das Abgangsrad 14 der Antriebsvorrichtung M stützt sich über den Kraftsensor 15 ab. Das Abgangsrad 14 ist über den Schaft mit der Mitnehmerstange 11 verbunden, welche die Antriebsstufe 22 der Spindelvorrichtung S rotatorisch antreibt. In der Fig. 6l ist ein teilbefülltes Reservoir A gezeigt. Das Priming ist hier bereits erfolgt, so dass sich die zweite Verschiebestufe 21 an ihrem an der Mitnehmerstange 11 ausgebildeten Anschlag 12 abstützt. Der Kolben K selbst ist als die erste Verschiebestufe 20 der Spindelvorrichtung S ausgebildet und ist über zwei O-Ringe 19 im Reservoir A gehalten. Die Konnektierungsnadel 17 des ersten Verbrauchsteils 1, also des Reservoirs A, ragt durch das Septum 60 des Kanülengehäuses in den fluidführenden Kanal 58 hinein. Der Kanal 58 weist eine Umlenkung von 90 Winkelgrad auf und mündet an der Kanüle 59. Die Kanüle 59 selbst weist einen Kragen 68 auf, der sich besonders gut für ein Umspritzen bei der Herstellung eignet. Die Einstichnadel 62 ist hier vom Anwender bereits entfernt worden, so dass die fluidführende Verbindung F ausgehend vom Reservoir A bis an den Kanülenaustritt mit Medikamentenfluid durch das Priming befüllt ist. Die Dosiervorrichtung D ist für eine Medikamentenabgabe an den Anwender bereit.

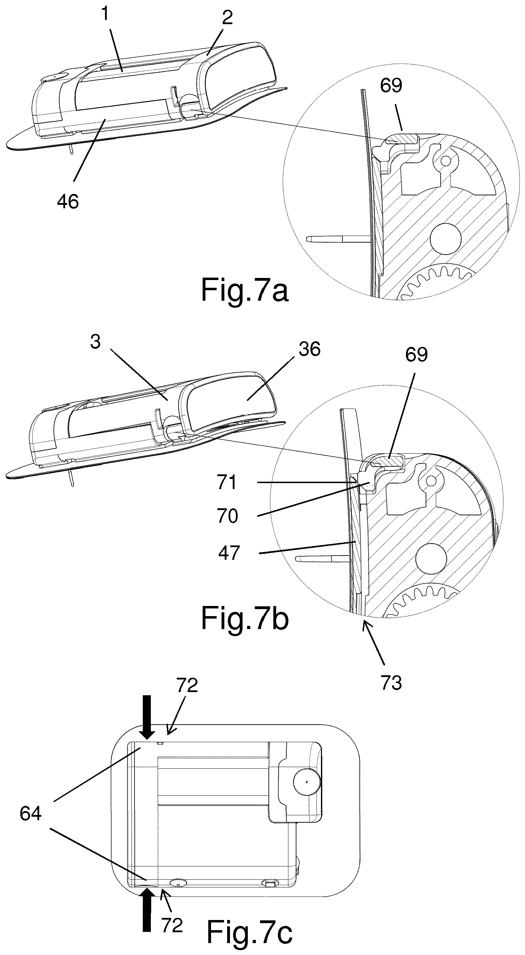

Die Schnapphakenverbindung 64 der Fig. 6j ist in den Fig. 7a bis 7c nochmals im Detail dargestellt. Vorzugweise wird der wiederverwendbare Teil 2 am zweiten Verbrauchsteil 46 über mindestens zwei am Gehäuse 3 und der Grundplatte 47 ausgebildete, lösbare Schnapphakenverbindungen 64 axial fixiert. Beim Lösen der Schnapphakenverbindung 64 wird ein flexibler Bedienknopf 69 bedient und ausgelenkt, wie dies in Fig. 7b dargestellt ist. Der Bedienknopf 69 weist einen Spreizkopf 70 auf. Wird der Bedienknopf 69 vom Anwender ausgelenkt, so spreizt der Spreizkopf 70 die Grundplatte 47 vom Gehäuse 3 und löst dabei die Schnapphakenverbindung 64 zwischen der Grundplatte 47 und dem Gehäuse 3. Sowohl der Spreizkopf 70 als auch die Grundplatte 47 können keilförmig ausgebildet sein, wodurch die Grundplatte 47 im Ort des Eingriffs gut spreizbar ist. Der Spreizkopf 70 wie die Grundplatte 47 können hierzu schiefe Ebenen 71 aufweisen, wodurch ein gutes Eingreifen an der Grundplatte 47 durch den Spreizkopf 70 möglich ist. Der Bedienknopf 69 ist vorzugsweise Teil des Gehäuses 3, insbesondere des dritten Gehäusebauteiles 36. Gemäss Fig. 7c ist es vorgesehen, dass die Dosiervorrichtung D zwei Schnapphakenverbindung 64 aufweist und diese über seitliche, an gegenüberliegenden Seitenflächen 72 des Gehäuses 3 angeordnete Bedienknöpfe 69 zu betätigen sind und zum Lösen beide Bedienknöpfe 69 gleichzeitig mit einer Hand, insbesondere mit einem Daumen und einem Zeigfinger, betätigt werden. Durch diese Anordnung gemäss der Fig. 7c kann sichergestellt werden, dass ein fälschliches Lösen einer Schnapphakenverbindung 64 nicht zu einem Lösen des Gehäuses 3 von der Grundplatte 47 führt. Es müssen stets beide Schnapphakenverbindungen 64 gleichzeitig vom Anwender bedient und gelöst werden, um das Gehäuse 3 von der Grundplatte 47 zu trennen. Weiter kann eine dritte Schnapphakenverbindung 73 vorgesehen sein. Diese kann wiederum zwischen dem Gehäuse 3 und der Grundplatte 47 angeordnet sein. Diese dritte Schnapphakenverbindung 73 ist lediglich durch Auslenken der Grundplatte 47 lösbar und bedarf daher einer minimalen Zugkraft am Gehäuse 3. Beim Lösen geht der Anwender daher folgendermassen vor. Über Zeigefinger und Daumen beispielsweise löst er die beiden seitlichen Schnapphakenverbindungen 64. Durch axiales Ziehen wird schliesslich die dritte Schnapphakenverbindung 73 gelöst, so dass das Gehäuse 3 von der Grundplatte 47 entlang der Längsführung 53 getrennt werden kann.

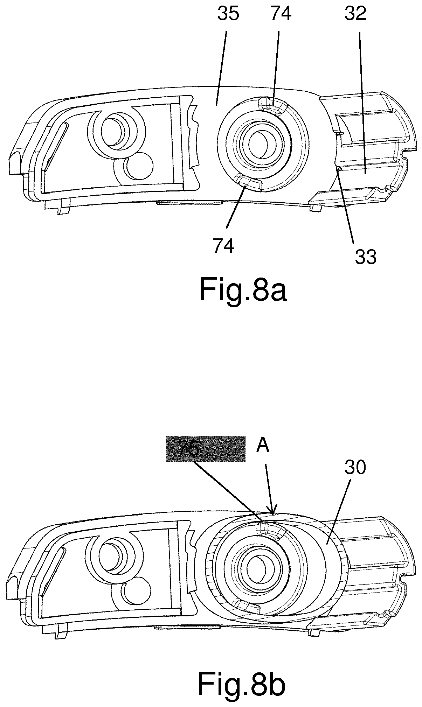

In den Fig. 8a und 8b ist eine besonders vorteilhafte Abstützung und Lagerung des Reservoirs A am Chassis 35 dargestellt. Am Chassis 35 kann hierzu mindestens ein Stützelement 74 angeordnet sein. Beim Verschieben des Reservoirs A zu seiner Lagerstelle entlang der Längsführung 32 greifen die Stützelemente 74 in das Reservoir A ein. Dabei berühren die Stützelemente 74 an ihrem äusseren Umfang die Innenwandung 30 des Reservoirs A und verhindern so eine radiale Auslenkung des Reservoirs A. Nachfolgend wird das Reservoir A in die Lagerschlitze 33 eingedreht. Dabei greifen die Nocken 31 in die Lagerschlitze 33 und bilden derart die Bajonettverbindung 31,33, wie dies bereits besprochen worden ist. Die Innenwandung 30 des Reservoirs A ist jetzt an den Stützelementen 74 nicht mehr über den Umfang sondern nur noch entlang einer Kontaktlinie 75 abgestützt. Eine äussere, auf das Reservoir A wirkende Kraft wird so teilweise von einem Lagerschlitz 33 am Chassis 35 der Bajonettverbindung aufgenommen, gleichzeitig wird ein Teil der äusseren Kraft über die Kontaktlinien 75 an die Stützelemente 74 geleitet.

Bezugszeichenliste

S    Spindelvorrichtung
M    Antriebsvorrichtung
A    Reservoir

| | |
|---|---|
| K | Kolben |
| F | fluidführende Verbindung |
| D | Dosiervorrichtung |
| H | Gesamthub Spindelvorrichtung |
| H1 | rückwärtiger Hub |
| H2 | vorwärtsgerichteter Hub |
| V | Verfahrweg eines Spindeltriebes |
| L | Längsachse des Kolbenquerschnitts |
| Do | Länge zwischen Dichtstellen am Kolben |

| | |
|---|---|
| 1 | Erster Verbrauchsteil |
| 2 | Wiederverwendbarer Teil |
| 3 | Gehäuse |
| 4 | Elektronikleiterplatte |
| 5 | Batterie |
| 6 | Motor |
| 7 | Planetengetriebe |
| 8 | Zahnräder |
| 9 | Stirnradgetriebe |
| 10 | Schaft |
| 11 | Mitnehmerstange |
| 12 | Anschlag |
| 13 | O-Ring |
| 14 | Abgangsrad |
| 15 | Kraftsensor |
| 16 | Aufziehstange |
| 17 | Konnektierungsnadel |
| 18 | Schutzkragen |
| 19 | O-Ring |
| 20 | erste Verschiebestufe |
| 21 | zweite Verschiebestufe |
| 22 | Antriebsstufe |
| 23 | Innengewinde erste Verschiebestufe |
| 24 | Aussengewinde zweite Verschiebestufe |
| 25 | Aussengewinde Antriebsstufe |
| 26 | Innengewinde Antriebsstufe |
| 27 | Langloch |
| 28 | Reservoirwandung |
| 29 | Flügel |
| 30 | Innenwandung |
| 31 | Nocken |
| 32 | Längsführung |
| 33 | Lagerschlitz |
| 34 | Erster Gehäusebauteil |
| 35 | Zweiter Gehäusebauteil, Chassis |
| 36 | Dritter Gehäusebauteil, Getriebeboden |
| 37 | Druckknopf, Bedienknopf |
| 38 | Ladestecker |
| 39 | Lasche |
| 40 | Flexprint |
| 41 | Biegebalken |
| 42 | Platte |
| 43 | Linearführung |
| 44 | Deckblatt |
| 45 | Führungsstift |
| 46 | Zweiter Verbrauchsteil |
| 47 | Grundplatte |
| 48 | Insertionskopf |

| 49 | Unterseite |
| --- | --- |
| 50 | Schutzfolie |
| 51 | Sperrelement |
| 52 | Griff |
| 53a | Längsführung |
| 53b | Längsführung |
| 54 | Anschlagsbasis |
| 55 | Schiene |
| 56 | Schnapphaken |
| 57 | Kanülengehäuse |
| 58 | Kanal |
| 59 | Softkanüle |
| 60 | Septum |
| 61 | Septum |
| 62 | Einstichnadel |
| 63 | Führung |
| 64 | Schnapphakenverbindung |
| 65 | Haken |
| 66 | Aussparung |
| 67 | Kante |
| 68 | Kragen |
| 69 | Bedienknopf |
| 70 | Spreizkopf |
| 71 | Schiefe Ebene |
| 72 | Seitenflächen am Gehäuse |
| 73 | Dritte Schnapphakenverbindung |
| 74 | Stützelement |
| 75 | Kontaktlinie |
| 76 | Haube |

**Patentansprüche**

1. Dosiervorrichtung zur Verabreichung von Medikamentenfluid in den Körper eines Anwenders, wobei das Medikamentenfluid zum Zwecke einer Infusion oder einer Injektion aus einem Reservoir (A) durch Vortrieb eines im Reservoir (A) gehaltenen Kolbens (K) dosiert ausschüttbar ist, die Dosiervorrichtung (D) weiter umfassend:

   a) einen wiederverwendbaren Teil (2) umfassend ein Gehäuse (3), eine Steuereinheit und eine Antriebsvorrichtung (M), wobei die Antriebsvorrichtung (M) ein Kupplungsglied zur Übertragung einer Antriebsleistung an eine Spindelvorrichtung (S) zum Vortrieb des Kolbens (K) aufweist, und das Kupplungsglied mit einem Abgangsrad (14) der Antriebsvorrichtung (M) fest verbunden ist und einen axialen Anschlag (12) für die Spindelvorrichtung (S) aufweist;
   b) mindestens einen Verbrauchsteil (1) umfassend das Reservoir (A) und den im Reservoir (A) gehaltenen und von der Spindelvorrichtung (S) vortriebbaren Kolben (K), wobei die Spindelvorrichtung (S) am mindestens einen Verbrauchsteil (1) angeordnet ist und mindestens einen Spindeltrieb mit einem Verfahrweg (V) pro Spindeltrieb aufweist, wobei die Spindelvorrichtung (S) des Verbrauchsteils (1) über das Kupplungsglied mit dem wiederverwendbaren Teil (2) kuppelbar ist;
   c) eine fluidführende Verbindung (F) zwischen dem Reservoir (A) und dem Anwender; und
   d) einen am wiederverwendbaren Teil (2) angeordneten Kraftsensor (15), der eine als Mass für den Druck im Reservoir (A) dienende Reaktionskraft der Spindelvorrichtung (S) misst,

   wobei

   i) die Dosiervorrichtung derart ausgestaltet ist, dass sich die Spindelvorrichtung (S) des Verbrauchsteils (1) nach einer Teil- oder Ganzbefüllung des Reservoirs (A) im eingefahrenen Zustand befindet und so ein definierter, stets konstanter Gesamthub (H) der Spindelvorrichtung (S)2 verfahrbar ist,
   ii) das Kupplungsglied axial gleitgelagert ist und sich das Abgangsrad (14) über den Kraftsensor (15) an einer fixen Basis abstützt, wobei aufgrund der festen Verbindung zwischen Kupplungsglied und Abgangsrad (14) beim Anliegen der Spindelvorrichtung (S) am Anschlag (12) die am Anschlag (12) axial wirkende Reaktionskraft der Spindelvorrichtung (S) über das Abgangsrad (14) an den Kraftsensor (15) geleitet wird, so dass eine direkte Kraftübertragung vom Anschlag (12) über das Abgangsrad (14) an den Kraftsensor (15) erfolgt.

2. Dosiervorrichtung nach Anspruch 1, **dadurch gekennzeichnet, dass** zwischen dem Abgangsrad (14) und dem Kupplungsglied eine Dichtungsstelle angeordnet ist.

3. Dosiervorrichtung nach Anspruch 1 oder 2, **dadurch gekennzeichnet, dass** die Dosiervorrichtung derart ausgestaltet ist, dass nach Kupplung eines neu befüllten Verbrauchsteils (1) und des wiederverwendbaren Teils (2) die Spindelvorrichtung (S) gegen den Anschlag (12) zur Aufhebung eines Längsspiels entgegen der Vortriebsrichtung ausfahrbar ist und / oder bei Anliegen der Spindelvorrichtung (S) am Anschlag (12) in Vortriebsrichtung ausfahrbar ist.

4. Dosiervorrichtung nach einem der Ansprüche 1 bis 3, **dadurch gekennzeichnet, dass** das Kupplungsglied als profilierte Mitnehmerstange (11) mit dem Anschlag (12) für die Spindelvorrichtung (S) ausgebildet ist.

5. Dosiervorrichtung nach Anspruch 4, **dadurch gekennzeichnet, dass** bei der Kupplung zwischen dem mindestens einen teil- oder ganzbefüllten Verbrauchsteil (1) und dem wiederverwendbaren Teil (2) die Mitnehmerstange (11) des wiederverwendbaren Teils (2) in ein axiales Langloch (27) der Spindelvorrichtung (S) des Verbrauchsteils (1) einführbar ist und durch Ausziehen der Mitnehmerstange (11) vom axialen Langloch (27) der Verbrauchsteil (1) vom wiederverwendbaren Teil (2) entkuppelbar ist.

6. Dosiervorrichtung nach einem der Ansprüche 4 oder 5, **dadurch gekennzeichnet**, die Länge der Mitnehmerstange (11) ausgehend vom Anschlag (12) gleich gross oder grösser als ein Verfahrweg (V) eines Spindeltriebs ist.

7. Dosiervorrichtung nach einem der Ansprüche 1 bis 6, **dadurch gekennzeichnet, dass** die Antriebsvorrichtung (M) für das rückwärtige Ausfahren und für das in Vortriebsrichtung gerichtete Ausfahren einen gleichen Drehsinn aufweist.

8. Dosiervorrichtung nach einem der Ansprüche 1 bis 7, **dadurch gekennzeichnet, dass** die Spindelvorrichtung (S) am Kolben (K) angeordnet ist und bei der Befüllung der Kolben (K) zusammen mit der Spindelvorrichtung (S) verschiebbar ist.

9. Dosiervorrichtung nach einem der Ansprüche 1 bis 8, **dadurch gekennzeichnet, dass** die Spindelvorrichtung (S) für jedes neue Reservoir (A) stets einen gleichen Gesamthub (H) ausfahrbar ist, unabhängig davon, ob das Reservoir (A) teil- oder ganzbefüllt ist.

10. Dosiervorrichtung nach Anspruch 9, **dadurch gekennzeichnet, dass** sich der Gesamthub (H) aus einem rückwärtigen Hub (H1) zur Aufhebung des Längsspiels und einem in Vortriebsrichtung gerichteten Hub (H2) zur Verabreichung von Medikamentenfluid zusammensetzt.

11. Dosiervorrichtung nach einem der Ansprüche 1 bis 10, **dadurch gekennzeichnet, dass** die Antriebsvorrichtung (M) einen Motor (6) und ein vom Motor (6) angetriebenes Getriebe (7,9) umfasst, wobei ein letztes Getriebezahnrad als das Abgangsrad (14) ausgestaltet ist.

12. Dosiervorrichtung nach einem der Ansprüche 1 bis 11, **dadurch gekennzeichnet, dass** das Gehäuse (3) des wiederverwendbaren Teils (2) aus drei Gehäuseteilen (34,35,36) gebildet ist, wobei ein erster Gehäuseteil (34) mindestens die Steuereinheit und / oder eine Energiequelle (5) aufnimmt, ein zweiter Gehäuseteil (35) als Chassis (35) ausgebildet ist und dabei mindestens den Motor (6), das Getriebe (7,9) und das als Mitnehmerstange (11) ausgebildete Kupplungsglied aufnimmt und ein dritter Gehäuseteil (36) als Getriebeboden (36) ausgebildet ist.

13. Dosiervorrichtung nach Anspruch 12, **dadurch gekennzeichnet, dass** die Basis für den Kraftsensor (15) durch eine am Chassis (35) befestigbare Platte (42) gebildet ist.

14. Dosiervorrichtung nach einem der Ansprüche 12 oder 13, **dadurch gekennzeichnet, dass** das Chassis (35) den ersten (34) und den dritten Gehäuseteil (36) aufnimmt.

15. Dosiervorrichtung nach einem der Ansprüche 1 bis 14, **dadurch gekennzeichnet, dass** der mindestens eine Verbrauchsteil (1) und der wiederverwendbare Teil (2) über einer Bajonettverbindung miteinander verbindbar sind.

**16.** Dosiervorrichtung nach Anspruch 15, **dadurch gekennzeichnet, dass** die Bajonettverbindung am Chassis (35) ausgebildet ist.

**17.** Dosiervorrichtung nach einem der Ansprüche 15 oder 16, **dadurch gekennzeichnet, dass** die Bajonettverbindung in Bezug zu einer axialen Verfahrachse für die Spindelvorrichtung (S) auf in etwa der Höhe des Anschlags (12) für die Spindelvorrichtung (S) angeordnet ist.

**18.** Dosiervorrichtung nach einem der Ansprüche 15 bis 17, **dadurch gekennzeichnet, dass** das Gehäuse (3) eine axiale Längsführung (32) für den mindestens einen Verbrauchsteil (1) aufweist, über welche die Spindelvorrichtung (S) geführt mit dem Kupplungsglied des wiederverwendbaren Teils (2) kuppelbar ist und über welche der Verbrauchsteil (1) geführt einer Lagerstelle für die Bajonettverbindung zuführbar ist.

**19.** Dosiervorrichtung nach Anspruch 18, **dadurch gekennzeichnet, dass** die axiale Längsführung (32) aus zwei zueinander gerichteten Nuten gebildet ist und der mindestens eine Verbrauchsteil (1) zwei, jeweils in eine Nut eingreifende Nocken (31) aufweist.

**Claims**

**1.** A metering device for administering pharmaceutical fluid to the body of a user wherein the pharmaceutical fluid is dispensable in metered manner for the purpose of an infusion or an injection from a reservoir (A) by the advance of a plunger (K) held in the reservoir (A), the metering device (D) additionally comprising:

a) a reusable part (2) comprising a housing (3), a control unit and a driving device (M) wherein the driving device (M) has a coupling element for transfer of a drive power to a spindle device (S) for advancing the plunger (K), and the coupling element is fixedly connected to an output wheel (14) of the driving device (M) and has an axial stop (12) for the spindle device (S);
b) at least one disposable part (1) comprising the reservoir (A) and the plunger (K) which is held in the reservoir (A) and can be advanced by the spindle device (S), wherein the spindle device (S) is disposed on at least one disposable part (1) and has at least one spindle drive with a path of movement (V) per spindle drive wherein the spindle device (S) of the disposable part (1) can be coupled to the reusable part (2) via the coupling element;
c) a fluid-carrying connection (F) between the reservoir (A) and the user; and
d) a force sensor (15) disposed on the reusable part (2) which measures a reactive force of the spindle device (S) which serves as a measure of the pressure in the reservoir (A),

wherein

i) the metering device is designed so that the spindle device (S) of the disposable part (1) is in the retracted state after a partial or complete filling of the reservoir (A) and thus a defined total stroke (H) of the spindle device (S)2 which is always constant can be travelled,
ii) the coupling element is supported axially with a friction bearing and the output wheel (14) is supported on a fixed base via the force sensor (15) wherein because of the fixed connection between the coupling element and the output wheel (14) when the spindle drive (S) comes to rest against the stop (12) the reactive force of the spindle device (S) acting axially on the stop (12) is directed to the force sensor (15) via the output wheel (14) so that there is a direct transfer of force from the stop (12) via the output wheel (14) to the force sensor (15).

**2.** The metering device according to claim 1, **characterized in that** a sealing site is disposed between the output wheel (14) and the coupling element.

**3.** The metering device according to claim 1 or 2, **characterized in that** the metering device is designed so that after coupling a recently-filled disposable part (1) and the reusable part (2), the spindle device (S) can be extended against the stop (12) to eliminate a longitudinal play in the direction opposite the forward direction and/or can be extended when the spindle device (S) comes to rest against the stop (12) in the forward direction.

**4.** The metering device according to any one of claims 1 to 3, **characterized in that** the coupling element is designed as a profiled driving rod (11) with the stop (12) for the spindle device (S).

**5.** The metering device according to claim 4, **characterized in that** the driving rod (11) of the reusable part (2) can be inserted into an axial elongated hole (27) in the spindle device (S) of the disposable part (1) in coupling between the at least one disposable part (1) that is partially or completely filled and the reusable part (2), and the disposable part (1) can be uncoupled from the reusable part (2) by pulling out the driving rod (11) from the axial elongated hole (27).

6. The metering device according to either of claims 4 or 5, **characterized in that** the length of the driving rod (11) starting from the stop (12) is equal to or greater than the distance of travel (V) of a spindle drive.

7. The metering device according to any one of claims 1 to 6, **characterized in that** the driving device (M) has the same direction of rotation for extending it in reverse and for extending it in forward direction.

8. The metering device according to any one of claims 1 to 7, **characterized in that** the spindle device (S) is disposed on the plunger (K) and is displaceable together with the spindle device (S) in filling the plunger (K).

9. The metering device according to any one of claims 1 to 8, **characterized in that** the spindle device (S) can always be extracted by the same total stroke (H) for each new reservoir (A), regardless of whether the reservoir (A) is filled partially or completely.

10. The metering device according to claim 9, **characterized in that** the total stroke (H) is comprised of a reverse stroke (H1) for eliminating the longitudinal play and a stroke (H2) directed in the forward direction for administering the pharmaceutical fluid.

11. The metering device according to any one of claims 1 to 10, **characterized in that** the driving device (M) comprises a motor (6) and a gear (7, 9) driven by the motor (6), wherein a last gearwheel is designed as the output wheel (14).

12. The metering device according to any one of claims 1 to 11, **characterized in that** the housing (3) of the reusable part (2) is formed from three housing parts (34, 35, 36), wherein a first housing part (34) accommodates at least the control unit and/or a power source (5), a second housing part (35) is designed as a chassis (35) and accommodates at least the motor (6), the gear (7, 9) and the coupling element which is designed as a driving rod (11), and a third housing part (36) is designed as a gear bottom (36).

13. The metering device according to claim 12, **characterized in that** the base for the force sensor (15) is formed by a plate (42) that can be fastened onto the chassis (35).

14. The metering device according to either of claims 12 or 13, **characterized in that** the chassis (35) accommodates the first (34) and third housing parts (36).

15. The metering device according to any one of claims 1 to 14, **characterized in that** the at least one disposable part (1) and the reusable part (2) can be connected to one another via a bayonet connection.

16. The metering device according to claim 15, **characterized in that** the bayonet connection is formed on the chassis (35).

17. The metering device according to either of claims 15 or 16, **characterized in that** the bayonet connection is disposed at approximately the height of the stop (12) for the spindle device (S) with respect to an axial axis of movement for the spindle device (S).

18. The metering device according to any one of claims 15 to 17, **characterized in that** the housing (3) has an axial longitudinal guide (32) for the at least one disposable part (1) by means of which the spindle device (S) is guided and can be coupled with the coupling element of the reusable part (2) and by means of which the disposable part (1) is guided and can be supplied to a depository for the bayonet connection.

19. The metering device according to claim 18, **characterized in that** the axial longitudinal guide (32) is formed by two grooves directed toward one another and the at least one disposable part (1) has two cams (31), each engaging in a groove.

**Revendications**

1. Dispositif de dosage destiné à administrer un médicament liquide au corps d'un utilisateur dans lequel le médicament liquide est distribué d'une manière dosée dans le but d'une infusion ou d'une injection à partir d'un réservoir (A) par l'avancement d'un piston (K) maintenu dans le réservoir (A), le dispositif de dosage (D) comprenant en outre :

   a) une partie réutilisable (2) comprenant un boîtier (3), une unité de commande et un dispositif d'entraînement (M) dans lequel le dispositif d'entraînement (M) possède un élément de couplage pour le transfert d'une puissance motrice vers un dispositif à broche (S) pour faire avancer le piston (K), et l'élément de couplage est raccordé de manière fixe à une roue de sortie (14) du dispositif d'entraînement (M) et possède une butée axiale (12) pour le dispositif à broche (S) ;
   b) au moins une partie jetable (1) comprenant le réservoir (A) et le piston (K) qui est maintenu dans le réservoir (A) et peut être mise en avant par le dispositif à broche (S), dans laquelle le dispositif à broche (S) est disposé sur au moins une partie jetable (1) et possède au moins un entraînement à broche avec une voie de mouvement (V) par entraînement à broche dans lequel le dispositif à broche (S) de la partie jetable (1) peut être couplé à la partie réutilisable (2) par l'intermédiaire de l'élément de couplage;
   c) un raccordement de transport fluidique (F) entre le réservoir (A) et l'utilisateur et
   d) un capteur de force (15) disposé sur la partie réutilisable (2) qui mesure une force réactive du dispositif à broche (S) qui sert en tant que mesure de la pression dans le réservoir (A),

   dans lequel

   i) le dispositif de dosage est conçu de sorte que le dispositif à broche (S) de la partie jetable (1) se trouve dans l'état rétracté après un remplissage partiel ou complet du réservoir (A) et ainsi une course totale (H) définie du dispositif à broche (S) 2 qui est toujours constante peut être parcourue,
   ii) l'élément de couplage est supporté de manière axiale par un palier de friction et la roue de sortie (14) est supportée sur une base fixée par l'intermédiaire du capteur de force (15) dans lequel à cause du raccordement fixe entre l'élément de couplage et la roue de sortie (14) lorsque l'entraînement à broche (S) se repose contre la butée (12), la force réactive du dispositif à broche (S) agissant de manière axiale sur la butée (12) est dirigée vers le capteur de force (15) par l'intermédiaire de la roue de sortie (14) de sorte qu'il existe un transfert direct de force à partir de la butée (12) par l'intermédiaire de la roue de sortie (14) vers le capteur de force (15).

2. Dispositif de dosage selon la revendication 1, **caractérisé en ce qu'**un site d'étanchéité est disposé entre la roue de sortie (14) et l'élément de couplage.

3. Dispositif de dosage selon la revendication 1 ou 2, **caractérisé en ce que** le dispositif de dosage est conçu de sorte qu'après le couplage d'une partie jetable récemment remplie (1) et de la partie réutilisable (2), le dispositif à broche (S) peut être étendu contre la butée (12) pour éliminer un jeu longitudinal dans la direction opposée à la direction avant et/ou peut être étendu lorsque le dispositif à broche (S) se repose contre la butée (12) dans la direction avant.

4. Dispositif de dosage selon l'une quelconque des revendications 1 à 3, **caractérisé en ce que** l'élément de couplage est conçu en tant qu'une tige d'entraînement profilée (11) avec la butée (12) pour le dispositif à broche (S).

5. Dispositif de dosage selon la revendication 4, **caractérisé en ce que** la tige d'entraînement (11) de la partie réutilisable (2) peut être insérée à l'intérieur d'une perforation allongée axiale (27) dans le dispositif à broche (S) de la partie jetable (1) en couplage avec l'au moins une partie jetable (1) qui est partiellement ou complètement remplie et la partie réutilisable (2), et la partie jetable (1) peut être découplée de la partie réutilisable (2) en retirant la tige d'entraînement (11) de la perforation allongée axiale (27).

6. Dispositif de dosage selon l'une quelconque des revendications 4 ou 5, **caractérisé en ce que** la longueur de la tige d'entraînement (11) commençant au niveau de la butée (12) est supérieure ou égale à la distance de parcours (V) d'un entraînement à broche.

7. Dispositif de dosage selon l'une quelconque des revendications 1 à 6, **caractérisé en ce que** le dispositif d'entraînement (M) possède la même direction de rotation pour l'étendre dans la direction inverse et pour l'étendre dans la direction avant.

8. Dispositif de dosage selon l'une quelconque des revendications 1 à 7, **caractérisé en ce que** le dispositif à broche (S) est disposé sur le piston (K) et est mobile ensemble avec le dispositif à broche (S) lors du remplissage du piston (K).

9. Dispositif de dosage selon l'une quelconque des revendications 1 à 8, **caractérisé en ce que** le dispositif à broche (S) peut toujours être extrait par la même course totale (H) pour chaque nouveau réservoir (A), peu importe si le réservoir (A) est rempli partiellement ou complètement.

10. Dispositif de dosage selon la revendication 9, **caractérisé en ce que** la course totale (H) est composée d'une course de retour (H1) pour l'élimination du jeu longitudinal et d'une course (H2) dirigée dans la direction avant pour l'administration du médicament liquide.

11. Dispositif de dosage selon l'une quelconque des revendications 1 à 10, **caractérisé en ce que** le dispositif d'entraînement (M) comprend un moteur (6) et un engrenage (7, 9) entraîné par le moteur (6), dans lequel une dernière roue d'engrenage est conçue comme la roue de sortie (14).

12. Dispositif de dosage selon l'une quelconque des revendications 1 à 11, **caractérisé en ce que** le boîtier (3) de la partie réutilisable (2) est formé à partir de trois parties de boîtier (34, 35, 36), dans lequel une première partie de boîtier (34) loge au moins l'unité de commande et/ou une source d'alimentation (5), une seconde partie de boîtier (35) est conçue en tant que châssis (35) et loge au moins le moteur (6), l'engrenage (7, 9) et l'élément de couplage qui est conçu en tant que tige d'entraînement (11), et une troisième partie de boîtier (36) est conçue en tant que fond d'engrenage (36).

13. Dispositif de dosage selon la revendication 12, **caractérisé en ce que** la base pour le capteur de force (15) est formée d'une plaque (42) qui peut être attachée sur le châssis (35).

14. Dispositif de dosage selon l'une quelconque des revendications 12 ou 13, **caractérisé en ce que** le châssis (35) loge les première (34) et troisième parties de boîtier (36).

15. Dispositif de dosage selon l'une quelconque des revendications 1 à 14, **caractérisé en ce que** l'au moins une partie jetable (1) et la partie réutilisable (2) peuvent être raccordées l'une à l'autre par l'intermédiaire d'un raccordement à baïonnette.

16. Dispositif de dosage selon la revendication 15, **caractérisé en ce que** le raccordement à baïonnette est formé sur le châssis (35).

17. Dispositif de dosage selon l'une quelconque des revendications 15 ou 16, **caractérisé en ce que** le raccordement à baïonnette est approximativement disposé au niveau de la hauteur de la butée (12) pour le dispositif à broche (S) par rapport à un axe de mouvement axial pour le dispositif à broche (S).

18. Dispositif de dosage selon l'une quelconque des revendications 15 à 17, **caractérisé en ce que** le boîtier (3) possède un guide longitudinal axial (32) pour l'au moins une partie jetable (1) au moyen duquel le dispositif à broche (S) est guidé et peut être couplé avec l'élément de couplage de la partie réutilisable (2) et au moyen duquel la partie jetable (1) est guidée et peut être fournie à un dépôt pour le raccordement à baïonnette.

19. Dispositif de dosage selon la revendication 18, **caractérisé en ce que** le guide longitudinal axial (32) est formé de deux rainures tournées l'une vers l'autre et l'au moins une partie jetable (1) possède deux cames (31), chacune s'engageant dans une rainure.

17
18

A
1
K

31

S

28

19
27

30
22
20
23
24
25
26
29
21

3
32

2
4

5

6

11
12
10
13
14
15

7

8

9

M

# Fig. 1a

L

Do

33

# Fig. 1b

EP 3 142 728 B1

Fig. 2a      Fig. 2b      Fig. 2c      Fig. 2d

33

Fig. 3a

Fig. 3b

Fig. 3c

34

37

5

4

6

7

15

35

42

9

36

Fig. 3d

39

Fig. 3e

45

1

A

17

18

45

16

## Fig.4a

S

19

20

(23)

22

25

(26)

29

21

24

K

31

## Fig.4b

52

51

46

47

**Fig.5a**

62

48

54

76

61

F →

57

60

**Fig.5b**

55

56

53b

53a

**Fig.5c**

Fig.5d

Fig.5e

Fig.6a

Fig.6b

Fig.6c

48

46

51

59

62

49/50

## Fig.6d

57

56          56

55

## Fig.6e

57

56

59

## Fig.6f

66
65
67
69

59

49

46

53a

47

53b

1

2

D →

64

Fig.6g          Fig.6h          Fig.6i          Fig.6j

63  45

4

3

SECTION A-A

Fig.6k

59     68 58

F

19

20

22

11
21
3
14
15

SECTION B-B

Fig.6l

B

48

D

A

1

2

A

B

35    74      32

Fig.8a

74

33

75    A

30

Fig.8b

**IN DER BESCHREIBUNG AUFGEFÜHRTE DOKUMENTE**

**In der Beschreibung aufgeführte Patentdokumente**

- DE 19717107 B4
- DE 19840992 A1
- WO 2006104806 A
- WO 2010076792 A1
- US 20110213329 A1
- WO 2009125398 A
- WO 2008024814 A2
- WO 9415660 A
- WO 9700091 A